(19) Europäisches Patentamt
European Patent Office
Office européen des brevets

(11) Veröffentlichungsnummer: **0 098 241**
**B1**

(12) # EUROPÄISCHE PATENTSCHRIFT

(45) Veröffentlichungstag der Patentschrift: **02.10.85**

(21) Anmeldenummer: **83810256.4**

(22) Anmeldetag: **10.06.83**

(51) Int. Cl.⁴: **C 07 C 69/734,** C 07 C 67/347, G 03 C 7/30, C 07 C 59/52, C 07 C 103/78, C 07 D 307/06, C 07 D 309/06, C 07 C 121/66, C 07 C 43/178, C 07 F 9/145

(54) **Hydrochinonäther und ein Verfahren zu deren Herstellung.**

(30) Priorität: **16.06.82 GB 8217445**
**15.12.82 GB 8235665**

(43) Veröffentlichungstag der Anmeldung: **11.01.84 Patentblatt 84/2**

(45) Bekanntmachung des Hinweises auf die Patenterteilung: **02.10.85 Patentblatt 85/40**

(84) Benannte Vertragsstaaten: **BE DE FR GB IT**

(56) Entgegenhaltungen:
**EP - A - 0 025 692**
**FR - A - 2 269 333**

(73) Patentinhaber: **CIBA-GEIGY AG, Postfach, CH-4002 Basel (CH)**

(72) Erfinder: **Howell, Frederick Harold, Dr., 27 High Bank, Atherton Lancashire M29 9HZ (GB)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



BUNDESDRUCKEREI BERLIN

**Beschreibung**

Die vorliegende Erfindung betrifft neue Hydrochinonäther, insbesondere funktionell alkylierte Hydrochinonäther, welche in photographischen Systemen Anwendung finden können.

Hydrochinonderivate mit funktionellen Gruppen sind beispielsweise in der GB-Patentschrift Nr. 1 465 082 (monoalkylierte Hydrochinone) und in der JP-Patentanmeldung Nr. 54/73 032 (Hydrochinondiäther) beschrieben.

Die neuen erfindungsgemäßen Hydrochinonäther entsprechen der Formel I

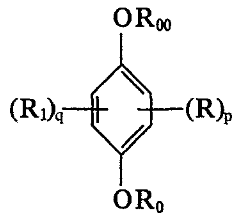

(I)

worin p 1 oder 2 und q 0 oder 1 sind, mit der Maßgabe, daß p + q 1 oder 2 ist,

R einen Rest der Formel II

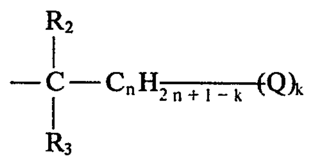

(II)

bedeutet, in welcher

Q die folgenden Reste bedeutet:

i) $-COOR_4$ oder $-CON(R_4)(R_5)$, wobei
$R_4$ unabhängig Wasserstoff, $C_{1-20}$-Alkyl, das durch ein bis fünf Sauerstoffatome unterbrochen und durch eine Gruppe $-OR_6$ substituiert sein kann, eine bifunktionelle $C_{2-20}$-Alkylengruppe, $C_{3-20}$-Alkenyl, $C_{3-12}$-Cycloalkyl, $C_{6-10}$-Aryl, das durch eine oder zwei $C_{1-8}$-Alkylgruppen substituiert sein kann, $C_{7-13}$-Aralkyl, einen 5- oder 6gliedrigen, sauerstoffhaltigen Heterocyclus, der durch eine oder zwei $C_{1-4}$-Alkylgruppen substituiert sein kann, oder Methyl darstellt, das durch einen 5- oder 6gliedrigen, ein Sauerstoffatom enthaltenden, unsubstituierten oder durch eine oder zwei $C_{1-4}$-Alkylgruppen substituierten Heterocyclus substituiert ist,
$R_5$ Wasserstoff, $C_{1-20}$-Alkyl oder $C_{5-6}$-Cycloalkyl bedeutet oder $R_4$ und $R_5$ zusammen mit dem Stickstoffatom, an das sie gebunden sind, einen 5- oder 6gliedrigen Heterocyclus bilden, der durch eine oder zwei $C_{1-4}$-Alkylgruppen substituiert sein kann, und
$R_6$ $C_{3-12}$-Cycloalkyl, $C_{3-20}$-Alkenyl, $C_{6-10}$-Aryl, das durch eine oder zwei $C_{1-4}$-Alkylgruppen substituiert sein kann, oder $C_{7-13}$-Aralkyl bedeutet,

ii) $-OR_7$ oder $-OCOR_7$, wobei
$R_7$ Wasserstoff, $C_{1-20}$-Alkyl, $C_{3-20}$-Alkenyl, $C_{3-12}$-Cycloalkyl, $C_{7-13}$-Aralkyl oder $C_{6-10}$-Aryl darstellt, das durch eine oder zwei $C_{1-4}$-Alkylgruppen substituiert sein kann,

iii) $-N(R_8)(R_9)$, wobei
$R_8$ und $R_9$ unabhängig voneinander $-H$, $C_{1-20}$-Alkyl, $C_{3-20}$-Alkenyl, $C_{3-12}$-Cycloalkyl, $C_{7-13}$-Aralkyl oder $C_{6-10}$-Aryl, das durch eine oder zwei $C_{1-4}$-Alkylgruppen substituiert sein kann, bedeuten, oder ferner die Gruppe $-COR_7$ bedeuten, wobei $R_7$ die oben angegebene Bedeutung hat, oder $R_8$ und $R_9$ zusammen mit dem Stickstoffatom, an das sie gebunden sind, einen unsubstituierten oder durch eine oder zwei $C_{1-4}$-Alkylgruppen substituierten 5- oder 6gliedrigen heterocyclischen Ring bilden,

iv) $-PO(OR_{10})([O]_xR_{11})$, wobei x die Zahl 0 oder 1 ist,
$R_{10}$ und $R_{11}$ bei $x = 1$ unabhängig voneinander Wasserstoff, $C_{1-20}$-Alkyl, $C_{6-10}$-Aryl, das durch eine oder zwei $C_{1-4}$-Alkylgruppen substituiert sein kann, oder $R_{10}$ und $R_{11}$ zusammen $C_{2-3}$-Alkylen darstellen, das durch eine bis vier $C_{1-20}$-Alkylgruppen substituiert sein kann, und bei $x = 0$ $R_{10}$ die oben angegebene Bedeutung hat und
$R_{11}$ $C_{1-5}$-Alkyl bedeutet,

v) $-SO_2R_{12}$, wobei $R_{12}$ $-OH$, $-Cl$ oder $-N(R_5)(R_7)$ ist, und $R_5$ und $R_7$ die oben angegebene Bedeutung haben, mit der Maßgabe, daß $R_1$ einen Rest der Formel II darstellt, wenn $R_{12}$ $-OH$ bedeutet,

vi) $-CN$, Halogen oder $-NO_2$, und

vii) $-COR_{15}$, wobei $R_{15}$ $-H$ oder $C_{1-20}$-Alkyl oder Halogen bedeutet,
n eine ganze Zahl von 1 bis 20 und
k die Zahl 1 oder 2 sind,

$R_2$ und $R_3$ unabhängig voneinander $C_{1-5}$-Alkyl sind, wobei, wenn Q $-COOR_4$ darstellt, eines von $R_2$ oder $R_3$ durch eine Gruppe $-COOR_4$ mit gleichen oder verschiedenen $R_4$ substituiert sein kann, oder $R_2$ oder $R_3$ so mit dem Rest $-C_nH_{2n}-COOR_4$ verbunden sein können, daß ein durch die Gruppe $-COOR_4$ mit gleichen oder verschiedenen $R_4$ substituierter $C_{5-12}$-Cycloalkylenrest gebildet wird, wobei $R_4$ die oben angegebene Bedeutung hat, oder wenn Q $-COR_{15}$ bedeutet, $R_2$ und $R_3$ so mit dem Rest $-C_nH_{2n}-COR_{15}$ verbunden sein können, daß einen $C_{5-12}$-Cycloalkylenrest gebildet wird, wobei $R_{15}$ die oben angegebene Bedeutung hat, mit der Maßgabe, daß p und k die Zahl 1 sind und die Gruppe der Formel I die Formel Ia aufweist, wenn die Gruppe Q den Rest $-COOR_4$ bedeutet, wobei $R_4$ einen bifunktionellen $C_{2-20}$-Alkylenrest darstellt:

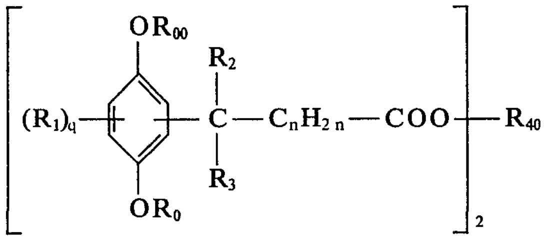

(Ia)

wobei $R_{40}$ ein bifunktionelles $C_{2-20}$-Alkylen ist, und wenn Q einen Rest $-CON(R_4)(R_5)$ und $R_4$ ein bifunktionelles $C_{2-20}$-Alkylen darstellen, p und k die Zahl 1 sind und der Rest der Formel I die Formel Ib aufweist:

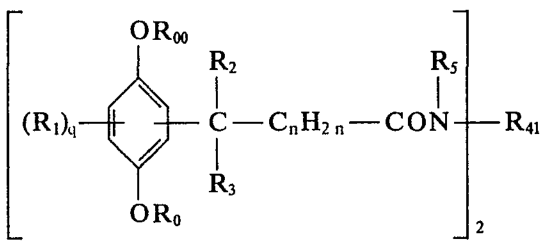

(Ib)

wobei $R_2$, $R_3$, q und n die oben angegebene Bedeutung haben und $R_{41}$ $C_{2-20}$-Alkylen bedeutet,

$R_1$ $C_{1-8}$-Alkyl, $C_{5-7}$-Cycloalkyl, das durch eine oder zwei Methyl- oder Äthylgruppen substituiert sein kann, $C_{7-9}$-Aralkyl oder einen Rest der Formel II, wie oben definiert, bedeutet, und wenn $R_1$ ein Rest der Formel II ist, können $R_1$ und R gleich oder verschieden sein, oder

$R_1$ einen Rest der Formel III bedeutet

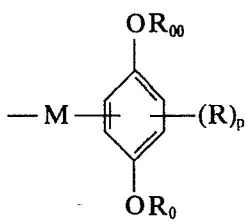

(III)

in welcher

R und p die oben angegebene Bedeutung haben, M eine direkte Bindung,

$-C(R_{13})(R_{14})-$, wobei $R_{13}$ und $R_{14}$ unabhängig voneinander $-H$, $C_{1-20}$-Alkyl, das durch ein bis drei Schwefelatome unterbrochen sein kann, oder $C_{6-10}$-Aryl bedeuten, oder $R_{13}$ und $R_{14}$ zusammen mit dem Kohlenstoffatom, an das sie gebunden sind, ein 5- oder 6gliedriges Alkylen darstellen können, das durch eine oder zwei $C_{1-8}$-Alkylgruppen substituiert sein kann, ferner M $-S-$, $-S-S-$, $-SO_2-$, $-CH_2SCH_2-$, $-CH_2OCH_2-$ oder

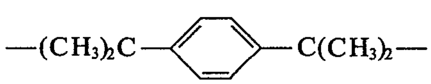

bedeutet,

$R_0$ und $R_{00}$ unabhängig voneinander –H, $C_{1-20}$-Alkyl, das durch 1 bis 5 Sauerstoffatome unterbrochen sein kann, $C_{3-12}$-Cycloalkyl, $C_{6-10}$-Aryl, das durch eine oder zwei $C_{1-4}$-Alkylgruppen substituiert sein kann, oder $C_{7-13}$-Aralkyl bedeuten, mit der Maßgabe, daß $R_0$ und $R_{00}$ gleichzeitig nicht Wasserstoff darstellen können, oder $R_{00}$ die oben angegebene Bedeutung hat und $R_0$ einen Rest der Formel IV bedeutet:

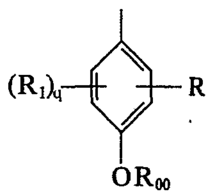

(IV)

in welcher

R, $R_{00}$, $R_1$ und q die oben angegebene Bedeutung haben, oder $R_0$ und $R_1$, wenn sie in ortho-Stellung zueinander stehen, zusammen mit den Kohlenstoffatomen, an die sie gebunden sind, einen gegebenenfalls substituierten Rest der Formel V

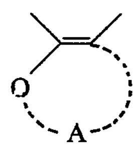

(V)

bilden können, wobei A ein Kohlenstoffrest, enthaltend 4 bis 20 C-Atome, welcher ein substituiertes Chroman- oder Coumaransystem bildet, oder $R_0$ und $R_1$ zusammen einen Rest der Formel VI oder der Formel VIII bilden:

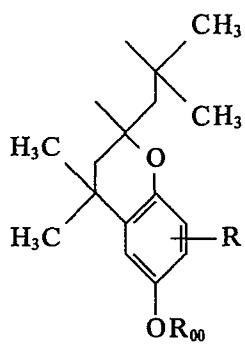

(VI)

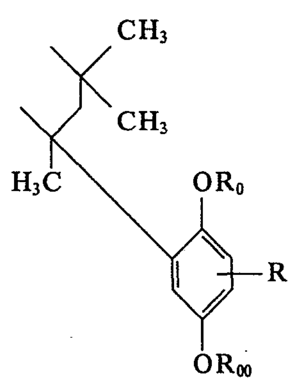

(VII)

wobei R, $R_0$ und $R_{00}$ die oben angegebene Bedeutung haben, mit der Maßgabe, daß die Verbindung der Formel I einen einzigen Rest der Formel III oder IV enthält, oder $R_{00}$ –H und $R_0$ ein Rest der Formel IVa oder IVb sind:

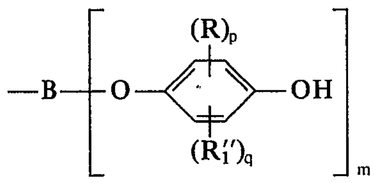

(IVa)

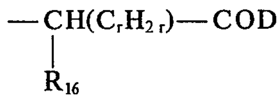

(IVb)

wobei p, q und R die oben angegebene Bedeutung haben, $R_1''$ $C_{1-8}$-Alkyl, $C_{7-9}$-Aralkyl oder ein Rest der Formel II, wie oben definiert, ist, m die Zahl 1 oder 2 ist, B bei m = 1 $C_{2-12}$-Alkylen, das durch ein bis drei Sauerstoff- oder Schwefelatome unterbrochen sein kann, $C_{4-10}$-Alkenylen, $C_{5-12}$-Cycloalkylen, $C_{6-12}$-Arylen, $C_{8-12}$-Aralkylen, $C_{4-6}$-Alkynylen, Xylylen, $-CH_2CH(OH)CH_2-$, $-CH_2CH(OH)CH_2-O-Y-O-CH_2CH(OH)CH_2-$,

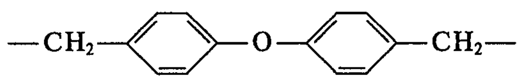 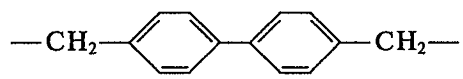

oder $-CH_2COO-B'-OCOCH_2-$ bedeutet, wobei Y $C_{2-10}$-Alkylen, $C_{6-12}$-Arylen,

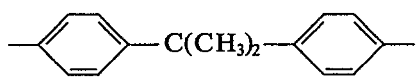

oder $C_{6-12}$-Cycloalkylen bedeutet,

B' $C_{2-8}$-Alkylen, $C_{4-8}$-Oxaalkylen oder Cyclohexylen und B bei m = 2 einen Rest der Formel

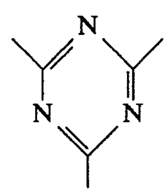 oder 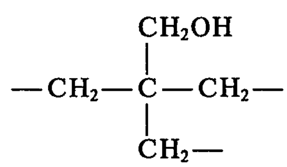 bedeuten,

r eine ganze Zahl von 0 bis 12, vorzugsweise 0 ist, und $R_{16}$ –H, $C_{1-12}$-Alkyl und D –$OR_4$ oder –$N(R_4)(R_5)$ bedeuten, wobei $R_4$ und $R_5$ die oben angegebene Bedeutung haben,

sowie Salze von Verbindungen der Formel I mit Basen oder organischen oder anorganischen Säuren, wenn Q eine saure oder basische Gruppe ist.

Die Reste, welche Alkyl- oder Alkenylgruppen bedeuten und noch weitere Alkylsubstituenten an denselben Resten tragen, können, sofern nichts anderes angegeben ist, geradkettig oder verzweigt sein.

Ist $R_1$ geradkettiges oder verzweigtes $C_{1-8}$-Alkyl, so handelt es sich z. B. um Methyl, Äthyl, n-Propyl, Isopropyl, n-Butyl, Isobutyl, tert.-Butyl, tert.-Pentyl oder 1,1,3,3-Tetramethylbutyl. Beispiele von geradkettigen oder verzweigten $C_{1-5}$-Alkylgruppen $R_2$ und $R_3$ sind die Methyl-, Äthyl-, n-Propyl-, Isopropyl-, n-Butyl-, sek.-Butyl-, n-Pentyl- oder Neopentylgruppen. Bedeuten $R_4$, $R_0$ und $R_{00}$ geradkettiges oder verzweigtes $C_{1-20}$-Alkyl, das durch ein bis fünf Sauerstoffatome unterbrochen sein kann, so kommen z. B. die folgenden Gruppen in Betracht: Methyl, Äthyl, 2-Methoxyäthyl, 2-Äthoxyäthyl, 2-n-Propoxyäthyl, 2-n-Butoxyäthyl, n-Pentyl, n-Hexyl, n-Heptyl, n-Octyl, 2-Äthylhexyl, n-Nonyl, n-Decyl, n-Dodecyl, n-Tetradecyl, n-Hexadecyl, n-Octadecyl, n-Eikosyl, $-(C_2H_4O)_2CH_3$, $-(C_2H_4O)_3CH_3$, $-(C_2H_4O)_4CH_3$ oder $-(C_2H_4O)_5CH_3$.

Beispiele von geradkettigen oder verzweigten Alkenylgruppen $R_4$, $R_6$, $R_7$, $R_8$ oder $R_9$ sind: Prop-2-enyl, n-But-2-enyl, 2-Methyl-prop-2-enyl, n-Pent-2-enyl, n-Hex-2-enyl, n-Hexa-2,4-dienyl, n-Dec-10-enyl oder n-Eikos-2-enyl.

Stellen $R_4$, $R_6$, $R_7$, $R_8$, $R_9$, $R_0$ und $R_{00}$ $C_{3-12}$-Cycloalkylgruppen dar, so kommen z. B. Cyclopropyl-, Cyclobutyl-, Cyclopentyl-, Cyclohexyl-, Cyclooctyl-, Cyclodecyl-, Adamantyl- oder Cyclododecylgruppen in Betracht. $R_5$ als $C_{5-6}$-Cycloalkyl ist beispielsweise Cyclopentyl oder Cyclohexyl.

Als Beispiele von $C_{7-13}$-Aralkylgruppen $R_4$, $R_6$, $R_7$, $R_8$, $R_9$, $R_0$ oder $R_{00}$ seien Benzyl, Phenyläthyl, Benzhydryl oder Naphthylmethyl genannt. $R_1$ als $C_{7-9}$-Aralkyl ist beispielsweise Cumyl. Bedeuten $R_6$, $R_7$, $R_8$, $R_9$, $R_{10}$, $R_{11}$, $R_0$ oder $R_{00}$ $C_{6-10}$-Aryl, das durch eine oder zwei $C_{1-4}$-Alkylgruppen substituiert sein kann, so handelt es sich z. B. um Phenyl-, Tolyl-, Xylyl-, Cumyl-, Butylphenyl- oder Naphthylgruppen. $R_4$ durch ein oder zwei $C_{1-8}$-Alkyl substituiertes $C_{6-10}$-Aryl ist beispielsweise Phenyl, Tolyl, Xylyl, Cumyl, Butylphenyl, durch eine oder zwei 1,1,3,3-Tetramethylbutylgruppen substituiertes Phenyl oder Naphthyl.

Stellt $R_4$ einen 5- oder 6gliedrigen, sauerstoffhaltigen Heterocyclus dar, der durch eine oder zwei $C_{1-4}$-Alkylgruppen substituiert sein kann, so handelt es sich z. B. um Tetrahydrofuran-3-yl, Tetrahydropyran-4-yl oder 2,6-Dimethyl-tetrahydropyran-4-yl. Ist $R_4$ Methyl, das durch einen 5- oder 6gliedrigen, Sauerstoffatom enthaltenden und gegebenenfalls durch eine oder zwei $C_{1-4}$-Alkylgruppen substituierten Heterocyclus substituiert ist, so kommen beispielsweise Furfuryl, Tetrahydrofurfuryl oder Tetrahydropyran-2-yl-methyl in Betracht.

Bilden $R_4$ und $R_5$ oder $R_8$ und $R_9$ zusammen mit dem Stickstoffatom, an das sie gebunden sind, einen 5- oder 6gliedrigen Heterocyclus, der durch eine oder zwei $C_{1-4}$-Alkylgruppen substituiert sein kann, so kann es sich z. B. um einen Pyrrolidin-, Piperidin-, Morpholin- oder 2,5-Dimethylmorpholinring handeln.

Geradkettige oder verzweigte $C_{1-20}$-Alkylgruppen $R_5$, $R_7$ bis $R_{11}$, $R_{13}$ bis $R_{15}$, $R_0$ und $R_{00}$ können gleich oder verschieden sein und z. B. Methyl, Äthyl, n-Propyl, Isopropyl, n-Butyl, sek-Butyl, n-Pentyl, n-Hexyl, n-Heptyl, n-Octyl, 2-Äthylhexyl, n-Nonyl, n-Decyl, n-Dodecyl, n-Tetradecyl, n-Hexadecyl, n-Octadecyl oder n-Eikosyl darstellen.

Bilden $R_{10}$ und $R_{11}$ zusammen gegebenenfalls durch eine bis vier $C_{1-20}$-Alkylgruppen substituiertes $C_{2-3}$-Alkylen, so kommen z. B. die folgenden Gruppen in Betracht:

$-CH_2CH_2-$, $-CH_2CH_2CH_2-$, $-CH_2CH(CH_3)-$, $-CH_2CH(C_2H_5)-$, $-CH_2CH(C_{20}H_{41})-$,
$-CH(CH_3)CH(CH_3)-$, $-CH(CH_3)C(CH_3)_2-$, $-C(CH_3)_2C(CH_3)_2-$, $-CH_2CH_2C(CH_3)_2-$
oder $-CH(CH_3)CH_2CH(CH_3)-$.

Bilden $R_{13}$ und $R_{14}$ zusammen gegebenenfalls durch eine oder zwei $C_{1-8}$-Alkylgruppen substituiertes $C_{4-5}$-Alkylen, so kommen z. B. die folgenden Gruppen in Betracht:

$-(CH_2)_4-$, $-(CH_2)_5-$, $-CH_2CH_2-CH(CH_3)-CH_2CH_2-$,
$-CH_2CH_2-CH(t.C_4H_9)-CH_2CH_2-$ oder $-CH_2CH_2CH(CH_3)CH_2CH(CH_3)-$.

Bilden $R_0$ und R zusammen mit den Kohlenstoffatomen, an die sie gebunden sind, einen substituierten Chroman- oder Coumaranring, so handelt es sich z. B. um 2,2,4-Trimethyl- oder 2,2-Dimethyl-4-isopropylchroman oder 2,2-Dimethylcoumaran.

Beispiele von gegebenenfalls durch ein bis drei Sauerstoff- oder Schwefelatome unterbrochenen $C_{2-22}$-Alkylengruppen B sind

$-(CH_2)_2-$, $-(CH_2)_3-$, $-(CH_2)_4-$, $-(CH_2)_6-$, $-(CH_2)_8-$, $-(CH_2)_{10}-$, $-(CH_2)_{16}-$, $-(CH_2)_{22}-$,
$-CH_2CH_2OCH_2CH_2-$, $-CH_2CH_2(OCH_2CH_2)_2-$, $-CH_2CH_2(OCH_2CH_2)_3-$ oder $-CH_2CH_2SCH_2CH_2-$.

Ist B $C_{4-10}$-Alkenylen, so handelt es sich beispielsweise um

$-CH_2CH{=}CH-CH_2$, $-CH_2CH{=}CH-CH{=}CH-CH_2-$, $-(CH_2)_4-CH{=}CH-(CH_2)_4-$.

B als $C_{5-8}$-Cycloalkylen ist beispielsweise 1,1-Cyclohexylen, 1,2-Cyclohexylen oder 1,4-Cyclohexylen.

B und Y als $C_{6-12}$-Arylen ist beispielsweise 1,2-Phenylen, 1,4-Phenylen oder 4,4'-Biphenylen.

B als $C_{8-12}$-Aralkylen ist beispielsweise

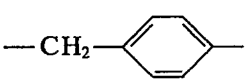 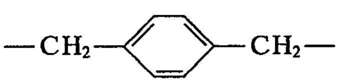 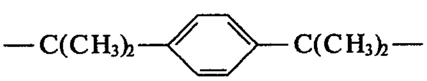

B als $C_{4-6}$-Alkynylen ist beispielsweise $-CH_2-C{\equiv}C-CH_2-$ oder $-C{\equiv}C-C{\equiv}C-$.

Y als $C_{2-10}$-Alkylen ist beispielsweise $-(CH_2)_2-$, $-(CH_2)_3-$, $-(CH_2)_4-$, $-(CH_2)_6-$, $-(CH_2)_8-$ oder $-(CH_2)_{10}-$.

Y als $C_{6-12}$-Cycloalkylen ist beispielsweise 1,2-Cyclohexylen, 1,3-Cyclohexylen, 1,4-Cyclohexylen, 1,2-Cyclooctylen oder 1,2-Cyclododecylen.

B' als $C_{2-8}$-Alkylen ist beispielsweise $-(CH_2)_2-$, $-(CH_2)_3-$, $-(CH_2)_4-$, $-(CH_2)_6-$ oder $-(CH_2)_8-$.

B' als $C_{4-8}$-Oxaalkylen ist beispielsweise $-CH_2CH_2OCH_2CH_2-$ oder $-CH_2CH_2(OCH_2CH_2)_3-$.

$R_{16}$ als geradkettiges oder verzweigtes $C_{1-12}$-Alkyl ist beispielsweise Methyl, Äthyl, n-Propyl, Isopropyl, Butyl, sec.Butyl, n-Pentyl, n-Hexyl, n-Heptyl, n-Octyl, 2-Äthylhexyl, n-Nonyl, n-Decyl oder n-Dodecyl.

$R_{15}$ als Halogen ist beispielsweise Fluor, Chlor oder Brom.

Als Beispiele von Salzen, wenn Q eine saure Gruppe, d. h. $-COOH$ darstellt, seien Salze mit Alkali- und Erdalkalimetallen und Aminen genannt. Ist Q eine Gruppe $-N(R_8)(R_9)$, so kommen Salze mit organischen oder anorganischen Säuren, z. B. Chlorwasserstoffsäure, Schwefelsäure, para-Toluolsulfonsäure und Oxalsäure, in Betracht.

Gemäß einer bevorzugten Ausführungsform sind R und $R_1$ in 2- und 5-Stellung an den Hydrochinonäther der Formel I gebunden.

Des weiteren bevorzugt sind Verbindungen der Formel VIII:

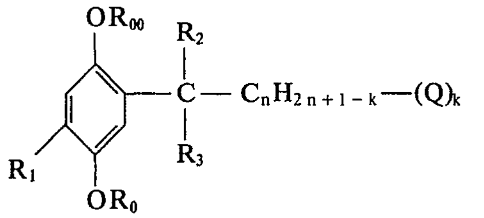

(VIII)

worin $R_2$, $R_3$, $R_0$, $R_{00}$, n, k und Q die oben angegebene Bedeutung haben und $R_1$ einen Rest der Formel II, wie oben definiert, oder einen Rest der Formel IX:

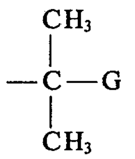

(IX)

darstellt, worin G $C_{1-5}$-Alkyl oder Phenyl darstellt, sowie Salze von Verbindungen der Formel VIII.

Ebenfalls bevorzugt sind Verbindungen der Formel VIII, worin $R_1$ eine Gruppe der Formel II oder IX, Q $-COOR_4$, $-CON(R_4)(R_5)$ oder $-OR_7$ bedeuten, wobei $R_4$, $R_5$ und $R_7$ die oben angegebene Bedeutung haben, $R_2$ und $R_3$ unabhängig voneinander Methyl, Äthyl, n-Propyl, Isopropyl oder Neopentyl bedeuten, oder entweder $R_2$ oder $R_3$ durch eine Gruppe $-COOR_4$ substituiert sein können, oder $R_2$ oder $R_3$ so an den Rest $-C_nH_{2n}-COOR_4$ gebunden sind, daß ein durch $-COOR_4$ substituierter $C_{5-8}$-Cycloalkylenrest gebildet wird, wobei n, k, $R_0$, $R_{00}$ und $R_4$ die oben angegebene Bedeutung haben, sowie Salze dieser Verbindungen.

Besonders bevorzugt sind Verbindungen der Formel VIII, worin k die Zahl 1 ist, $R_1$ eine Gruppe der Formel II oder IX ist, Q $-COOR_4$, $-CON(R_4)(R_5)$ oder $-OR_7$ darstellt, n eine Zahl von 1 bis 10 ist, $R_2$ und $R_3$ unabhängig voneinander Methyl, Äthyl oder Neopentyl darstellen, oder entweder $R_2$ oder $R_3$ so an den Rest $-C_nH_{2n}-COOR_4$ gebunden sind, daß ein durch $-COOR_4$ substituierter Cyclohexylenrest gebildet wird, $R_4$ Wasserstoff oder $C_{1-20}$-Alkyl bedeutet, das durch 1 bis 3 Sauerstoffatome unterbrochen und/oder durch eine Gruppe $-OR_6$ substituiert sein kann, wobei $R_6$ Cyclopentyl, Cyclohexyl, Cyclooctyl, $C_{3-10}$-Alkenyl, Phenyl, Benzyl, Phenäthyl, Benzhydryl oder Naphthylmethyl bedeutet, ferner $R_4$ $C_{3-15}$-Alkenyl, Phenyl, das durch eine oder zwei $C_{1-4}$-Alkylgruppen substituiert sein kann, Benzyl, Phenäthyl, Cyclopentyl, Cyclohexyl, einen sauerstoffhaltigen 5- oder 6gliedrigen heterocyclischen Ring, der durch eine oder zwei $C_{1-4}$-Alkylgruppen substituiert sein kann oder Methyl bedeutet, das durch einen sauerstoffhaltigen 5- oder 6gliedrigen heterocyclischen Ring substituiert ist, und $R_5$ die oben angegebene Bedeutung hat, oder $R_4$ und $R_5$ zusammen mit dem Stickstoffatom, an das sie gebunden sind, einen 5- oder 6gliedrigen heterocyclischen Ring bilden, der durch eine oder zwei $C_{1-4}$-Alkylgruppen substituiert sein kann und $R_0$, $R_{00}$ und $R_7$ die oben angegebene Bedeutung haben, sowie Salze dieser Verbindungen.

Weitere bevorzugte Verbindungen entsprechen der Formel VIII, worin k die Zahl 1 ist, $R_1$ eine Gruppe der Formel II oder IX bedeutet, Q $-COOR_4$, $-CON(R_4)(R_5)$ oder $-OR_7$ darstellt, n eine Zahl von 1 bis 10 ist, $R_2$ und $R_3$ unabhängig voneinander Methyl, Äthyl oder Neopentyl bedeuten oder eines von $R_2$ oder $R_3$ so an den Rest $-C_nH_{2n}-COOR_4$ gebunden sind, daß ein durch $-COOR_4$ substituiertes Cyclohexylen gebildet wird, $R_4$ $C_{1-20}$-Alkyl, das durch ein oder zwei Sauerstoffatome unterbrochen und/oder durch Cyclohexyloxy, $C_{3-10}$-Alkenyloxy, Phenoxy oder Benzyloxy substituiert ist, ferner $R_4$ $C_{3-15}$-Alkenyl, Phenyl, Benzyl, Phenäthyl, Cyclopentyl, Cyclohexyl, einen sauerstoffhaltigen 5- oder 6gliedrigen heterocyclischen Ring, oder Methyl bedeutet, das durch einen sauerstoffhaltigen 5- oder 6gliedrigen heterocyclischen Ring substituiert sein kann, und $R_4$ insbesondere unsubstituiertes oder durch ein Sauerstoffatom unterbrochenes oder durch Phenoxy substituiertes $C_{1-16}$-Alkyl, oder $C_{3-15}$-Alkenyl, Phenyl, Benzyl, Tetrahydrofuran-3-yl oder Tetrahydrofurfuryl bedeutet, $R_5$ die oben angegebene Bedeutung hat, oder $R_4$ und $R_5$ zusammen mit dem Stickstoffatom, an das sie gebunden sind, einen 5- oder 6gliedrigen heterocyclischen Ring bilden, der durch eine oder zwei $C_{1-4}$-Alkylgruppen substituiert sein kann, $R_7$ $-H$ oder $C_{1-15}$-Alkyl ist, und $R_0$ und $R_{00}$ $-H$ oder $C_{1-8}$-Alkyl, das durch ein oder zwei O-Atome unterbrochen sein kann, Cyclohexyl, Phenyl oder Benzyl sind, oder $R_0$ und $R_1$ zusammen einen Rest der Formel V, wie oben definiert, bilden, sowie Salze davon.

Besonders bevorzugt sind Verbindungen der Formel VIII, worin k die Zahl 1, $R_1$ eine Gruppe der Formel II oder IX ist, Q $-COOR_4$, $-CON(R_4)(R_5)$ oder $-OR_7$ darstellt, n eine Zahl von 1 bis 3 ist, $R_2$ und $R_3$ Methyl oder Neopentyl darstellen, $R_4$ $C_{1-16}$-Alkyl, das durch ein Sauerstoffatom unterbrochen oder durch Phenoxy substituiert sein kann, ferner $R_4$ $C_{3-15}$-Alkenyl, Phenyl, Benzyl, Tetrahydrofuran-3-yl oder Tetrahydrofurfuryl bedeutet, oder $R_4$ und $R_5$ zusammen mit dem Stickstoffatom, an das sie gebunden sind, einen 5- oder 6gliedrigen heterocyclischen Ring bilden, der durch eine oder zwei $C_{1-4}$-Alkylgruppen substituiert sein kann, $R_7$ $-H$ oder $C_{1-15}$-Alkyl, und $R_0$ und $R_{00}$ $-H$ oder $C_{1-4}$-Alkyl sind, oder $R_0$ und $R_1$ zusammen einen Rest der Formel V, wie oben definiert, darstellen, und $R_5$ die oben angegebene

Bedeutung hat, sowie Salze dieser Verbindungen.

Wenn Q $-OR_7$ darstellt, ist $R_7$ insbesondere $C_{1-8}$-Alkyl.

Eines von $R_0$ und $R_{00}$ ist vorzugsweise $-H$ und das andere ist $C_{1-4}$-Alkyl oder $R_0$ und $R_1$ sind ein Rest der Formel V wie oben angegeben.

Als nicht limitierende Beispiele von Verbindungen der Formel I seien genannt:

2-(3-Methoxycarbonyl-2'-methyl-prop-2'-yl)-4-methoxyphenol
2-(3-n-Hexyloxycarbonyl-2'-methyl-prop-2'-yl)-4-methoxyphenol
5-t-Butyl-2-(3'-n-hexyloxycarbonyl-2'-methyl-prop-2'-yl)-4-methoxyphenol
5-t-Butyl-2-(3'-n-dodecyloxycarbonyl-2'-methyl-prop-2'-yl)-4-methoxyphenol
2,5-Bis-(3'-methoxycarbonyl-2'-methyl-prop-2'-yl)-4-methoxyphenol
2,5-Bis-(3'-n-hexyloxycarbonyl-2'-methyl-prop-2'-yl)-4-methoxyphenol
2,5-Bis-(3'-n-dodecyloxycarbonyl-2'-methyl-prop-2'-yl)-4-methoxyphenol
2-(4'-Methoxycarbonyl-1'-methyl-cyclohex-1'-yl)-4-methoxyphenol
2-(4'-n-Hexyloxycarbonyl-1'-methyl-cyclohex-1'-yl)-4-methoxyphenol
2,5-Bis-(4'-methoxycarbonyl-1'-methyl-cyclohex-1'-yl)-4-methoxyphenol
2,5-Bis-(4'-n-hexyloxycarbonyl-1'-methyl-cyclohex-1'-yl)-4-methoxyphenol
2,5-Bis-(3',4'-bis-methoxycarbonyl-1'-methyl-cyclohex-1'-yl)-4-methoxyphenol
2- und 3-(3',4'-Bis-methoxycarbonyl-cyclohex-1'-yl)-4-methoxyphenol
2- und 3-(4'-Acetyl-1'-methyl-cyclohex-1'-yl)-4-methoxyphenol
2- und 3-(5'-Methoxycarbonyl-2'-methyl-pent-2'-yl)-4-methoxyphenol
2-(5'-n-Hexyloxycarbonyl-2'-methyl-pent-2'-yl)-4-methoxyphenol
2-(5'-n-Hexyloxycarbonyl-2'-methyl-pent-2'-yl)-5-methyl-4-methoxyphenol
5-(5'-Methoxycarbonyl-2'-methyl-pent-2'-yl)-2-methyl-4-methoxyphenol
5-(5'-n-Hexyloxycarbonyl-2'-methyl-pent-2'-yl)-2-t-butyl-4-methoxyphenol
2-(5'-n-Hexyloxycarbonyl-2'-methyl-pent-2'-yl)-5-t-butyl-4-methoxyphenol
5-(5'-n-Hexyloxycarbonyl-2'-methyl-pent-2'-yl)-2-t-butyl-4-methoxyphenol
5-(5'-n-Dodecyloxycarbonyl-2'-methyl-pent-2'-yl)-2-t-butyl-4-methoxyphenol
5-(5'-Methoxycarbonyl-2'-methyl-pent-2'-yl)-2-(1',1',3',3'-tetramethylbutyl)-4-methoxyphenol
2-(5'-Methoxycarbonyl-2'-methyl-pent-2'-yl)-5-(1',1',3',3'-tetramethylbutyl)-4-methoxyphenol
2,5-Bis-(5'-methoxycarbonyl-2'-methyl-pent-2'-yl)-4-methoxyphenol
2,6-Bis-(5'-methoxycarbonyl-2'-methyl-pent-2'-yl)-4-methoxyphenol
2,5-Bis-(5'-äthoxycarbonyl-2'-methyl-pent-2'-yl)-4-methoxyphenol
2,5-Bis-(5'-n-propyloxycarbonyl-2'-methyl-pent-2'-yl)-4-methoxyphenol
2-(5'-n-Hexyloxycarbonyl-2'-methyl-pent-2'-yl)-5-(3'-n-hexyloxycarbonyl-2'-methyl-prop-2'-yl)-4-methoxyphenol
2-(5'-n-Hexyloxycarbonyl-2'-methyl-pent-2'-yl)-5-(3'-n-octyloxycarbonyl-2'-methyl-prop-2'-yl)-4-methoxyphenol
2,5-Bis-(5'-iso-propyloxycarbonyl-2'-methyl-pent-2'-yl)-4-methoxyphenol
2,5-Bis-(5'-n-butyloxycarbonyl-2'-methyl-pent-2'-yl)-4-methoxyphenol
2,5-Bis-(5'-iso-butyloxycarbonyl-2'-methyl-pent-2'-yl)-4-methoxyphenol
2,5-Bis-(5'-n-pentyloxycarbonyl-2'-methyl-pent-2'-yl)-4-methoxyphenol
2,5-Bis-(5'-iso-pentyloxycarbonyl-2'-methyl-pent-2'-yl)-4-methoxyphenol
2,5-Bis-(5'-n-hexyloxycarbonyl-2'-methyl-pent-2'-yl)-4-methoxyphenol
2,5-Bis-(5'-n-heptyloxycarbonyl-2'-methyl-pent-2'-yl)-4-methoxyphenol
2,5-Bis-(5'-cyclohexyloxycarbonyl-2'-methyl-pent-2'-yl)-4-methoxyphenol
2,5-Bis-(5'-n-octyloxycarbonyl-2'-methyl-pent-2'-yl)-4-methoxyphenol
2,5-Bis-[5'-(2"-äthylhexyloxycarbonyl)-2'-methyl-pent-2'-yl]-4-methoxyphenol
2,5-Bis-(5'-n-dodecyloxycarbonyl-2'-methyl-pent-2'-yl)-4-methoxyphenol
2,5-Bis-(5'-n-hexadecyloxycarbonyl-2'-methyl-pent-2'-yl)-4-methoxyphenol
2,5-Bis-[5'-(2"-methoxyäthoxycarbonyl)-2'-methyl-pent-2'-yl]-4-methoxyphenol
2,5-Bis-[5'-(2"-n-butyloxyäthoxycarbonyl)-2'-methyl-pent-2'-yl]-4-methoxyphenol
2,5-Bis-[5'-(2"-cyclohexyloxyäthoxycarbonyl)-2'-methyl-pent-2'-yl]-4-methoxyphenol
2,5-Bis-[5'-(2"-allyloxyäthoxycarbonyl)-2'-methyl-pent-2'-yl]-4-methoxyphenol
2,5-Bis-[5'-(2"-benzyloxyäthoxycarbonyl)-2'-methyl-pent-2'-yl]-4-methoxyphenol
2,5-Bis-[5'-(2"-phenoxyäthoxycarbonyl)-2'-methyl-pent-2'-yl]-4-methoxyphenol
2,5-Bis-(5'-phenoxycarbonyl-2'-methyl-pent-2'-yl)-4-methoxyphenol
2,5-Bis-(5'-benzyloxycarbonyl-2'-methyl-pent-2'-yl)-4-methoxyphenol
2,5-Bis-(5'-tetrahydrofurfuryloxycarbonyl-2'-methyl-pent-2'-yl)-4-methoxyphenol
2,5-Bis-(5'-furfuryloxycarbonyl-2'-methyl-pent-2'-yl)-4-methoxyphenol
2,5-Bis-[5'-(tetrahydropyran-4"-yloxycarbonyl)-2'-methyl-pent-2'-yl]-4-methoxyphenol
2-(5'-n-Hexyloxycarbonyl-2'-methyl-pent-2'-yl)-5-(5'-methoxycarbonyl-2'-methyl-pent-2'-yl)-4-methoxyphenol
2,5-Bis-(5'-carboxy-2'-methyl-pent-2'-yl)-4-methoxyphenol sowie dessen Na-Salz.

2,5-Bis-(5′-methoxycarbonyl-2′-methyl-pent-2′-yl)-4-äthoxyphenol
2,5-Bis-(5′-äthoxycarbonyl-2′-methyl-pent-2′-yl)-4-äthoxyphenol
2,5-Bis-(5′-n-propyloxycarbonyl-2′-methyl-pent-2′-yl)-4-äthoxyphenol
2,5-Bis-(5′-n-butoxycarbonyl-2′-methyl-pent-2′-yl)-4-äthoxyphenol
2-(5′-n-Hexyloxycarbonyl-2′-methyl-pent-2′-yl)-5-(3′-n-octyloxycarbonyl-2′-methyl-prop-2′-yl)-4-äthoxyphenol
2-(5′-n-Hexyloxycarbonyl-2′-methyl-pent-2′-yl)-5-(3′-n-octyloxycarbonyl-2′-methyl-prop-2′-yl)-4-äthoxyphenol
2,5-Bis-(5′-iso-propyloxycarbonyl-2′-methyl-pent-2′-yl)-4-äthoxyphenol
2,5-Bis-(5′-n-butyloxycarbonyl-2′-methyl-pent-2′-yl)-4-äthoxyphenol
2,5-Bis-(5′-iso-butyloxycarbonyl-2′-methyl-pent-2′-yl)-4-äthoxyphenol
2,5-Bis-(5′-n-pentyloxycarbonyl-2′-methyl-pent-2′-yl)-4-äthoxyphenol
2,5-Bis-(5′-iso-pentyloxycarbonyl-2′-methyl-pent-2′-yl)-4-äthoxyphenol
2,5-Bis-(5′-n-hexyloxycarbonyl-2′-methyl-pent-2′-yl)-4-äthoxyphenol
2,5-Bis-(5′-n-heptyloxycarbonyl-2′-methyl-pent-2′-yl)-4-äthoxyphenol
2,5-Bis-(5′-cyclohexyloxycarbonyl-2′-methyl-pent-2′-yl)-4-äthoxyphenol
2,5-Bis-(5′-n-octyloxycarbonyl-2′-methyl-pent-2′-yl)-4-äthoxyphenol
2,5-Bis-[5′-(2″-äthylhexyloxycarbonyl)-2′-methyl-pent-2′-yl]-4-äthoxyphenol
2,5-Bis-(5′-n-dodecyloxycarbonyl-2′-methyl-pent-2′-yl)-4-äthoxyphenol
2,5-Bis-(5′-n-hexadecyloxycarbonyl-2′-methyl-pent-2′-yl)-4-äthoxyphenol
2,5-Bis-[5′-(2″-methoxyäthoxycarbonyl)-2′-methyl-pent-2′-yl]-4-äthoxyphenol
2,5-Bis-[5′-(2″-n-butyloxyäthoxycarbonyl)-2′-methyl-pent-2′-yl]-4-äthoxyphenol
2,5-Bis-[5′-(2″-cyclohexyloxyäthoxycarbonyl)-2′-methyl-pent-2′-yl]-4-äthoxyphenol
2,5-Bis-[5′-(2″-allyloxyäthoxycarbonyl)-2′-methyl-pent-2′-yl]-4-äthoxyphenol
2,5-Bis-[5′-(2″-benzyloxyäthoxycarbonyl)-2′-methyl-pent-2′-yl]-4-äthoxyphenol
2,5-Bis-[5′-(2″-phenoxyäthoxycarbonyl)-2′-methyl-pent-2′-yl]-4-äthoxyphenol
2,5-Bis-(5′-phenoxycarbonyl-2′-methyl-pent-2′-yl)-4-äthoxyphenol
2,5-Bis-(5′-benzyloxycarbonyl-2′-methyl-pent-2′-yl)-4-äthoxyphenol
2,5-Bis-(5′-tetrahydrofurfuryloxycarbonyl-2′-methyl-pent-2′-yl)-4-äthoxyphenol
2,5-Bis-(5′-furfuryloxycarbonyl-2′-methyl-pent-2′-yl)-4-äthoxyphenol
2,5-Bis-[5′-(tetrahydropyran-4″-yloxycarbonyl)-2′-methyl-pent-2′-yl]-4-äthoxyphenol
2-(5′-n-Hexyloxycarbonyl-2′-methyl-pent-2′-yl)-5-(5′-methoxycarbonyl-2′-methyl-pent-2′-yl)-4-äthoxyphenol
2,5-Bis-(5′-carboxy-2′-methyl-pent-2′-yl)-4-äthoxyphenol sowie dessen Na-Salz

2,5-Bis-(2′-methyl-6′-hydroxy-hex-2′-yl)-4-äthoxyphenol
2,5-Bis-(5′-methoxycarbonyl-2′-methyl-pent-2′-yl)-4-n-butoxyphenol
2,5-Bis-(5′-äthoxycarbonyl-2′-methyl-pent-2′-yl)-4-n-butoxyphenol
2,5-Bis-(5′-n-propyloxycarbonyl-2′-methyl-pent-2′-yl)-4-n-butoxyphenol
2-(5′-n-Hexyloxycarbonyl-2′-methyl-pent-2′-yl)-5-(3′-n-hexyloxycarbonyl-2′-methyl-prop-2′-yl)-4-n-butoxyphenol
2-(5′-n-Hexyloxycarbonyl-2′-methyl-pent-2′-yl)-5-(3′-n-octyloxycarbonyl-2′-methyl-prop-2′-yl)-4-n-butoxyphenol
2,5-Bis-(5′-iso-propyloxycarbonyl-2′-methyl-pent-2′-yl)-4-n-butoxyphenol
2,5-Bis-(5′-n-butyloxycarbonyl-2′-methyl-pent-2′-yl)-4-n-butoxyphenol
2,5-Bis-(5′-iso-butyloxycarbonyl-2′-methyl-pent-2′-yl)-4-n-butoxyphenol
2,5-Bis-(5′-n-pentyloxycarbonyl-2′-methyl-pent-2′-yl)-4-n-butoxyphenol
2,5-Bis-(5′-iso-pentyloxycarbonyl-2′-methyl-pent-2′-yl)-4-n-butoxyphenol
2,5-Bis-(5′-n-hexyloxycarbonyl-2′-methyl-pent-2′-yl)-4-n-butoxyphenol
2,5-Bis-(5′-n-heptyloxycarbonyl-2′-methyl-pent-2′-yl)-4-n-butoxyphenol
2,5-Bis-(5′-cyclohexyloxycarbonyl-2′-methyl-pent-2′-yl)-4-n-butoxyphenol
2,5-Bis-(5′-n-octyloxycarbonyl-2′-methyl-pent-2′-yl)-4-n-butoxyphenol
2,5-Bis-[5′-(2″-äthylhexyloxycarbonyl)-2′-methyl-pent-2′-yl]-4-n-butoxyphenol
2,5-Bis-(5′-n-dodecyloxycarbonyl-2′-methyl-pent-2′-yl)-4-n-butoxyphenol
2,5-Bis-(5′-n-hexadecyloxycarbonyl-2′-methyl-pent-2′-yl)-4-n-butoxyphenol
2,5-Bis-[5′-(2″-methoxyäthoxycarbonyl)-2′-methyl-pent-2′-yl]-4-n-butoxyphenol
2,5-Bis-[5′-(2″-n-butyloxyäthoxycarbonyl)-2′-methyl-pent-2′-yl]-4-n-butoxyphenol
2,5-Bis-[5′-(2″-cyclohexyloxyäthoxycarbonyl)-2′-methyl-pent-2′-yl]-4-n-butoxyphenol
2,5-Bis-[5′-(2″-allyloxyäthoxycarbonyl)-2′-methyl-pent-2′-yl]-4-n-butoxyphenol
2,5-Bis-[5′-(2″-benzyloxyäthoxycarbonyl)-2′-methyl-pent-2′-yl]-4-n-butoxyphenol
2,5-Bis-[5′-(2″-phenoxyäthoxycarbonyl)-2′-methyl-pent-2′-yl]-4-n-butoxyphenol
2,5-Bis-(5′-phenoxycarbonyl-2′-methyl-pent-2′-yl)-4-n-butoxyphenol
2,5-Bis-(5′-benzyloxycarbonyl-2′-methyl-pent-2′-yl)-4-n-butoxyphenol
2,5-Bis-(5′-tetrahydrofurfuryloxycarbonyl-2′-methyl-pent-2′-yl)-4-n-butoxyphenol
2,5-Bis-(5′-furfuryloxycarbonyl-2′-methyl-pent-2′-yl)-4-n-butoxyphenol

2,5-Bis-[5'-(tetrahydropyran-4"-yloxycarbonyl)-2'-methyl-pent-2'-yl]-4-n-butoxyphenol
2-(5'-n-Hexyloxycarbonyl-2'-methyl-pent-2'-yl)-5-(5'-methoxycarbonyl-2'-methyl-pent-2'-yl)-4-n-butoxyphenol
2,5-Bis-(5'-carboxy-2'-methyl-pent-2'-yl)-4-n-butoxyphenol sowie dessen Na-Salz

2,5-Bis-(5'-methoxycarbonyl-2'-methyl-pent-2'-yl)-4-n-hexyloxyphenol
2,5-Bis-(5'-methoxycarbonyl-2'-methyl-pent-2'-yl)-4-cyclohexyloxyphenol
2,5-Bis-(5'-methoxycarbonyl-2'-methyl-pent-2'-yl)-4-(2'-n-butoxyäthoxy)-phenol
2,5-Bis-(5'-methoxycarbonyl-2'-methyl-pent-2'-yl)-4-(2'-äthylhexyloxy)-phenol
2,5-Bis-(5'-methoxycarbonyl-2'-methyl-pent-2'-yl)-4-n-dodecyloxyphenol
2,5-Bis-(5'-methoxycarbonyl-2'-methyl-pent-2'-yl)-4-n-hexadecyloxyphenol
2,5-Bis-(5'-methoxycarbonyl-2'-methyl-pent-2'-yl)-4-benzyloxyphenol
2,5-Bis-(5'-methoxycarbonyl-2'-methyl-pent-2'-yl)-4-phenoxyphenol
2,5-Bis-(5'-cyano-2'-methyl-pent-2'-yl)-4-methoxyphenol
2,5-Bis-(5'-carbamoyl-2'-methyl-pent-2'-yl)-4-methoxyphenol
2,5-Bis-(5'-N-n-butylcarbamoyl-2'-methyl-pent-2'-yl)-4-methoxyphenol
2,5-Bis-(5'-N-N-dimethylcarbamoyl-2'-methyl-pent-2'-yl)-4-methoxyphenol
2,5-Bis-(5'-N-n-octylcarbamoyl-2'-methyl-pent-2'-yl)-4-methoxyphenol
2,5-Bis-(5'-N,N-di-n-butylcarbamoyl-2'-methyl-pent-2'-yl)-4-methoxyphenol
2,5-Bis-(5'-N-eicosylcarbamoyl-2'-methyl-pent-2'-yl)-4-methoxyphenol
2,5-Bis-(5'-N-allylcarbamoyl-2'-methyl-pent-2'-yl)-4-methoxyphenol
2,5-Bis-(5'-N-cyclohexylcarbamoyl-2'-methyl-pent-2'-yl)-4-methoxyphenol
2,5-Bis-(5'-N-benzylcarbamoyl-2'-methyl-pent-2'-yl)-4-methoxyphenol
2,5-Bis-(5'-N-phenylcarbamoyl-2'-methyl-pent-2'-yl)-4-methoxyphenol
2,5-Bis-(5'-morpholinocarbamoyl-2'-methyl-pent-2'-yl)-4-methoxyphenol
2-(7'-Methoxycarbonyl-2'2'4'-trimethyl-hept-4'-yl)-5-t-butyl-4-methoxyphenol
2-(1',7'-Di-methoxycarbonyl-4'-methyl-hept-4'-yl)-4-methoxyphenol
2,5-Bis-(2'-methyl-6'-hydroxy-hex-2'-yl)-4-methoxyphenol
2,5-Bis-(2',6'-dimethyl-8'-hydroxy-oct-2'-yl)-4-methoxyphenol
2,5-Bis-(8'-acetyloxy-2',6'-dimethyl-oct-2'-yl)-4-methoxyphenol
2,5-Bis-(2',6'-dimethyl-8'-propionyloxy-oct-2'-yl)-4-methoxyphenol
2,5-Bis-(8'-butyryloxy-2',6'-dimethyl-oct-2'-yl)-4-methoxyphenol
2,5-Bis-(2',6'-dimethyl-8'-hexanoyloxy-oct-2'-yl)-4-methoxyphenol
2-(5'-Diäthylphosphono-5"-äthoxycarbonyl-2'-methyl-pent-2'-yl)-4-methoxyphenol
2,5-Bis-(5',5'-diäthoxycarbonyl-2'-methyl-pent-2'-yl)-4-methoxyphenol
2,5-Bis-(2',6'-dimethyl-8-eicosanoyloxy-oct-2'-yl)-4-methoxyphenol
2,5-Bis-(8'-crotonoyloxy-2',6'-dimethyl-oct-2'-yl)-4-methoxyphenol
2,5-Bis-(8'-benzoyloxy-2',6'-dimethyl-oct-2'-yl)-4-methoxyphenol
2,5-Bis-(2',6'-dimethyl-8'-phenacetyloxy-oct-2'-yl)-4-methoxyphenol
2,5-Bis-(8'-cyclohexylcarbonyl-2',6'-dimethyl-oct-2'-yl)-4-methoxyphenol
2,5-Bis-(8'-methoxy-2',6'-dimethyl-oct-2'-yl)-4-methoxyphenol
2,5-Bis-(8'-butoxy-2',6'-dimethyl-oct-2'-yl)-4-methoxyphenol
2,5-Bis-(8'-pentadecyloxy-2',6'-dimethyl-oct-2'-yl)-4-methoxyphenol
2- und 3-(8'-Amino-2',6'-dimethyl-oct-2'-yl)-4-methoxyphenol
2,5-Bis-(6'-amino-2'-methyl-hept-2'-yl)-4-methoxyphenol sowie dessen Hydrochlorid

2,5-Bis-(6'-N-methylamino-2'-methyl-hept-2'-yl)-4-methoxyphenol
2,5-Bis-(6'-N,N-dimethylamino-2'-methyl-hept-2'-yl)-4-methoxyphenol
2,5-Bis-(6'-N-äthylamino-2'-methyl-hept-2'-yl)-4-methoxyphenol
2,5-Bis-(6'-N,N-diäthylamino-2'-methyl-hept-2'-yl)-4-methoxyphenol
2,5-Bis-(6'-N-n-butylamino-2'-methyl-hept-2'-yl)-4-methoxyphenol
2,5-Bis-(6'-N,N-di-n-butylamino-2'-methyl-hept-2'-yl)-4-methoxyphenol
2,5-Bis-(2'-methyl-6'-morpholino-hept-2'-yl)-4-methoxyphenol
2- und 3-(6'-acetamido-2'-methyl-hept-2'-yl)-4-methoxyphenol
2,5-Bis-(6'-acetamido-2'-methyl-hept-2'-yl)-4-methoxyphenol
2,5-Bis-(6'-hexanamido-2'-methyl-hept-2'-yl)-4-methoxyphenol
2,5-Bis-(12'-amino-2',13'-dimethyl-tetradec-2'-yl)-4-methoxyphenol
2,5-Bis-(12'-amino-3',13'-dimethyl-tetradec-3'-yl)-4-methoxyphenol
2,5-Bis-(12'-acetamido-2',13'-dimethyl-tetradec-2'-yl)-4-methoxyphenol
2,5-Bis-(12'-acetamido-3',13'-dimethyl-tetradec-3'-yl)-4-methoxyphenol
2-(2'-Methyl-4'-phosphono-but-2'-yl)-4-methoxyphenol sowie deren Na-Salze.

2-(2'-Methyl-4'-dimethylphosphono-but-2'-yl)-4-methoxyphenol
2-(4'-Diäthylphosphono-2'-methyl-but-2'-yl)-4-methoxyphenol
2-(4'-Di-n-butylphosphono-2'-methyl-but-2'-yl)-4-methoxyphenol

5-t-Butyl-2-(2′-methyl-4′-dimethylphosphono-but-2′-yl)-4-methoxyphenol
5-(1′,1′,3′,3′-Tetramethylbutyl)-2-(2′-methyl-4′-dimethylphosphono-but-2′-yl)-4-methoxyphenol
2,5-Bis-(2′-methyl-4′-dimethylphosphono-but-2′-yl)-4-methoxyphenol
2,5-Bis-(2′-methyl-4′-diäthylphosphono-but-2′-yl)-4-methoxyphenol
2,5-Bis-(2′-methyl-4′-di-n-propylphosphono-but-2′-yl)-4-methoxyphenol
2-(5′-n-Hexyloxycarbonyl-2′-methyl-pent-2′-yl)-5-(2′-methyl-4′-diäthylphosphono-but-2′-yl)-4-methoxyphenol
2,5-Bis-(2′-methyl-4′-di-iso-propylphosphono-but-2′-yl)-4-methoxyphenol
2,5-Bis-(2′-methyl-4′-di-n-butyl-phosphono-but-2′-yl)-4-methoxyphenol
2,5-Bis-[2′-methyl-4′-(di-2″-äthylhexyl-phosphono)-but-2′-yl]-4-methoxyphenol
2,5-Bis-(2′-methyl-4′-di-n-dodecylphosphono-but-2′-yl)-4-methoxyphenol
2,5-Bis-[2′-methyl-4′-di-(2″-oxo-1″,3″,2″-dioxaphospholan-2″-yl)-but-2′-yl]-4-methoxyphenol
2,5-Bis-[2′-methyl-4′-(4″-methyl-2″-oxo-1″,3″,2″-dioxaphospholan-2″-yl)-but-2′-yl]-4-methoxyphenol
2,5-Bis-[4′-(äthyl-äthylphosphino)-2′-methyl-but-2′-yl]-4-methoxyphenol
2,5-Bis-(5′-äthoxycarbonyl-5′-diäthylphosphono-2′-methyl-pent-2′-yl)-4-methoxyphenol
2,5-Bis-(2′-methyl-3′-sulfo-prop-2′-yl)-4-methoxyphenol
2-(2′-Methyl-3′-sulfonamido-prop-2′-yl)-4-methoxyphenol
5-t-Butyl-2-(2′-methyl-3′-sulfonamido-prop-2′-yl)-4-methoxyphenol
2-(2′-Methyl-3′-N-methylsulfonamido-prop-2′-yl)-4-methoxyphenol
5-(1′,1′,3′,3′-Tetramethylbutyl)-2-(2′-methyl-3′-N-methylsulfonamido-prop-2′-yl)-4-methoxyphenol
2-(2′-Methyl-3′-N,N-di-n-butylsulfonamido-prop-2′-yl)-4-methoxyphenol
2-(2′-Methyl-3′-N-n-octylsulfonamido-prop-2′-yl)-4-methoxyphenol
5-t-Butyl-2-(2′-methyl-3′-N-n-octylsulfonamido-prop-2′-yl)-4-methoxyphenol
2-(7′-Cyano-2′,6′-dimethyl-hept-2′-yl)-4-methoxyphenol
2-(5′-Methoxycarbonyl-2′-methyl-pent-2′-yl)-1,4-dimethoxybenzol
2-(5′-n-Hexyloxycarbonyl-2′-methyl-pent-2′-yl)-1,4-dimethoxybenzol
2-Methyl-5-(5′-methoxycarbonyl-2′-methyl-pent-2′-yl)-1,4-dimethoxybenzol
2-t-Butyl-5-(5′-methoxycarbonyl-2′-methyl-pent-2′-yl)-1,4-dimethoxybenzol
2-(5′-Methoxycarbonyl-2′-methyl-pent-2′-yl)-5-(1′,1′,3′,3′-tetramethylbutyl)-1,4-dimethoxybenzol
2,5-Bis-(5′-methoxycarbonyl-2′-methyl-pent-2′-yl)-1,4-dimethoxybenzol
2,5-Bis-(5′-äthoxycarbonyl-2′-methyl-pent-2′-yl)-1,4-dimethoxybenzol
2,5-Bis-(5′-n-propyloxycarbonyl-2′-methyl-pent-2′-yl)-1,4-dimethoxybenzol
2,5-Bis-(5′-iso-propyloxycarbonyl-2′-methyl-pent-2′-yl)-1,4-dimethoxybenzol
2,5-Bis-(5′-n-butoxycarbonyl-2′-methyl-pent-2′-yl)-1,4-dimethoxybenzol
2,5-Bis-(5′-n-hexyloxycarbonyl-2′-methyl-pent-2′-yl)-1,4-dimethoxybenzol
2,5-Bis-[5′-(2″-äthylhexyloxycarbonyl)-2′-methyl-pent-2′-yl]-1,4-dimethoxybenzol
2,5-Bis-(5′-n-butoxyäthoxycarbonyl-2′-methyl-pent-2′-yl)-1,4-dimethoxybenzol
2,5-Bis-(5′-tetrahydrofurfuryloxycarbonyl-2′-methyl-pent-2′-yl)-1,4-dimethoxybenzol
2,5-Bis-(5′-carboxy-2′-methyl-pent-2′-yl)-1,4-dimethoxybenzol
2,5-Bis-(4′-keto-2′-methyl-pent-2′-yl)-1,4-dimethoxybenzol
2-(5′-Methoxycarbonyl-2′-methyl-pent-2′-yl)-1,4-diäthoxybenzol
2-(5′-n-Hexyloxycarbonyl-2′-methyl-pent-2′-yl)-1,4-diäthoxybenzol
2-Methyl-5-(5′-methoxycarbonyl-2′-methyl-pent-2′-yl)-1,4-diäthoxybenzol
2-t-Butyl-5-(5′-methoxycarbonyl-2′-methyl-pent-2′-yl)-1,4-diäthoxybenzol
2-(5′-Methoxycarbonyl-2′-methyl-pent-2′-yl)-5-(1′,1′,3′,3′-tetramethylbutyl)-1,4-diäthoxybenzol
2,5-Bis-(5′-methoxycarbonyl-2′-methyl-pent-2′-yl)-1,4-diäthoxybenzol
2,5-Bis-(5′-äthoxycarbonyl-2′-methyl-pent-2′-yl)-1,4-diäthoxybenzol
2,5-Bis-(5′-n-propyloxycarbonyl-2′-methyl-pent-2′-yl)-1,4-diäthoxybenzol
2,5-Bis-(5′-iso-propyloxycarbonyl-2′-methyl-pent-2′-yl)-1,4-diäthoxybenzol
2,5-Bis-(5′-n-butoxycarbonyl-2′-methyl-pent-2′-yl)-1,4-diäthoxybenzol
2,5-Bis-(5′-n-hexyloxycarbonyl-2′-methyl-pent-2′-yl)-1,4-diäthoxybenzol
2,5-Bis-[5′-(2″-äthylhexyloxycarbonyl)-2′-methyl-pent-2′-yl]-1,4-diäthoxybenzol
2,5-Bis-(5′-n-butoxyäthoxycarbonyl-2′-methyl-pent-2′-yl)-1,4-diäthoxybenzol
2,5-Bis-(5′-tetrahydrofurfuryloxycarbonyl-2′-methyl-pent-2′-yl)-1,4-diäthoxybenzol
2,5-Bis-(5′-carboxy-2′-methyl-pent-2′-yl)-1,4-diäthoxybenzol
1-Äthoxy-4-methoxy-2-(5′-methoxycarbonyl-2′-methyl-pent-2′-yl)-benzol
4-Äthoxy-1-methoxy-2-(5′-methoxycarbonyl-2′-methyl-pent-2′-yl)-benzol
2-t-Butyl-1-äthoxy-4-methoxy-5-(5′-methoxycarbonyl-2′-methyl-pent-2′-yl)-benzol
2-t-Butyl-4-äthoxy-1-methoxy-5-(5′-methoxycarbonyl-2′-methyl-pent-2′-yl)-benzol

1-Äthoxy-4-methoxy-5-(5′-methoxycarbonyl-2′-methyl-pent-2′-yl)-2-(1′,1′,3′,3′-tetramethylbutyl)-benzol
4-Äthoxy-1-methoxy-5-(5′-methoxycarbonyl-2′-methyl-pent-2′-yl)-2-(1′,1′,3′,3′-tetramethylbutyl)-benzol
1-Äthoxy-4-methoxy-2,5-bis-(5′-methoxycarbonyl-2′-methyl-pent-2′-yl)-benzol
1-Äthoxy-4-methoxy-2,5-bis-(5′-äthoxycarbonyl-2′-methyl-pent-2′-yl)-benzol
1-Äthoxy-4-methoxy-2,5-bis-(5′-n-hexyloxycarbonyl-2′-methyl-pent-2′-yl)-benzol
1-Äthoxy-4-methoxy-2,5-bis-(5′-n-butoxyäthoxycarbonyl-2′-methyl-pent-2′-yl)-benzol
1-Äthoxy-4-methoxy-2,5-bis-(5′-tetrahydrofurfuryl-oxycarbonyl-2′-methyl-pent-2′-yl)-benzol
1-Äthoxy-4-methoxy-2,5-bis-(5′-carboxy-2′-methyl-pent-2′-yl)-benzol
5-Hydroxy-6-(5′-methoxycarbonyl-2′-methyl-pent-2′-yl)-2,2-dimethyl-coumaran
5-Hydroxy-6-(5′-n-hexyloxycarbonyl-2′-methyl-pent-2′-yl)-2,2-dimethyl-coumaran
6-Hydroxy-7-(5′-methoxycarbonyl-2′-methyl-pent-2′-yl)-2,2-dimethyl-chroman
6-Hydroxy-7-(5′-n-hexyloxycarbonyl-2′-methyl-pent-2′-yl)-2,2-dimethyl-chroman
6-Hydroxy-7-(5′-methoxycarbonyl-2′-methyl-pent-2′-yl)-2,2,4-trimethyl-chroman
6-Hydroxy-7-(5′-n-hexyloxycarbonyl-2′-methyl-pent-2′-yl)-2,2,4-trimethyl-chroman
6-Hydroxy-7-(5′-methoxycarbonyl-2′-methyl-pent-2′-yl)-2,2-dimethyl-4-isopropyl-chroman
6-Hydroxy-7-(5′-n-hexyloxycarbonyl-2′-methyl-pent-2′-yl)-2,2-dimethyl-4-isopropyl-chroman
6-Hydroxy-7-(5′-methoxycarbonyl-2′-methyl-pent-2′-yl)-2-methyl-2-(4′,8′,12′-trimethyl-tridec-1′- yl)-chroman
Bis-[5-hydroxy-2-methoxy-4-(5′-methoxycarbonyl-2′-methyl-pent-2′-yl)-phenyl]-methan
Bis-[5-hydroxy-2-methoxy-4-(5′-n-hexyloxycarbonyl-2′-methyl-pent-2′-yl)-phenyl]-methan
Bis-[2-hydroxy-5-methoxy-3-(5′-methoxycarbonyl-2′-methyl-pent-2′-yl)-phenyl]-methan
Bis-2,2-[2′-hydroxy-5′-methoxy-4′-(5″-methoxycarbonyl-2″-methyl-pent-2″-yl)-phenyl]-propan
Bis-2,2-[2′-hydroxy-5′-methoxy-4′-(5″-n-hexyloxycarbonyl-2″-methyl-pent-2″-yl)-phenyl]-propan
Bis-[4-hydroxy-2,5-bis-(5′-methoxycarbonyl-2′-methyl-pent-2′-yl)-phenyl]-oxid
6-Hydroxy-2-[2′,5′-dihydroxy-4′-(5″-methoxycarbonyl-2″-methyl-pent-2″-yl)-phenyl]-7-(5′-methoxycarbonyl-2′-methyl-pent-2′-yl)-2,4,4-trimethyl-chroman
6,6′-Dihydroxy-7,7′-bis-(5″-methoxycarbonyl-2″-methyl-pent-2″-yl)-4,4,4′,4′-tetramethyl-2,2′-spiro-bis-chroman
5-(5′-n-Hexyloxycarbonyl-2′-methyl-pent-2′-yl)-2-cumyl-4-methoxy-phenol
Bis-[5-(2′-hydroxy-5′-methoxy-phenyl)-5-methyl-hexansäure]-ester des Hexan-1,6-diols
N,N′-[5-(2′-hydroxy-5′-methoxy-phenyl)-5-methyl-hexansäure]-amid des Hexan-1,6-diamins
1,4-Butyl-bis-[3′-(5″-methoxycarbonyl-2″-methyl-pent-2″-yl)-4′-hydroxy-phenyl]-äther.

Weitere nicht limitierende Beispiele von Verbindungen der Formel I, worin $R_0$ eine Gruppe der Formel IVa oder IVb darstellt, haben die Formel

$[J]-(CH_2)_4-[J]$

$[J]-CH_2-COO-(CH_2)_2OCOCH_2-[J]$

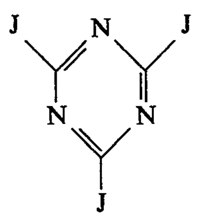

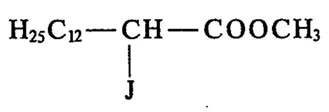

wobei J einen Rest der Formel

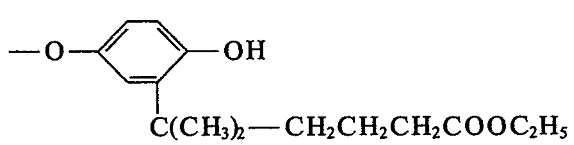

bedeutet.

Als nicht limitierende Beispiele von Verbindungen der Formel XIV (siehe Seite 32) seien genannt:

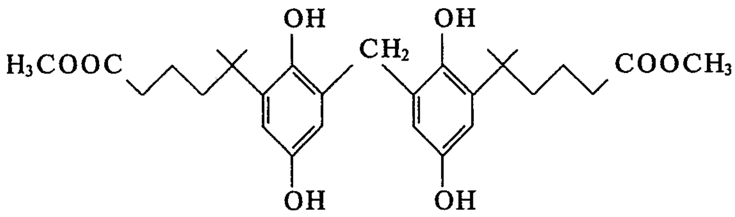

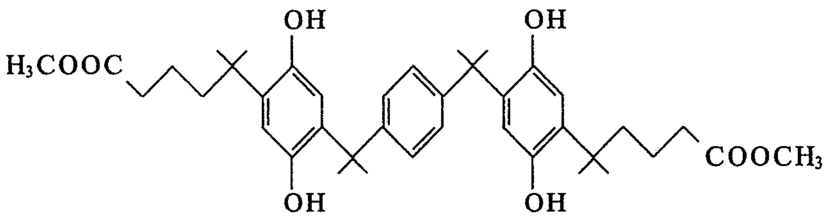

Gegenstand der Erfindung ist auch ein Verfahren zur Herstellung von Verbindungen der Formel I, indem man in Gegenwart eines sauren Katalysators eine Verbindung der Formel X

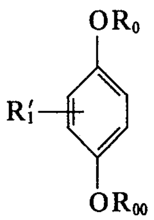

(X)

mit einem funktionellen Alkylierungsmittel, das zur Einführung einer oder zwei Gruppen der Formel II oder eine Gruppe der Formel IX in einen oder zwei aromatischen Ringe der Verbindung der Formel X befähigt ist, umsetzt. Dabei stellt $R'_1$ –H, $C_{1-8}$-Alkyl oder $C_{7-9}$-Aralkyl dar, und $R_0$ und $R_{00}$ haben die oben angegebene Bedeutung oder $R'_1$ und $R_0$ bilden zusammen einen Rest der Formel V, wie oben definiert.

Die Alkylierung wird zweckmäßig bei einer Temperatur zwischen 20° und 150 °C, bevorzugt zwischen 20° und 100°C, durchgeführt. Der saure Katalysator kann eine Brønsted- oder Lewis-Säure oder eine aktive Erde sein. Für diesen Zweck geeignete Brønsted-Säuren können organisch oder anorganisch oder teilweise Salze davon sein. In Betracht kommen z. B. anorganische Mineralsäuren, wie Chlorwasserstoffsäure, Schwefel-, Perchlor- und Orthophosphorsäure; alkyl-, aryl- oder aralkyl-substituierte anorganische Säuren, wie Methan- und Äthansulfonsäure, Benzolsulfonsäure, p-Toluolsulfonsäure und Methanphosphonsäure; organische Säuren, wie Dichloressigsäure, Trichloressigsäure und Trifluoressigsäure. Für die Alkylierung geeignete Lewis-Säuren sind z. B. Bortrifluorid, Eisen(III)chlorid, Aluminiumchlorid und Zinn(IV)chlorid. Für die Alkylierung geeignete aktive Erden sind z. B. Fumont® 237 und Fulcat® 22.

Die oben beschriebenen Katalysatoren können allein oder mit Lösungsmitteln oder Gemischen davon verwendet werden. Geeignete Lösungsmittel sind beispielsweise Äther, Wasser, Methanol, Essigsäure, Benzol und Nitromethan. Bevorzugte Katalysatoren sind Schwefelsäure, enthaltend Methanol, p-Toluolsulfonsäure und Aluminiumchlorid in Kombination mit Nitromethan.

Verbindungen der Formel I, worin q 0 oder 1 und p 1 oder 2 sind, können aus 1 Mol der Verbindung der Formel X, worin $R_0$ und $R_{00}$ die oben angegebene Bedeutung haben, bzw. aus einer Verbindung der Formel X, worin $R_0$, $R_{00}$ und R' die oben angegebene Bedeutung haben, und 0,1 bis 2 Mol eines Alkylierungsmittels, das zur Einführung eines Restes der Formel II in mindestens einen aromatischen Kern der Verbindung der Formel X befähigt ist, erhalten werden.

Verbindungen der Formel I, worin p und q 1 sind, können durch Reaktion von einem Mol einer Verbindung der Formel XI

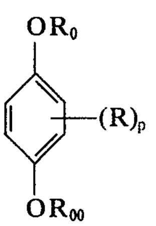

(XI)

worin p 1 ist, R den Rest der Formel II bedeutet und $R_0$ und $R_{00}$ die oben angegebene Bedeutung haben, mit mindestens einem Mol eines Alkylierungsmittels, das zur Einführung eines Restes der Formel II oder IX in den Kern der Verbindung der Formel XI befähigt ist, in Gegenwart eines sauren Katalysators hergestellt werden.

Verbindungen der Formel Ic

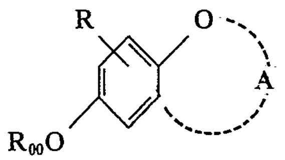

(Ic)

worin A für einen Kohlenstoffrest mit 4 bis 20 C-Atomen steht, der ein Chroman- oder Coumaransystem bildet, können durch Reaktion von einem Mol einer Verbindung der Formel XA

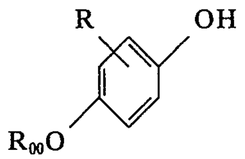

(XA)

worin R und $R_{00}$ die oben angegebene Bedeutung haben, mit mindestens einem Mol eines Alkylierungsmittels, das zur Einführung des Rests A in den Kern der Verbindung der Formel XA befähigt ist, in Gegenwart eines sauren Katalysators hergestellt werden.

Die für diesen Zweck befähigten Alkylierungsmittel haben zwei reaktive Zentren und sind beispielsweise Diene, Diole, Hydroxyolefine oder halogenierte Olefine, wie z. B. Isopren, 2-Methyl-penta-2,4-dien, 2-Methyl-hexa-2,4-dien, 2,5-Dimethyl-hexa-2,4-dien, Methallylalkohol, Methallylchlorid, Prenylalkohol, Phytol, 2-Phenylpentan-2,4-diol, 2,5-Dimethyl-hexan-1,5-diol.

Verbindungen der Formel Id

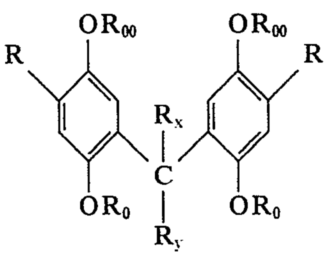

(Id)

worin R, $R_0$ und $R_{00}$ die oben angegebene Bedeutung haben und $R_x$ und $R_y$ unabhängig voneinander —H oder $C_{1-4}$-Alkyl sind, können durch Reaktion von einem Mol einer Verbindung der Formel XIA

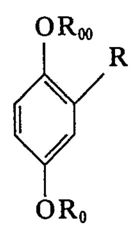

(XIA)

worin R, $R_0$ und $R_{00}$ die oben angegebene Bedeutung haben, mit bis zu 0,5 Mol eines Aldehyds oder eines Ketons, enthaltend 1 bis 9 C-Atome, in Gegenwart eines sauren Katalysators hergestellt werden.

Verbindungen der Formel Ie

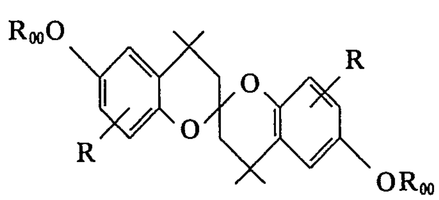

(Ie)

worin R und $R_{00}$ die oben angegebene Bedeutung haben, können durch Reaktion von einem Mol einer Verbindung der Formel XA mit mindestens 1,5 Mol Aceton oder mit bis zu 0,5 Mol Phoron hergestellt werden.

Verbindungen der Formel If

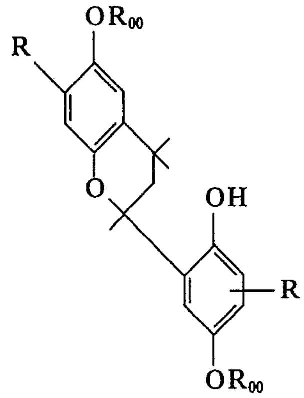

(If)

worin R und $R_{00}$ die oben angegebene Bedeutung haben, können durch Reaktion von einem Mol einer Verbindung der Formel XA mit mindestens einem Mol Aceton oder mit bis zu 0,5 Mol Mesityloxid in Gegenwart eines sauren Katalysators hergestellt werden.

Die Verbindungen der Formel I, worin $R_1$ $C_{1-8}$-Alkyl, $C_{7-9}$-Aralkyl oder ein Rest der Formel II oder III ist, eines von $R_0$ oder $R_{00}$ —H und das andere $R_0$ oder $R_{00}$ unsubstituiertes oder durch ein bis fünf Sauerstoffatome unterbrochenes $C_{1-20}$-Alkyl, ferner $C_{3-12}$-Cycloalkyl, $C_{6-10}$-Aryl oder $C_{7-13}$-Aralkyl sind, und R die oben angegebene Bedeutung hat, können durch Reaktion einer Verbindung der Formel XII

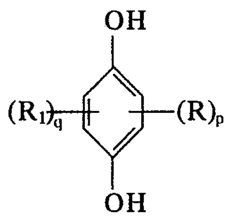

(XII)

in Gegenwart eines Chinons, vorzugsweise eines analogen Chinons der Formel XIII

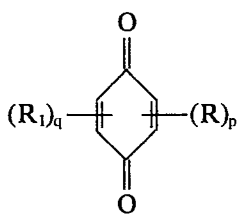

(XIII)

worin in den Formeln XII und XIII $R_1$ $C_{1-8}$-Alkyl, $C_{7-9}$-Aralkyl oder ein Rest der Formel II oder III ist und R, p und q die oben angegebene Bedeutung haben, mit einer Verbindung der Formel $R_0$ —OH oder $R_{00}$ —OH, worin $R_0$ und $R_{00}$ unabhängig voneinander gegebenenfalls durch ein bis fünf O-Atome unterbrochenes $C_{1-20}$-Alkyl, $C_{3-12}$-Cycloalkyl, $C_{6-10}$-Aryl oder $C_{7-13}$-Aralkyl bedeuten, in saurem Medium hergestellt werden. Weitere Einzelheiten über diesen Prozeß sind in der GB-Patentschrift Nr. 1 557 237 beschrieben.

Verbindungen der Formel I, worin eines von $R_0$ und $R_{00}$ —H ist, werden durch Reaktion der analogen Chinone der Formel XIII mit einer Verbindung der Formel $P(OR_0)_3$ oder $P(OR_{00})_3$, worin $R_0$ und $R_{00}$ unabhängig voneinander gegebenenfalls durch ein bis fünf O-Atome unterbrochenes $C_{1-20}$-Alkyl, ferner $C_{3-12}$-Cycloalkyl, $C_{6-10}$-Aryl oder $C_{7-13}$-Aralkyl bedeuten, und anschließende Hydrolyse der so erhaltenen Phosphate gemäß der in J. Am. Chem. Soc. 87, 4338 (1959), beschriebenen Methode von F. Ramirez hergestellt.

Weiterhin können die Verbindungen der Formel I, worin eines von $R_0$ und $R_{00}$ kein Wasserstoff ist, durch Reaktion einer Verbindung der Formel I, worin $R_0$ und $R_{00}$ beide —H oder eines —H sind, mit einer Verbindung der Formel $R_0$ —OH oder $R_{00}$ —OH in saurem Medium oder mit einer Verbindung der Formel $R_0$—X (oder $R_{00}$—X) oder $(R_0)_2SO_4$ [oder $(R_{00})_2SO_4$] in alkalischem Medium hergestellt werden. Dabei stehen $R_0$ und $R_{00}$ unabhängig voneinander für gegebenenfalls durch ein bis fünf O-Atome unterbrochenes $C_{1-20}$-Alkyl, ferner $C_{3-12}$-Cycloalkyl, $C_{6-10}$-Aryl oder $C_{7-13}$-Aralkyl und X für Halogen.

Verbindungen der Formel XII, worin $R_1$ $C_{7-9}$-Aralkyl, sind neu. Sie können durch Reaktion eines Hydrochinons der Formel X ($R_0$ und $R_{00}$ bedeuten beide —H und $R_1'$ —H oder $C_{1-8}$-Alkyl oder $C_{7-9}$-Aralkyl) mit einem funktionellen Alkylierungsmittel in Gegenwart eines sauren Katalysators hergestellt werden, wobei das Alkylierungsmittel zur Einführung von p Mol eines Restes R, wie oben definiert, befähigt ist.

Verbindungen der Formel XII, welche einen Rest der Formel III enthalten, sind neu und entsprechen der Formel XIV

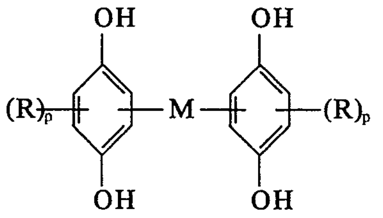

(XIV)

in welcher R, M und p die oben angegebene Bedeutung haben.

Verbindungen der Formel XIV sind als Stabilisatoren für Magentakuppler wirksam.

Verbindungen der Formel XIV können durch an und für sich gut bekannte Methoden hergestellt werden, beispielsweise durch Kondensation von 2 Mol einer Verbindung der Formel XII, worin q = o, mit einem Mittel, das zur Einführung der Brücke —M— befähigt ist.

Verbindungen der Formel XIII sind auch neue Verbindungen und können durch Oxidation der Verbindungen der Formel XII erhalten werden. Geeignete Oxidationsmittel sind z. B. Salze von elektronenanziehenden Verbindungen mit einem geeigneten Redoxpotential, wie Hypohalogenite, z. B. Natriumhypochlorit; Perhalogenate, z. B. Natriumperchlorat; Salpetersäure und Oxide des Stickstoffs; Chromtrioxid, Chromate, Jones Reagenz; Sauerstoff und Luft, gegebenenfalls katalysiert durch Metallsalze, wie Kupfersalze. Es können auch organische Oxidationsmittel verwendet werden, z. B. Chloranil.

Die Herstellung der Verbindung der Formel I, worin $R_0$ eine Gruppe der Formel IVa oder IVb ist, können durch Reaktion von 2 bis 3 Mol einer Verbindung der Formel XIIA:

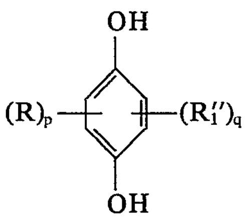

(XIIA)

worin R, $R_1''$, p und q die oben angegebene Bedeutung haben, mit einem entsprechenden Di- oder Trihalogenid Hal-B(Hal)$_m$, Epichlorhydrin, Epibromhydrin oder mit einem Diglycidyläther

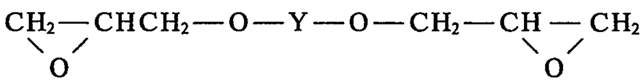

in Gegenwart einer Base, hergestellt werden, oder durch Reaktion einer Verbindung der Formel XIIA mit einem Halogenid Hal—CH($R_{16}$)—($C_rH_{2r}$)—CO—D in Gegenwart einer Base hergestellt werden, wobei m, r, B, D, Y und $R_{16}$ die oben angegebene Bedeutung haben und Hal Chlor und insbesondere Brom bedeutet.

Die Verbindungen der Formel XIIA können nach dem bereits für die Herstellung der Verbindungen der Formel XII oben beschriebenen Verfahren hergestellt werden, indem eine Verbindung der Formel X, worin $R_0$ und $R_{00}$ beide —H sind, mit einem funktionellen Alkylierungsmittel umgesetzt wird, das zur Einführung eines oder zwei Reste der Formel II befähigt ist.

Als nicht limitierende Beispiele von Verbindungen der Formel X seien genannt:

Hydrochinon-monomethyläther, Hydrochinon-monoäthyläther, Hydrochinon-mono-n-propyläther, Hydrochinon-mono-iso-propyläther, Hydrochinon-mono-n-butyläther, Hydrochinon-mono-sec-butyläther, Hydrochinon-mono-n-pentyläther, Hydrochinon-mono-n-hexyläther, Hydrochinon-mono-n-octyläther, Hydrochinon-mono-2-äthylhexyläther, Hydrochinon-mono-dodecyläther, Hydrochinon-mono-hexadecyläther, Hydrochinon-mono-cyclohexyläther, Hydrochinon-mono-n-butoxyäthyläther, Hydrochinon-mono-benzyläther, Hydrochinon-mono-phenyläther, 2-Methyl-4-methoxyphenol, 2-Äthyl-4-methoxyphenol, 2-iso-Propyl-4-methoxyphenol, 2-t-Butyl-4-methoxyphenol, 2-(1′,1′,3′,3′-tetramethylbutyl)-4-methoxyphenol, 1,4-Dimethoxybenzol, 2-Methyl-1,4-dimethoxybenzol, 2-t-Butyl-1,4-dimethoxybenzol, 1,4-Diäthoxybenzol, 2-Methyl-1,4-diäthoxybenzol, 2-t-Butyl-1,4-diäthoxybenzol, 1,4-Di-n-propoxybenzol, 1,4-Di-n-butoxybenzol, 2-t-Butyl-1-äthoxy-4-methoxybenzol.

Die funktionellen Alkylierungsmittel, die einen Rest der Formel II einführen können und mit den Hydrochinonen der Formel V umgesetzt werden, erhalten ein aktives Zentrum, z. B. eine olefinische Gruppe oder eine Hydroxylgruppe, die während der Alkylierungsreaktion eliminiert, verändert oder umgelagert wird.

Beispiele von funktionellen Olefinen, die sich zur Alkylierung von Verbindungen der Formeln X, XA und XI eignen, sind: 5-Methylhex-5-ensäure, 5-Methylhex-5-ensäuremethylester, 5-Methylhex-5-ensäureäthylester, 5-Methylhex-5-ensäure-n-propylester, -isopropylester, -n-butylester, -isobutylester,

-sek.-butylester, -n-pentylester, -2-methylbutylester, -2,2-dimethylpropylester, -n-hexylester, -cyclohexylester, -2-äthylhexylester, -n-octylester, -n-dodecylester oder -n-hexadecylester, 5,7,7-Trimethyloct-4-ensäuremethylester, 1,7-Dimethoxycarbonyl-4-methyl-hept-3-en, 4-Methoxycarbonyl-1-methylcyclohex-1-en, 4-Acetyl-1-methylcyclohex-1-en, 4,5-Bis-methoxycarbonyl-1-methylcyclohex-1-en, Dimethylprenylphosphonat, Diäthylprenylphosphonat, Di-n-propylprenylphosphonat, Diisopropylprenylphosphonat, Di-n-butylprenylphosphonat, Di-n-octylprenylphosphonat, Citronellol, Citronellylnitril, Citronellylacetat, Citronellyl-methyläther, Citronellyl-butyläther, 2-Amino-6-methyl-hept-5-en, 2-Amino-6-methyl-hept-6-en, Diäthyl-2-äthoxycarbonyl-5-methyl-hex-4-en-2-phosphonat, 2-Äthoxycarbonyl-5-methyl-hex-4-ensäureäthylester, 2-Acetamido-6-methyl-hept-5-en, 2-Acetamido-6-methyl-hept-6-en, 2-Methyl-1-propen-1-sulfonsäure, 2-Methyl-2-propen-1-sulfonsäureamid, N-n-Butyl-2-propen-1-sulfonsäureamid, N,N-Di-n-butyl-2-propen-1-sulfonsäureamid, N-n-Octyl-2-propen-1-sulfonsäureamid.

Beispiele von funktionellen Hydroxyverbindungen für die funktionelle Alkylierung von Verbindungen der Formeln X, XA und XI sind: 2-Amino-6-hydroxy-6-methylheptan, 2-Acetamido-6-hydroxy-6-methylheptan, 11-Amino-2,2,12-trimethyl-tridecan-1-ol, sowie aus 11-Aminoundecanolen der Formel

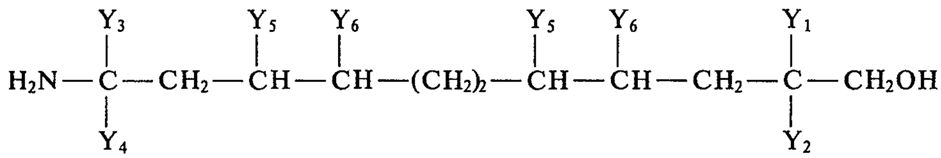

ausgewählte Verbindungen, worin $Y_1$ und $Y_3$ unabhängig voneinander Wasserstoff oder $C_{1-8}$-Alkyl, $Y_2$ und $Y_4$ unabhängig voneinander $C_{1-8}$-Alkyl und $Y_5$ und $Y_6$ unabhängig voneinander Wasserstoff oder $C_{1-4}$-Alkyl bedeuten. Derartige 11-Aminoundecanole und deren Herstellung sind ausführlich in der DE-OS 2 831 299 beschrieben.

Beispiele von für die Alkylierung von Verbindungen der Formel XI geeigneten Olefinen sind Isobutylen, Diisobutylen und $\alpha$-Methylstyren. Dabei stehen p für 1, $R_1$, $R_0$ und $R_{00}$ für die oben angegebene Bedeutung und R für einen Rest der Formel II.

Als Alkohole für die Alkylierung von Verbindungen der Formel XI mit $p = 1$ und $R_1$, $R_0$ und $R_{00}$ wie oben definiert eignen sich zum Beispiel tert.-Butanol, 1,1,3,3-Tetramethyl-1-butan-1-ol und Cumylalkohol. Die funktionellen Derivate der Formel I können auch in andere funktionelle Derivate übergeführt werden. Stellt Q z. B. $-COOR$ dar, so kann diese Gruppe mit einem Alkohol $R_4-OH$ zum entsprechenden Ester $-COOR_4$ umgesetzt werden. Estergruppen $Q = -COOR_4$ können durch Umesterung in andere Gruppen $R_4$ übergeführt werden, oder Estergruppen $-COOR_4$ können durch Behandlung mit Verbindungen $NH(R_4)(R_5)$, worin $R_4$ und $R_5$ die oben angegebene Bedeutung haben, in Amide $-CON(R_4)(R_5)$ übergeführt werden.

Mit der vorliegenden Erfindung wird die Einführung von verschiedenartigen funktionellen Resten in das Hydrochinonmolekül ermöglicht, so daß die photographische Wirkung dieses Hydrochinons optimal ist. Beispielsweise können die Polarität und/oder der Ballast in den Hydrochinonen der Formel I so eingestellt werden, daß sie für photographische Systeme eine wirkliche Kontrolle der Löslichkeit, Verträglichkeit und Beweglichkeit/Unbeweglichkeit ermöglichen. Mit derartigen Eigenschaften stellen die Verbindungen der Formel I wertvolle Zwischenprodukte für die Herstellung von photographisch komplizierteren Verbindungen dar. Ferner sind sie als photographische Stabilisatoren verwendbar, wobei sie im Gegensatz zu vorbekannten substituierten Hydrochinonen keinen schädlichen Effekt auf die Farbstoff-Ausbeute bewirken.

Die Verbindungen der Formel I wie auch Farbkuppler können in bekannter Weise in photographische Schichten eingearbeitet werden, z. B. in Silberhalogenidemulsionen, die Gelatine und/oder andere Bindemittel enthalten. Sie finden z. B. Anwendung in Silberbromid-, Silberchlorid- oder Silberjodidemulsionen oder in Emulsionen, die Gemische von Silberhalogeniden enthalten, wie Silberbromid/jodid- oder Silberchlorid/bromid-Emulsionen. Die Emulsionen können chemisch sensibilisiert werden und auch übliche organische Stabilisatoren wie gehinderte Amine, phenolische Komponenten wie gehinderte Phenole, Alkoxyphenole, Aryloxyphenole, Hydroxycoumarane, Hydroxychromane oder Dihydroxyspirochromane oder Hydrochinone und insbesondere Hydrochinone der Formel XII, wobei synergistische Effekte erzielt werden können, ferner UV-Absorber, optische Aufheller und photographisch wirksame Verbindungen, ferner Antischleiermittel, Verbindungen, welche photochemisch wirksame Produkte wie DIR-Verbindungen freisetzen können , sowie übliche Weichmacher, wie Glyzerin, enthalten. Die Emulsionen können auch mit für Gelatine üblichen Härtern gehärtet werden. Schließlich können die Emulsionen auch übliche Beschichtungshilfsmittel enthalten. Die Emulsionen können auf übliche Trägermaterialien für photographisches Bildmaterial appliziert werden. Gegebenenfalls kann ein Gemisch verschiedener Kolloide verwendet werden, um die Silberhalogenide zu dispergieren. Die Emulsionen können auch Kuppler enthalten, welche allein oder in Gemischen Farbstoffe während der Entwicklung erzeugen, z. B. Gelb-, Magenta-, Cyan- oder Schwarz-Farbstoffe.

Zum Entwickeln des Bildmaterials für Farbphotographie können übliche Entwicklungsbäder eingesetzt werden. Diese Bäder enthalten in der Regel eine Entwicklungssubstanz des p-Phenyldiamin-Typs, einen Entwicklungsverzögerer, wie Kaliumbromid, ein Antioxidans, wie Natriumsulfit, ein Salz der schwefligen Säure und/oder Hydroxylamin und eine Base, z. B. ein Alkalimetallhydroxid oder Alkalimetallcarbonat. Die Entwicklungsbäder können auch übliche Antischleiermittel, Komplexiermittel, Benetzungsmittel, optische Aufheller und sonstiges enthalten.

Entsprechende Anwendungsmöglichkeiten sind z. B. in den US-Patentschriften 2 304 939, 2 304 940, 2 322 027, 2 284 879, 2 801 170, 2 801 171, 2 749 360 und 2 825 382 beschrieben.

In den Beispielen sind Teile und Prozente Gewichtsteile bzw. Gewichtsprozente. Der Druck wird in Bar bzw. Millibar angegeben.

## Beispiel 1

a) 100 Teile 98%ige Schwefelsäure werden zu 32 Teilen Methanol bei einer Temperatur unter 10°C zugegeben. Dazu werden 35,5 Teile 5-Methylhex-5-ensäure-methylester und danach 12,4 Teile Hydrochinon-monomethyläther zugegeben. Nach Rühren während 24 Stunden bei Raumtemperatur wird die Reaktionsmischung in Wasser gegossen und die erhaltene ölige Schicht mit Diäthyläther extrahiert. Nach Waschen mit einer 2 N Natriumhydroxid-Lösung und dann mit Wasser wird die ätherische Phase eingedampft. Der erhaltene ölige Rückstand wird mit Petroläther (40—60°C), enthaltend ein wenig Diäthyläther, verdünnt. Man erhält 2,5-Bis-(5'-methoxycarbonyl-2'-methyl-pent-2'-yl)-4-methoxyphenol vom Schmelzpunkt 82—84°C und mit folgender Elementaranalyse:

| | | |
|---|---|---|
| Berechnet für $C_{23}H_{36}O_6$ | C 67,62 | H 8,88% |
| Gefunden | C 67,99 | H 9,16% |

b) Die alkalische wäßrige Phase, die nach dem Waschen der obigen ätherischen Phase erhalten wurde, wird 2 Stunden auf einem Wasserdampfbad erhitzt und dann angesäuert. Nach Filtration des festen Produktes und Waschen mit Wasser wird der Rückstand aus Eisessig/Wasser kristallisiert. Man erhält 2,5-Bis-(5'-carboxy-2'-methyl-pent-2'-yl)-4-methoxyphenol vom Smp. 177—99°C und mit folgender Elementaranalyse:

| | | |
|---|---|---|
| Berechnet für $C_{21}H_{32}O_6$ | C 66,29 | H 8,48% |
| Gefunden | C 65,80 | H 8,31% |

## Beispiel 2

a) 110 Teile Hydrochinon, 284 Teile 5-Methylhex-5-ensäure-methylester und 10 Teile p-Toluolsulfonsäure werden 24 Stunden auf einem Dampfbad erhitzt. Das abgekühlte Reaktionsgemisch verfestigt sich zum Teil und ergibt nach dem Verreiben mit Diäthyläther 2,5-Bis-(5'-methoxycarbonyl-2'-methyl-pent-2'-yl)-hydrochinon; Smp. 150—153°C. Nach dem Kristallisieren aus Methanol/Wasser hat das Produkt einen Smp. von 160—162°C.

Analyse:

| | | |
|---|---|---|
| gefunden | C 67,05% | H 8,96% |
| berechnet für $C_{22}H_{34}O_6$ | C 66,98% | H 8,69% |

b) 44 Teile 2,5-Bis-(5'-carboxy-2'-methyl-pent-2'-yl)-hydrochinon werden unter kräftigem Rühren portionenweise zu 375 Teilen 10—14%iger wäßriger Natriumhypochloritlösung gegeben, wobei die Temperatur mit einem kalten Wasserbad auf 25—30°C gehalten wird. Das Gemisch wird 1 Stunde bei Raumtemperatur gerührt, mit 300 Teilen Wasser verdünnt und mit konzentrierter Chlorwasserstoffsäure angesäuert. Nach dem Filtrieren erhält man 2,5-Bis-(5'-carboxy-2'-methyl-pent-2'-yl)-1,4-benzochinon, das nach dem Kristallisieren aus Methanol einen Smp. von 204—206°C aufweist.

Aus Methanol und 2,5-Bis-(5'-carboxy-2'-methyl-pent-2'-yl)-1,4-benzochinon in Gegenwart von Chlorwasserstoffsäure als Katalysator wird 2,5-Bis-(5'-methoxycarbonyl-2'-methyl-pent-2'-yl)-1,4-benzochinon als gelbes Produkt vom Smp. 85—87°C mit folgender Elementaranalyse erhalten.

| | | |
|---|---|---|
| Berechnet für $C_{22}H_{32}O_6$ | C 67,32 | H 8,22% |
| Gefunden | C 67,56 | H 8,20% |

c) 3,9 Teile 2,5-Bis-(5'-methoxycarbonyl-2'-methyl-pent-2'-yl)-hydrochinon (Beispiel 2a), 0,4 Teile 2,5-Bis-(5'-methoxycarbonyl-2'-methyl-pent-2'-yl)-1,4-benzochinon (Beispiel 2b), 1,0 Teil p-Toluolsulfonsäure und 50 Teile Methanol werden am Rückfluß 48 Stunden erhitzt. Nach Eindampfen des überschüssigen Methanols unter reduziertem Druck wird der Rückstand in Diäthyläther aufgenommen, die ätherische Phase zuerst mit einer Natriumbicarbonat-Lösung und dann mit Wasser gewaschen.

Nach dem Verdampfen des Äthers wird ein festes Produkt isoliert, welches durch GLC-Analyse die folgende Zusammensetzung in Gewichtsprozent aufweist:

| Verbindung | % |
|---|---|
| 2,5-Bis-(5'-methoxycarbonyl-2'-methyl-pent-2'-yl)-1,4-dimethoxybenzol | 21,3 |
| 2,5-Bis-(5'-methoxycarbonyl-2'-methyl-pent-2'-yl)-4-methoxyphenol | 67,0 |
| 2,5-Bis-(5'-methoxycarbonyl-2'-methyl-pent-2'-yl)-hydrochinon | 11,7 |

Nach Kristallisation des oben beschriebenen Produkts aus Petroläther (40–60°C) erhält man 2,5-Bis-(5'-methoxycarbonyl-2'-methyl-pent-2'-yl)-4-methoxyphenol vom Smp. 81–83°C, das dem Beispiel 1a identisch ist.

Beispiel 3

3,5 Teile 2,5-Bis-(5'-methoxycarboxy-2'-methyl-pent-2'-yl)-4-methoxyphenol, 100 Teile n-Hexanol und 0,5 Teile p-Toluolsulfonsäure werden auf einem Dampfbad während 48 Stunden erhitzt. Überschüssiges n-Hexanol wird danach unter vermindertem Druck abgezogen. Der Rückstand wird in Diäthyläther aufgenommen und mit Natriumcarbonatlösung und dann mit Wasser gewaschen. Nach dem Verdampfen des Diäthyläthers erhält man aus der ätherischen Lösung durch Kurzwegdestillation 2,5-Bis-(5'-n-hexyloxycarbonyl-2'-methyl-pent-2'-yl)-4-methoxyphenol als hellgelbes Öl.

Elementaranalyse:

| | | |
|---|---|---|
| Berechnet für $C_{33}H_{56}O_6$ | C 72,22 | H 10,29% |
| Gefunden | C 72,33 | H 10,45% |

Beispiel 4

2,3 Teile 2,5-Bis-(5'-carboxy-2'-methyl-pent-2'-yl)-4-methoxyphenol, 50 Teile Tetrahydrofurfurylalkohol und 0,2 Teile p-Toluolsulfonsäure werden auf einem Dampfbad 18 Stunden erhitzt. Der überschüssige Alkohol wird unter reduziertem Druck eingedampft. Das zurückbleibende Öl wird mit Diäthyläther aufgenommen, zuerst mit verdünnter Bicarbonatlösung und dann mit Wasser gewaschen und verdampft. Das zurückbleibende Öl ist 2,5-Bis-(5'-tetrahydrofurfuryloxycarbonyl-2'-methyl-pent-2'-yl)-4-methoxyphenol.

Elementaranalyse:

| | | |
|---|---|---|
| Berechnet für $C_{31}H_{48}O_8$ | C 67,86 | H 8,82% |
| Gefunden | C 67,97 | H 9,09% |

Beispiel 5

Analog dem in Beispiel 4 beschriebenen Verfahren wird aus n-Butoxyäthanol anstelle von Tetrahydrofurfurylalkohol 2,5-Bis-(5'-n-butoxyäthoxycarbonyl-2'-methyl-pent-2'-yl)-4-methoxyphenol als Öl mit folgender Elementaranalyse erhalten:

| | | |
|---|---|---|
| Berechnet für $C_{33}H_{56}O_8$ | C 68,24 | H 9,72% |
| Gefunden | C 68,33 | H 9,67% |

Beispiel 6

a) Das Verfahren gemäß Beispiel 1 wird wiederholt, indem 13,8 Teile Hydrochinonmonoäthyläther anstelle von Hydrochinonmonomethyläther eingesetzt wird. So wird 2,5-Bis-(5'-methoxycarbonyl-2'-methyl-pent-2'-yl)-4-äthoxyphenol als Öl (Kurzwegdestillation) erhalten. Siedepunkt bei 0,7 Millibar: 210°C.

Elementaranalyse:
Berechnet für $C_{24}H_{38}O_6$ C 68,22 H 9,06 %
Gefunden C 68,45 H 9,31 %

b) Analog dem in Beispiel 1b) beschriebenen Verfahren wird 2,5-Bis-(5'-carboxy-2'-methyl-pent-2'-yl)-4-äthoxyphenol vom Smp. 169—171° erhalten.

Elementaranalyse:
Berechnet für $C_{22}H_{34}O_6$ C 66,98 H 8,69%
Gefunden C 67,13 H 8,72%

Beispiel 7

Die Säure des Beispiels 6b) wird mit Äthanol gemäß dem in Beispiel 4 angegebenen Verfahren verestert. Dabei wird 2,5-Bis-(5'-äthoxycarbonyl-2'-methyl-pent-2'-yl)-4-äthoxyphenol vom Smp. 52—54°C aus Petroläther erhalten.

Elementaranalyse:
Berechnet für $C_{26}H_{42}O_6$ C 69,30 H 9,36%
Gefunden C 69,91 H 9,44%

Beispiel 8

Analog dem in Beispiel 4 beschriebenen Verfahren wird die Säure des Beispiels 6b) mit n-Hexanol versetzt. Man erhält 2,5-Bis-(5'-n-hexyloxycarbonyl-2'-methyl-pent-2'-yl)-4-äthoxyphenol als Öl (Kurzwegdestillation bei 0,7 Millibar).

Elementaranalyse:
Berechnet für $C_{34}H_{58}O_6$ C 72,56 H 10,39%
Gefunden C 72,61 H 10,39%

Beispiel 9

Analog dem in Beispiel 1a) und 1b) beschriebenen Verfahren wird aus 16,6 Teilen Hydrochinon-n-butyläther erhalten:
a) 2,5-Bis-(5'-methoxycarbonyl-2'-methyl-pent-2'-yl)-4-n-butoxyphenol mit Siedepunkt 238—40°C bei 0,9 Millibar

Elementaranalyse:
Berechnet für $C_{26}H_{42}O_6$ C 69,30 H 9,39%
Gefunden C 69,33 H 9,52%

b) 2,5-Bis-(5'-carboxy-2'-methyl-pent-2'-yl)-4-n-butoxyphenol vom Smp. 141—143°C aus Eisessig/Wasser

Elementaranalyse:
Berechnet für $C_{24}H_{38}O_6$ C 68,22 H 9,06%
Gefunden C 68,12 H 9,19%

c) Die Säure aus Beispiel 9b) wird mit Isopropanol gemäß dem in Beispiel 4 beschriebenen Verfahren verestert. Man erhält 2,5-Bis-(5'-isopropoxycarbonyl-2'-methyl-pent-2'-yl)-4-n-butoxyphenol vom Smp. 62—64°C aus Petroläther (40—60°C) bei —20°C.

Elementaranalyse:
Berechnet für $C_{30}H_{50}O_6$ C 71,11 H 9,95%
Gefunden C 71,37 H 10,00%

d) Die Säure aus Beispiel 9b) wird mit n-Hexanol nach dem in Beispiel 4 angegebenen Verfahren verestert. Man erhält 2,5-Bis-(5'-n-hexyloxycarbonyl-2'-methyl-pent-2'-yl)-4-n-butoxyphenyl als Öl nach Kurzweg-Destillation bei 0,3 Millibar.

Elementaranalyse:
Berechnet für $C_{36}H_{62}O_6$ C 73,18 H 10,58%
Gefunden C 73,09 H 10,51%

Beispiel 10

62 Teile Hydrochinonmonomethyläther, 28,4 Teile 5-Methylhex-5-ensäuremethylester und 2,0 Teile p-Toluolsulfonsäure werden auf einem Dampfbad 5 Tage erhitzt. Das abgekühlte Reaktionsgemisch wird in Diäthyläther aufgenommen, mit wäßriger 2N Natriumhydroxidlösung und dann mit Wasser gewaschen, bis die wäßrige Phase neutral ist. Nach dem Abziehen des Diäthyläthers wird der Rückstand destilliert. Man erhält 2-(5'-Methoxycarbonyl-2'-methyl-pent-2'-yl)-4-methoxyphenol vom Siedepunkt 154°C bei 0,1 Millibar mit folgender Elementaranalyse:

| | | |
|---|---|---|
| Berechnet für $C_{15}H_{22}O_4$ | C 67,65 | H 8,33% |
| Gefunden | C 67,92 | H 8,56% |

Durch Ansäuern des obigen alkalischen Waschwassers wird ein Öl erhalten, das nach Extraktion mit Diäthyläther und anschließender Destillation 3-(5'-Methoxycarbonyl-2'-methyl-pent-2'-yl)-4-methoxyphenol vom Siedepunkt 172°C bei 0,4 Millibar liefert.

Elementaranalyse:

| | | |
|---|---|---|
| Berechnet für $C_{15}H_{22}O_4$ | C 67,65 | H 8,33% |
| Gefunden | C 67,47 | H 8,32% |

Beispiel 11

Zu 6,8 Teilen Aluminiumchlorid (gelöst in 25 Teilen Nitromethan) werden 13,8 Teile Hydrochinonmethyläther und 7,1 Teile 5-Methyl-hex-5-ensäuremethylester (gelöst in 25 Teilen Nitromethan) bei Raumtemperatur zugetropft. Nach Stehenlassen bei Raumtemperatur während 2 Tagen wird das Reaktionsgemisch in Wasser gegossen und die organische Phase mit Diäthyläther extrahiert. Der ätherische Auszug wird zuerst mit Wasser, dann mit einer 2N Natriumhydroxid-Lösung und anschließend wiederum mit Wasser gewaschen und danach eingedampft. Nach Destillation des Rückstands erhält man 2-(5'-Methoxycarbonyl)-2'-methyl-pent-2'-yl)-1,4-dimethoxybenzol vom Siedepunkt 150°C/ 0,4 Millibar.

Elementaranalyse:

| | | |
|---|---|---|
| Berechnet für $C_{16}H_{24}O_4$ | C 68,55 | H 8,63% |
| Gefunden | C 68,66 | H 8,66% |

Beispiel 12

Analog dem in Beispiel 10 angegebenen Verfahren wird aus den doppelten Mengen Aluminiumchlorid und 5-Methyl-hex-5-ensäuremethylester 2,5-Bis-(5'-methoxycarbonyl-2'-methyl-pent-2'-yl)-1,4-dimethoxybenzol vom Smp. 81—83°C nach Kristallisation aus Petroläther (40—60°C) erhalten.

Elementaranalyse:

| | | |
|---|---|---|
| Berechnet für $C_{24}H_{38}O_6$ | C 68,22 | H 9,06% |
| Gefunden | C 68,48 | H 9,12% |

Beispiel 13

2,5 Teile 2-(5'-Methoxycarbonyl-2'-methyl-pent-2'-yl)-hydrochinon, 1,5 Teile 2,5-Dimethylhexa-2,4-dien und 0,1 Teile p-Toluolsulfonsäure werden auf einem Dampfbad während 3 Tagen erhitzt. Das abgekühlte Reaktionsgemisch wird mit Diäthyläther behandelt, dann zuerst mit einer wäßrigen 2N Natriumhydroxidlösung und dann mit Wasser gewaschen. Die ätherische Phase wird abgetrennt und verdampft. Der Rückstand liefert nach Kurzwegdestillation bei 0,3 Millibar 1,7 Teile einer tiefsiedenden Fraktion bei der Ofentemperatur von 175°C und 6-Hydroxy-7-(5'-methoxycarbonyl-2'-methyl-pent-2'-yl)-2,2-dimetyl-4-isopropyl-chroman bei der Ofentemperatur von 250°C.

Elementaranalyse:

| | | |
|---|---|---|
| Berechnet für $C_{22}H_{34}O_4$ | C 72,89 | H 9,45% |
| Gefunden | C 72,15 | H 9,56% |

Beispiel 14

24,8 Teile p-Methoxyphenol, 15,1 Teile Citronellylnitril und 2,0 Teile p-Toluolsulfonsäure werden auf einem Dampfbad 3 Tage erhitzt. Das Reaktionsgemisch wird mit Diäthyläther verdünnt und die ätherische Phase mit einer Natriumbicarbonatlösung und dann mit Wasser gewaschen. Nach Eindampfen des Äthers wird der Rückstand destilliert. Man erhält das Gemisch von 2- und 3-(7'-Cyano-2',6'-dimethyl-hept-2'-yl)-4-methoxyphenol vom Siedepunkt 178—186°C bei 0,3 Millibar.

Elementaranalyse:

| | | | |
|---|---|---|---|
| Berechnet für $C_{17}H_{25}NO_2$ | C 73,91 | H 9,39 | N 5,18% |
| Gefunden | C 74,14 | H 9,15 | N 5,09% |

Beispiel 15

Zu einer Suspension von 4,0 Teilen Lithiumaluminiumhydrid in 100 Teilen Diäthyläther werden unter Rühren 5,5 Teile 2- und 3-(7'-Cyano-2',6'-dimethyl-hept-2'-yl)-4-methoxyphenol (aus Beispiel 14), in 50 Teilen Diäthyläther gelöst, während 30 Minuten zugetropft. Es wird 2 Stunden weitergerührt. Zur Zerstörung des unreagierten Lithiumaluminiumhydrids wird dann Äthylacetat, anschließend 100 Teile einer 10N Natriumhydroxidlösung zugegeben. Die wäßrige Phase wird abgetrennt und die ätherische Phase mit Wasser gewaschen. Nach Verdampfen des Diäthyläthers und Destillation des Rückstands wird ein Gemisch von 2- und 3-(8'-Amino-2',6'-dimethyl-oct-2'-yl)-4-methoxyphenol vom Siedepunkt 182—185°C bei 0,7 Millibar als farbloses Öl mit folgender Elementaranalyse erhalten:

| | | | |
|---|---|---|---|
| Berechnet für $C_{17}H_{29}NO_2$ | C 73,07 | H 10,46 | N 5,01% |
| Gefunden | C 72,91 | H 10,46 | N 4,80% |

Beispiel 16

Analog dem in Beispiel 15 angegebenen Verfahren werden 9,2 Teile 2,5-Bis-(methoxycarbonyl-2'-methyl-pent-2'-yl)-4-äthoxyphenol mit 4,0 Teilen Lithiumaluminiumhydrid reduziert. Man erhält 2,5-Bis-(6'-hydroxy-2'-methyl-hex-2'-yl)-4-äthoxyphenol vom Siedepunkt 230°C/0,7 Millibar als hellgelbes Öl mit folgender Elementaranalyse:

| | | |
|---|---|---|
| Berechnet für $C_{22}H_{38}O_4$ | C 72,09 | H 10,45% |
| Gefunden | C 72,16 | H 10,69% |

Beispiel 17

62 Teile p-Methoxyphenol, 17,8 Teile Dimethylprenylphosphonat und 5,0 Teile Fulcat 22® werden bei 125—130°C 24 Stunden gerührt. Nach Abkühlen wird das Reaktionsgemisch mit Methanol verdünnt und vom Katalysator durch Filtration befreit. Nach Verdampfen des Methanols unter reduziertem Druck mit einem Rotationsverdampfer wird das zurückbleibende Öl zur Entfernung des unreagierten p-Methoxyphenols (115°C/0,7 Millibar) abdestilliert. Der ölige Rückstand wird mit Säulenchromatographie (enthaltend 200 Teile Kieselerde in Petroläther 40—60°C) gereinigt, indem mit Petroläther und anschließend mit Petroläther enthaltend zunehmende Mengen Diäthyläther eluiert wird. Man erhält 2-(2'-Methyl-4'-dimethylphosphono-but-2'-yl)-4-methoxyphenol als weiße Prismen vom Smp. 97—99°C aus Diäthyläther mit folgender Elementaranalyse:

| | | | |
|---|---|---|---|
| Berechnet für $C_{14}H_{23}O_5P$ | C 55,62 | H 7,67 | P 10,25% |
| Gefunden | C 56,00 | H 7,52 | P 10,13% |

Beispiel 18

Analog dem in Beispiel 1 angegebenen Verfahren werden 12,4 Teile p-Methoxyphenol und 9,4 Teile N-Acetyl-heptaminol zusammen versetzt. Man erhält ein Gemisch von 2- und 3-(6'-Acetamido-2'-methyl-pent-2'-yl)-4-methoxyphenol. Das 2-Isomer wird als weiße Kristalle vom Smp. 118—120°C durch Kristallisation aus Diäthyläther enthaltend kleine Mengen Aceton erhalten.

Elementaranalyse:

| | | | |
|---|---|---|---|
| Berechnet für $C_{17}H_{27}NO_3$ | C 69,59 | H 9,28 | N 4,77% |
| Gefunden | C 69,65 | H 9,62 | N 4,87% |

Beispiel 19

8,2 Teile 2,5-Bis-(5'-methoxycarbonyl-2'-methyl-pent-2'-yl)-4-methoxyphenol und 50 Teile n-Octylamin werden unter Rückfluß 20 Stunden versetzt. Nach Eindampfen des überschüssigen Octylamins unter reduziertem Druck wird der Rückstand mit Petroläther (40—60°C), enthaltend kleine Mengen Diäthyläther, verdünnt. Man erhält 2,5-Bis-(5'-N-n-octylcarbamoyl-2'-methyl-pent-2'-yl)-4-methoxyphenol vom Smp. 86—90°C mit folgender Elementaranalyse:

| | | | |
|---|---|---|---|
| Berechnet für $C_{37}H_{66}N_2O_4$ | C 73,71 | H 11,03 | N 4,65% |
| Gefunden | C 73,54 | H 11,88 | N 4,81% |

Beispiel 20

5,0 Teile 2,5-Bis-(5'-methoxycarbonyl-2'-methyl-pent-2'-yl)-4-methoxyphenol und 20 Teile Di-n-butylamin werden in einem Glasrohr eingeschmolzen und 45 Stunden auf 200°C erhitzt. Nach dem Entfernen des überschüssigen Ni-n-butylamins unter vermindertem Druck wird 2,5-Bis-(5'-di-N,N-n-butylcarbamoyl-2'-methyl-pent-2'-yl)-4-methoxyphenol als hellgelber, viskoser Sirup erhalten.

Beispiel 21

24,8 Teile p-Methoxyphenol, 13,8 Teile 4-Acetyl-1-methyl-cyclohex-1-en und 2,0 Teile p-Toluolsulfonsäure werden 3 Tage auf einem Dampfbad erhitzt. Dann wird das Reaktionsgemisch in Diäthyläther aufgenommen, die ätherische Phase mit 2N Natriumhydroxidlösung, danach mit Wasser gewaschen und Diäthyläther verdampft. Der erhaltene Rückstand wird destilliert und ergibt ein Gemisch von cis- und trans-2-(4'-Acetyl-1'-methyl-cyclohex-1'-yl)-4-methoxyphenol vom Siedepunkt 200—220°C/0,3 Millibar mit folgender Elementaranalyse:

| | | |
|---|---|---|
| Berechnet für $C_{16}H_{22}O_3$ | C 73,25 | H 8,45% |
| Gefunden | C 75,81 | H 8,96% |

Beispiel 22

Analog dem in Beispiel 21 angegebenen Verfahren werden aus 21,2 Teilen 4,5-Bis-methoxycarbonyl-1-methyl-cyclohex-1-en an Stelle von 4-Acetyl-1-methyl-cyclohex-1-en nach Destillation 2- und 3-(3',4'-Bismethoxycarbonyl-1'-methyl-cyclohex-1'-yl)-4-methoxyphenol vom Siedepunkt 218 bis 224°C/0,3 Millibar erhalten.

Elementaranalyse:

| | | |
|---|---|---|
| Berechnet für $C_{18}H_{24}O_6$ | C 64,27 | H 7,19% |
| Gefunden | C 64,23 | H 7,44% |

Beispiel 23

10,0 Teile 2,5-Bis-(5'-methoxycarbonyl-2'-methyl-pent-2'-yl)-4-methoxyphenol, 50 Teile Cetylalkohol und 1,0 Teil p-Toluolsulfonsäure werden auf einem Dampfbad 3 Tage erhitzt. Überschüssiger Cetylalkohol wird unter reduziertem Druck mit einem Rotationsverdampfer zum größten Teil eingedampft und das zurückbleibende Öl mit Diäthyläther aufgenommen. Die ätherische Lösung wird zuerst mit einer Natriumbicarbonatlösung, dann mit Wasser gewaschen und anschließend eingeengt. Das zurückbleibende Öl wird mit einem Rotationsdestillierapparat bei 0,7 Millibar und 230°C destilliert. Man erhält 2,5-Bis-(5'-hexadecyloxycarbonyl-2'-methyl-pent-2'-yl)-4-methoxyphenol als viskoses Öl mit folgender Elementaranalyse:

| | | |
|---|---|---|
| Berechnet für $C_{53}H_{96}O_6$ | C 76,81 | H 11,59% |
| Gefunden | C 77,15 | H 11,90% |

Beispiel 24

Analog dem in Beispiel 17 angegebenen Verfahren werden aus 30,6 Teilen Diäthyl-2-äthoxycarbonyl-5-methyl-hex-4-en-2-phosphonat 2- und 3-(5'-Äthoxycarbonyl-5'-diäthylphosphono-2'-methyl-pent-2'-yl)-4-methoxyphenol als viskoses Öl mit folgender Elementaranalyse erhalten:

| | | | |
|---|---|---|---|
| Berechnet für $C_{21}H_{35}O_7P$ | C 58,59 | H 8,19 | P 7,19% |
| Gefunden | C 58,51 | H 8,12 | P 6,95% |

Beispiel 25

5,1 Teile 2-(5'-Methoxycarbonyl-2'-methyl-pent-2'-yl)-1,4-hydrochinon, 1,9 Teile 1,4-Bis-(2'-hydroxyprop-2'-yl)-benzol und 0,2 Teile p-Toluolsulfonsäure in 25 Teilen Benzol werden auf einem Dampfbad 24 Stunden erhitzt. Das abgekühlte Reaktionsgemisch wird mit Diäthyläther verdünnt, zuerst mit einer 2N Natriumhydroxidlösung und dann mit Wasser gewaschen und Diäthyläther verdampft. Das zurückbleibende Produkt ergibt nach Kristallisation aus Benzol 1,4-Bis-{2'-[2'',5''-dihydroxy-4''-(5'''-methoxycarbonyl-2'''-methyl-pent-2'''-yl)-phenyl]-prop-2'-yl}-benzol vom Smp. 198 bis 201 °C mit folgender Elementaranalyse:

| | | |
|---|---|---|
| Berechnet für $C_{40}H_{54}O_8$ | C 72,48 | H 8,21% |
| Gefunden | C 72,68 | H 8,39% |

Beispiel 26

6,9 Teile Hydrochinondimethyläther, 6,2 Teile N-n-Octyl-2-propen-1-sulfonsäureamid und 13,4 Teile $AlCl_3$ in 50 Teilen Nitromethan werden 3 Tage bei Raumtemperatur stehengelassen. Das Reaktionsgemisch wird dann in Wasser gegossen und die organische Phase mit Diäthyläther extrahiert. Nach Waschen mit Wasser wird die ätherische Phase eingeengt und der Rückstand über Säulenchromatographie (Kieselsäure) gereinigt. Man erhält 2-(2'-Methyl-3'-N-n-octylsulfonamido-prop-2'-yl)-4-methoxyphenol als viskoses Öl mit folgender Elementaranalyse:

| | | | |
|---|---|---|---|
| Berechnet für $C_{20}H_{35}NO_4S$ | C 62,30 | H 9,15 | N 3,63% |
| Gefunden | C 62,17 | H 8,83 | N 3,83% |

Beispiel 27

13,3 Teile 2-(5'-Methoxycarbonyl-2'-methyl-pent-2'-yl)-4-methoxyphenol und 2,9 Teile Hexamethylendiamin werden bei 150°C 24 Stunden erhitzt. Das abgekühlte Reaktionsprodukt wird mit Petroläther (40—60°C) verrieben und ergibt N,N'-5-(2'-Hydroxy-5'-methoxyphenyl)-5-methyl-hexansäureamid des Hexansäure-1,6-diamids vom Smp. 58—60°C und mit folgender Elementaranalyse:

| | | | |
|---|---|---|---|
| Berechnet für $C_{34}H_{52}N_2O_6$ | C 69,83 | H 8,96 | N 4,79% |
| Gefunden | C 69,72 | H 9,62 | N 4,48% |

Beispiel 28

2,4 Teile 2,5-Bis-(6'-Hydroxy-2'-methyl-hex-2'-yl)-4-äthoxyphenol (aus Beispiel 16) in 15 Teilen Benzol werden mit 10 Teilen Acetylchlorid in 5 Teilen Benzol behandelt. Nach Stehenlassen über Nacht bei Raumtemperatur wird Benzol eingedampft. Der Rückstand liefert nach Kurzweg-Destillation bei 0,7 Millibar 2,5-Bis-(6'-acetoxy-2'-methyl-hex-2'-yl)-4-äthoxyphenol als hellgelbes, viskoses Öl.

Beispiel 29

a) 4,3 Teile 3-(5'-Methoxycarbonyl-2'-methyl-pent-2'-yl)-4-methoxyphenol (aus Beispiel 10), 2,0 Teile Isobutylen und 0,2 Teile p-Toluolsulfonsäure in 25 Teilen Benzol werden in einem Glasrohr bei 100°C während 40 Stunden erhitzt. Das abgekühlte Reaktionsgemisch wird mit einer 2N Natriumhydroxidlösung und dann mit Wasser gewaschen. Nach Eindampfen des Diäthyläthers und des Benzols wird 2-tert.-Butyl-5-(5'-methoxycarbonyl-2'-methyl-pent-2'-yl)-4-methoxyphenol als weißes, kristallines Produkt vom Smp. 71—73°C aus Petroläther (60—80°C) erhalten.

Elementaranalyse:

| | | |
|---|---|---|
| Berechnet für $C_{19}H_{30}O_4$ | C 70,77 | H 9,38% |
| Gefunden | C 70,65 | H 9,62% |

b) Die in Beispiel 29a) erhaltene Verbindung wird mit n-Hexanol gemäß dem in Beispiel 3 angegebenen Verfahren umgeestert. Nach Kurzweg-Destillation bei 0,1 Millibar wird 2-tert.-Butyl-5-(5'-n-hexyloxy-carbonyl-2'-methyl-pent-2'-yl)-4-methoxyphenol als hellgelbes, viskoses Öl mit folgender Elementaranalyse erhalten:

| | | |
|---|---|---|
| Berechnet für $C_{24}H_{40}O_4$ | C 73,43 | H 10,27% |
| Gefunden | C 73,76 | H 10,51% |

Beispiel 30

a) 2-(2'-Phenyl-prop-2'-yl)-5-(5'-methoxycarbonyl-2'-methyl-pent-2'-yl)-4-methoxyphenol vom Smp. 102—104°C aus Petroläther wird aus $\alpha$-Methylstyren und 3-(5'-Methoxycarbonyl-2'-methyl-pent-2'-yl)-4-methoxyphenol gemäß dem in Beispiel 29 angegebenen Verfahren erhalten:

Elementaranalyse:

| | | |
|---|---|---|
| Berechnet für $C_{24}H_{32}O_4$ | C 74,97 | H 8,39% |
| Gefunden | C 75,21 | H 8,49% |

b) Analog dem in Beispiel 3 beschriebenen Verfahren wird durch Umesterung des Methylesters der in Beispiel 30a) erhaltenen Verbindung mit n-Hexanol 2-(2'-Phenyl-prop-2'-yl)-5-(5'-n-hexyloxycarbonyl-2'-methyl-pent-2'-yl)-4-methoxyphenol als hellgelbes, viskoses Öl nach Kurzweg-Destillation bei 0,7 Millibar erhalten.

Elementaranalyse:

| | | |
|---|---|---|
| Berechnet für $C_{29}H_{42}O_4$ | C 76,61 | H 9,31% |
| Gefunden | C 76,62 | H 9,21% |

Beispiel 31

2,5 Teile 2-(5'-Methoxycarbonyl-2'-methyl-pent-2'-yl)-hydrochinon, 0,68 Teile Isopren, 0,1 Teil p-Toluolsulfonsäure und 15 Teile Benzol werden in einem Glasrohr bei 80°C während 3 Tagen erhitzt. Nach Aufarbeitung wie in Beispiel 13 wird 6-Hydroxy-7-(5'-methoxycarbonyl-2'-methyl-pent-2'-yl)-2,2-dimethylchroman als hellgelbes Öl nach Kurzweg-Destillation bei 0,7 Millibar mit folgender Elementaranalyse erhalten.

| | | |
|---|---|---|
| Berechnet für $C_{19}H_{28}O_4$ | C 71,22 | H 8,81% |
| Gefunden | C 71,44 | H 8,84% |

Beispiel 32

82 Teile 2-(5'-Methoxycarbonyl-2'-methyl-pent-2'-yl)-4-methoxyphenol werden in 300 ml Aceton gelöst. 125 Teile Jone's Reagenz werden dann innert einer Stunde bei einer Temperatur von unter 25°C zugetropft. Nach Zugabe von 500 ml Wasser wird das Reaktionsgemisch mit Diäthyläther behandelt und die so erhaltene organische Phase mit Wasser gewaschen, getrocknet und eingeengt. Man erhält 75 Teile eines orangen Öls, das durch Destillation gereinigt wird. Die erhaltene Fraktion zwischen 120—125°C/0,93 mBar entspricht der Verbindung 2-(5'-Methoxycarbonyl-2'-methyl-pent-2'-yl)-1,4-benzochinon.
Ausbeute: 43 g.

Beispiel 33

43 Teile (0,17 Mole) der gemäß Beispiel 32 erhaltenen Verbindung werden in 400 ml Methylenchlorid gelöst. Dazu werden 43 Teile Natriumdithionit (in 400 ml Wasser gelöst) gegeben und das Gemisch unter $N_2$ bei Raumtemperatur während 18 Stunden gerührt. Das Reaktionsgemisch wird mit 250 ml Diäthyläther behandelt und die so erhaltene organische Phase mit Wasser gewaschen, getrocknet und eingeengt. 41,2 Teile 2-(5'-Methoxycarbonyl-2'-methyl-pent-2'-yl)-1,4-hydrochinon werden als Öl erhalten, das nach Kristallisation aus Petroläther (80—100°C) als farbloses Produkt einen Schmelzpunkt von 97—98°C aufweist.

Beispiel 34

5,04 Teile der gemäß Beispiel 33 erhaltenen Verbindung, 3,06 Teile Bromacetessigsäuremethylester und 3,0 Teile wasserfreies Kaliumcarbonat werden in 100 ml wasserfreiem Aceton während 24 Stunden unter Rückfluß erhitzt. Die flüchtigen Anteile werden danach unter reduziertem Druck abgedampft und das so erhaltene Öl durch Säulen-Chromatographie über Kieselgel gereinigt. 2-(5'-Methoxycarbonyl-2'-methyl-pent-2'-yl)-4-(methoxycarbonyl-methoxy)-phenol wird als hellbeiges Öl erhalten, das beim Stehenlassen erstarrt und nach Kristallisation aus Toluol weiße Kristalle vom Smp. 93—95°C ergibt.

Verwendungsbeispiele

Verwendungsbeispiel 1

0,05 mMol des Magenta-Kupplers der Formel

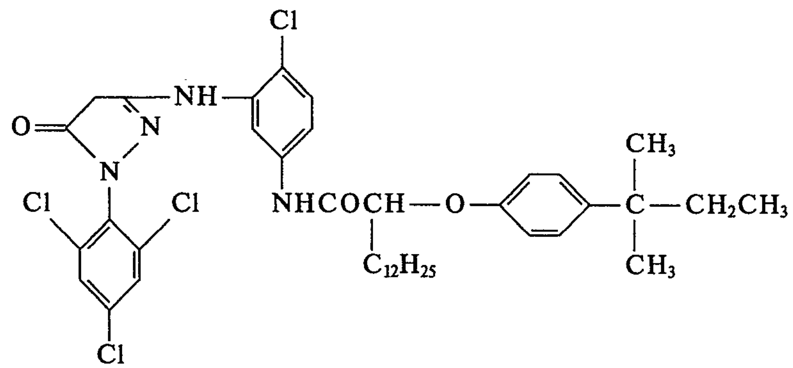

und 0,025 mMol einer Verbindung der Formel I werden in 2,0 ml Trikresylphosphat/Äthylacetat (0,75 g/100 ml) gelöst. 7,0 ml einer 6%igen Gelatinelösung, 1,0 ml einer 0,8%igen Lösung des Netzmittels der Formel

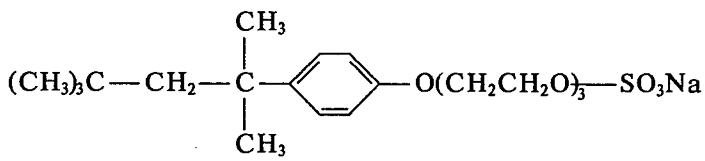

werden in Wasser eingetragen, dann 5 Minuten mit Hilfe eines 100 Watt-Ultraschallgeräts emulgiert. 2,5 ml der frisch im Ultraschallgerät behandelten Kuppler-Additiv-Emulsion, 2,0 ml einer Silberbromidemulsion mit einem Silbergehalt von 0,6%, 0,7 ml einer 1%igen wäßrigen Lösung des Härters der Formel

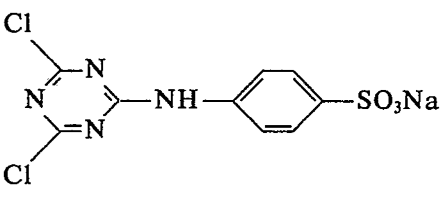

und 2,8 ml Wasser werden gemischt, auf einen pH-Wert von 6,5 eingestellt und bei 40°C auf ein Polyäthylenpapier mit den Massen 14 × 18 cm gegossen. Nach dem Härten der Schicht bei 10°C, wird das aufgegossene Gemisch bei Raumtemperatur getrocknet.

Verarbeitung

Muster des erhaltenen beschichteten Papiers werden 6 Sekunden unter einem Stufenkeil mit Licht von 600 Lux bestrahlt und dann wie folgt bei 32,8°C (±0,3°C) behandelt:

| | | |
|---|---|---|
| 1. | Entwicklungsbad | 3,5 Minuten |
| 2. | Bleich- und Fixierbad | 1,5 Minuten |
| 3. | Waschen | 3,0 Minuten |
| 4. | Trocknen | 1,0 Minute |

Das Entwicklungsbad hat die folgende Zusammensetzung:
4-Amino-3-methyl-N-äthyl-N-[β-(methyl-sulfonamido)-äthyl]-anilin

| | |
|---|---|
| 1 ½ $H_2SO_4 \cdot H_2O$ | 4,85 (g/Liter) |
| Kaliumbromid | 0,6 |
| Kaliumcarbonat | 32,0 |
| Lithiumsulfat | 1,8 |
| Kaliumsulfit | 2,0 |
| Hydroxylaminsulfit | 3,9 |
| Äthylenglykol | 21,3 |
| Benzylalkohol | 15,1 |
| Wasser | bis zu einem Liter |

Der pH-Wert beträgt 10,1.

Als Bleich- und Fixierbad wird ein übliches Bad eingesetzt, z. B. mit der folgenden Zusammensetzung:

| | |
|---|---|
| Ammoniumthiosulfat (80%ige Lösung) | 200 (g/Liter) |
| Natriumsulfit (wasserfrei) | 15 |
| Natriumcarbonat (wasserfrei) | 2,5 |
| Äthylendiamin-tetraessigsäure-Na-Salz | 2 |
| Na-Salz von Fe(III)-Äthylendiamin-tetraessigsäure | 50 |
| Wasser | bis zu einem Liter |

Nach dem Waschen und Trocknen erhält man klare, scharfe Magenta-Keile mit einem Absorptionsmaximum bei 537 nm und maximaler Dichte von 1,80. Ein auf diese Weise erhaltener Stufenkeil wird in einem Atlas-Gerät (2500-W-Lampe) durch ein Ultraviolett-Filter (Kodakfilter 2C) mit 42 $kJ/cm^2$ bestrahlt. Als Vergleich dient ein Stufenkeil, der ohne Zusatz einer erfindungsgemäßen Verbindung hergestellt wurde. Die restliche optische Dichte (OD) wird jeweils in % der Anfangsdichte (Anfangsdichte 1) gemessen. Die Ergebnisse sind in Tabelle 1 zusammengefaßt.

Tabelle 1

Lichtstabilisierende Wirkung von Verbindungen der Formel I

| Hydrochinon | % OD (mit UV-Filter; 42 $kJ/cm^2$) |
|---|---|
| ohne Stabilisator | 47 |
| Verbindung gemäß Beispiel 1a | 86 |
| Verbindung gemäß Beispiel 3 | 90 |
| Verbindung gemäß Beispiel 4 | 90 |
| Verbindung gemäß Beispiel 5 | 85 |
| Verbindung gemäß Beispiel 6a | 85 |
| Verbindung gemäß Beispiel 7 | 86 |
| Verbindung gemäß Beispiel 8 | 90 |
| Verbindung gemäß Beispiel 9a | 88 |
| Verbindung gemäß Beispiel 9c | 89 |
| Verbindung gemäß Beispiel 13 | 91 |

Im Vergleich zur ohne Stabilisator hergestellten Emulsion zeigen die Emulsionen, welche eine Verbindung der Formel I enthalten, eine bessere Lichtschutzwirkung.

Verwendungsbeispiel 2

Auf analoge Weise wie im Verwendungsbeispiel 1 wird eine Beschichtung hergestellt, welche 0,0125 mMol der Verbindung der Formel 3 und 0,0125 mMol der Verbindung der Formel A

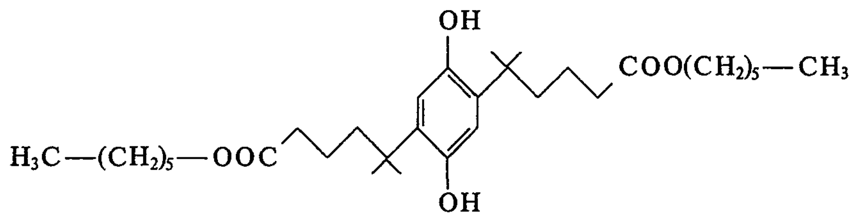

(A)

anstelle einer der im Verwendungsbeispiel 1 eingesetzten Hydrochinonverbindungen enthält, und gemäß dem im Verwendungsbeispiel 1 angegebenen Verfahren mit Licht bestrahlt, behandelt und wiederum bestrahlt. Die restliche optische Dichte beträgt 89% einer ursprünglichen Dichte von 1,0.

Die Verbindung der Formel A wird folgendermaßen hergestellt:

5,5 Teile Hydrochinon, 21,2 Teile 5-Methyl-hex-5-ensäure-n-hexylester und 1,0 Teil p-Toluolsulfonsäure werden auf dem Dampfbad während 3 Tagen erhitzt. Das abgekühlte Reaktionsgemisch wird mit Diäthyläther aufgenommen, mit einer 10%igen Natriumhydroxidlösung und dann mit Wasser bis neutral gewaschen. Nach Eindampfen des Diäthyläthers wird das zurückbleibende Öl, das teilweise erstarrt, mit Petroläther (40—60°C) verrieben. Man erhält 2,5-Bis-(5'-n-hexyloxycarbonyl-2'-methylpent-2'-yl)-hydrochinon vom Smp. 77—78°C, nach einer weiteren Kristallisation aus Petroläther (40—60°C) vom Smp. 83—86°C.

Elementaranalyse:

| | | |
|---|---|---|
| Berechnet für $C_{32}H_{54}O_6$ | C 71,87 | H 10,18 |
| Gefunden | C 71,77 | H 10,23 |

Verwendungsbeispiel 3

Beschichtungen mit einem molaren Verhältnis Kuppler zu Stabilisator von 2 : 1 werden analog dem in Verwendungsbeispiel 1 angegebenen Verfahren hergestellt und durch einen photographischen Stufenkeil Nr. 2 belichtet. Tabelle 2 stellt die grüne Remissionsdichte der Stufen Nr. 3, 5 und 7 zusammen.

Tabelle 2

Farbausbeute in Anwesenheit substituierter Hydrochinonäther

| Verbindung | Remissionsdichte | | |
|---|---|---|---|
| | Stufe 3 | Stufe 5 | Stufe 7 |
| keine | 52 | 90 | 134 |
| gemäß Beispiel 1a | 51 | 94 | 138 |
| gemäß Beispiel 7 | 53 | 91 | 142 |
| gemäß Beispiel 8 | 51 | 88 | 132 |

Daraus läßt sich erkennen, daß die erfindungsgemäßen Verbindungen keinen Einfluß auf die Farbausbeute haben.

Verwendungsbeispiel 4

Auf analoge Weise wie im Verwendungsbeispiel 1 wird eine weitere Verbindung der Formel I geprüft. Die Ergebnisse sind in Tabelle 3 aufgeführt.

Tabelle 3

Lichtstabilisierende Wirkung der Verbindung gemäß Beispiel 12

| Verbindung der Formel I, Hydrochinonäther | % OD (mit UV-Filter; 42 kJ/$cm^2$) |
|---|---|
| ohne Lichtstabilisator | 45 |
| Verbindung gemäß Beispiel 12 | 83 |

Verwendungsbeispiel 5

Analog dem in Verwendungsbeispiel 1 beschriebenen Verfahren wird eine Beschichtung enthaltend 0,05 mMol Magentakuppler, 0,025 mMol der Verbindung gemäß Beispiel Nr. 5 und 0,01 mMol der Verbindung der Formel B

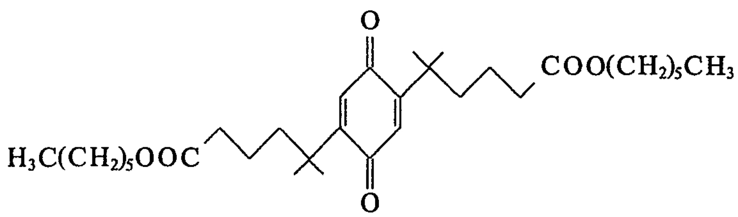

(B)

hergestellt. Stufenkeile werden gemäß Verwendungsbeispiel 1 hergestellt und in einem Atlas-Gerät (2500-W-Lampe) durch ein Ultraviolett-Filter (Kodakfilter 2C) mit einer Gesamtenergie von 84 kJ/$cm^2$ bestrahlt, wobei gleichzeitig ein Stufenkeil, der die gleiche Menge der Verbindung gemäß Beispiel 5 jedoch keine Verbindung der Formel B enthält, als Vergleich dient.

Die restliche optische Dichte (OD) wird in % der Anfangsdichte (Anfangsdichte = 1,0) gemessen. Die Ergebnisse sind in Tabelle 4 zusammengefaßt.

| | % Optische Dichte (OD) |
|---|---|
| Verbindung gemäß Beispiel 5 und Verbindung der Formel B | 83 |
| Verbindung gemäß Beispiel 5 | 79 |

**Patentansprüche**

1. Verbindungen der Formel I

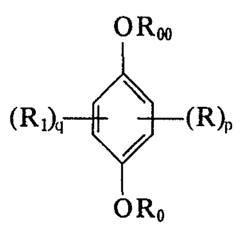

(I)

worin p 1 oder 2 und q 0 oder 1 sind, mit der Maßgabe, daß p+q 1 oder 2 ist,

R einen Rest der Formel II

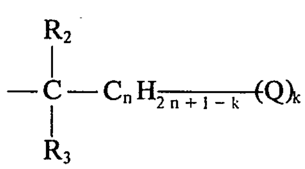

(II)

bedeutet, in welcher

Q die folgenden Reste bedeutet:

i) $-COOR_4$ oder $-CON(R_4)(R_5)$, wobei
$R_4$ unabhängig Wasserstoff, $C_{1-20}$-Alkyl, das durch ein bis fünf Sauerstoffatome unterbrochen und durch eine Gruppe $-OR_6$ substituiert sein kann, eine bifunktionelle $C_{2-20}$-Alkylengruppe, $C_{3-20}$-Alkenyl, $C_{3-12}$-Cycloalkyl, $C_{6-10}$-Aryl, das durch eine oder zwei $C_{1-8}$-Alkylgruppen substituiert sein kann, $C_{7-13}$-Aralkyl, einen 5- oder 6gliedrigen, sauerstoffhaltigen Heterocyclus, der durch eine oder zwei $C_{1-4}$-Alkylgruppen substituiert sein kann, oder Methyl darstellt, das durch einen 5- oder 6gliedrigen, ein Sauerstoffatom enthaltenden, unsubstituierten oder durch eine oder zwei $C_{1-4}$-Alkylgruppen substituierten Heterocyclus substituiert ist,
$R_5$ Wasserstoff, $C_{1-20}$-Alkyl oder $C_{5-6}$-Cycloalkyl bedeutet oder $R_4$ und $R_5$ zusammen mit dem Stickstoffatom, an das sie gebunden sind, einen 5- oder 6gliedrigen Heterocyclus bilden, der durch eine oder zwei $C_{1-4}$-Alkylgruppen substituiert sein kann, und
$R_6$ $C_{3-12}$-Cycloalkyl, $C_{3-20}$-Alkenyl, $C_{6-10}$-Aryl, das durch eine oder zwei $C_{1-4}$-Alkylgruppen substituiert sein kann, oder $C_{7-13}$-Aralkyl bedeutet,

ii) $-OR_7$ oder $-OCOR_7$, wobei
$R_7$ Wasserstoff, $C_{1-20}$-Alkyl, $C_{3-20}$-Alkenyl, $C_{3-12}$-Cycloalkyl, $C_{7-13}$-Aralkyl oder $C_{6-10}$-Aryl darstellt, das durch eine oder zwei $C_{1-4}$-Alkylgruppen substituiert sein kann,

iii) $-N(R_8)(R_9)$, wobei
$R_8$ und $R_9$ unabhängig voneinander $-H$, $C_{1-20}$-Alkyl, $C_{3-20}$-Alkenyl, $C_{3-12}$-Cycloalkyl, $C_{7-13}$-Aralkyl oder $C_{6-10}$-Aryl, das durch eine oder zwei $C_{1-4}$-Alkylgruppen substituiert sein kann, bedeuten, oder ferner die Gruppe $-COR_7$ bedeuten, wobei $R_7$ die oben angegebene Bedeutung hat, oder $R_8$ und $R_9$ zusammen mit dem Stickstoffatom, an das sie gebunden sind, einen unsubstituierten oder durch eine oder zwei $C_{1-4}$-Alkylgruppen substituierten 5- oder 6gliedrigen heterocyclischen Ring bilden,

iv) $-PO(OR_{10})([O]_xR_{11})$, wobei x die Zahl 0 oder 1 ist,
$R_{10}$ und $R_{11}$ bei $x=1$ unabhängig voneinander Wasserstoff, $C_{1-20}$-Alkyl, $C_{6-10}$-Aryl, das durch eine oder zwei $C_{1-4}$-Alkylgruppen substituiert sein kann, oder $R_{10}$ und $R_{11}$ zusammen $C_{2-3}$-Alkylen darstellen, das durch eine bis vier $C_{1-20}$-Alkylgruppen substituiert sein kann, und bei $x=0$ $R_{10}$ die oben angegebene Bedeutung hat und
$R_{11}$ $C_{1-5}$-Alkyl bedeutet,

v) $-SO_2R_{12}$, wobei $R_{12}$ $-OH$, $-Cl$ oder $-N(R_5)(R_7)$ ist und $R_5$ und $R_7$ die oben angegebene Bedeutung haben, mit der Maßgabe, daß $R_1$ einen Rest der Formel II darstellt, wenn $R_{12}$ $-OH$ bedeutet,

vi) $-CN$, Halogen oder $-NO_2$, und

vii) $-COR_{15}$, wobei $R_{15}$ $-H$ oder $C_{1-20}$-Alkyl oder Halogen bedeutet,

n eine ganze Zahl von 1 bis 20 und

k die Zahl 1 oder 2 sind,

$R_2$ und $R_3$ unabhängig voneinander $C_{1-5}$-Alkyl sind, wobei, wenn Q $-COOR_4$ darstellt, eines von $R_2$ oder $R_3$ durch eine Gruppe $-COOR_4$ mit gleichen oder verschiedenen $R_4$ substituiert sein kann, oder $R_2$ oder $R_3$ so mit dem Rest $-C_nH_{2n}-COOR_4$ verbunden sein können, daß ein durch die Gruppe $-COOR_4$ mit gleichen oder verschiedenen $R_4$ substituierter $C_{5-12}$-Cycloalkylenrest gebildet wird, wobei $R_4$ die oben angegebene Bedeutung hat, oder wenn Q $-COR_{15}$ bedeutet, $R_2$ und $R_3$ so mit dem Rest $-C_nH_{2n}-COR_{15}$ verbunden sein können, daß einen $C_{5-12}$-Cycloalkylenrest gebildet wird, wobei $R_{15}$ die oben angegebene Bedeutung hat, mit der Maßgabe, daß p und k die Zahl 1 sind und die Gruppe der Formel I die Formel Ia aufweist, wenn die Gruppe Q den Rest $-COOR_4$ bedeutet, wobei $R_4$ einen bifunktionellen $C_{2-20}$-Alkylenrest darstellt:

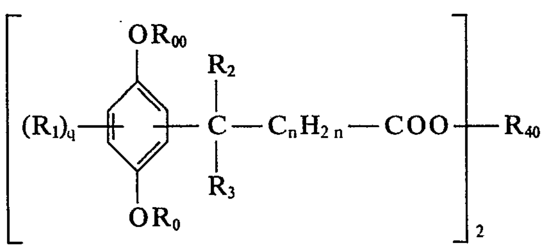

(Ia)

wobei $R_{40}$ ein bifunktionelles $C_{2-20}$-Alkylen ist, und wenn Q einen Rest $-CON(R_4)(R_5)$ und $R_4$ ein bifunktionelles $C_{2-20}$-Alkylen darstellen, p und k die Zahl 1 sind und der Rest der Formel I die Formel Ib aufweist:

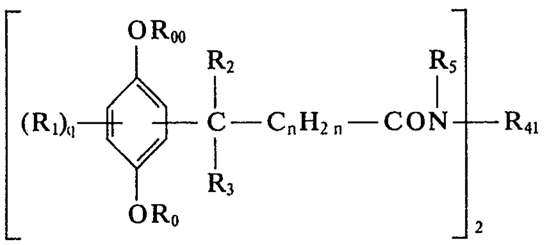

(Ib)

wobei $R_2$, $R_3$, q und n die oben angegebene Bedeutung haben und $R_{41}$ $C_{2-20}$-Alkylen bedeutet,

$R_1$ $C_{1-8}$-Alkyl, $C_{5-7}$-Cycloalkyl, das durch eine oder zwei Methyl- oder Äthylgruppen substituiert sein kann, $C_{7-9}$-Aralkyl oder einen Rest der Formel II, wie oben definiert, bedeutet, und wenn $R_1$ ein Rest der Formel II ist, können $R_1$ und R gleich oder verschieden sein, oder

$R_1$ einen Rest der Formel III bedeutet

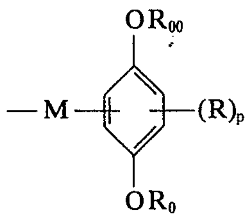

(III)

in welcher

R und p die oben angegebene Bedeutung haben, M eine direkte Bindung, $-C(R_{13})(R_{14})-$, wobei $R_{13}$ und $R_{14}$ unabhängig voneinander –H, $C_{1-20}$-Alkyl, das durch ein bis drei Schwefelatome unterbrochen sein kann, oder $C_{6-10}$-Aryl bedeuten, oder $R_{13}$ und $R_{14}$ zusammen mit dem Kohlenstoffatom, an das sie gebunden sind, ein 5- oder 6gliedriges Alkylen darstellen können, das durch eine oder zwei $C_{1-8}$-Alkylgruppen substituiert sein kann, ferner M –S–, –S–S–, $-SO_2-$, $-CH_2SCH_2-$, $-CH_2OCH_2-$ oder

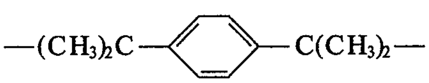

bedeutet,

$R_0$ und $R_{00}$ unabhängig voneinander –H, $C_{1-20}$-Alkyl, das durch 1 bis 5 Sauerstoffatome unterbrochen sein kann, $C_{3-12}$-Cycloalkyl, $C_{6-10}$-Aryl, das durch eine oder zwei $C_{1-4}$-Alkylgruppen substituiert sein kann, oder $C_{7-13}$-Aralkyl bedeuten, mit der Maßgabe, daß $R_0$ und $R_{00}$ gleichzeitig nicht Wasserstoff darstellen können, oder $R_{00}$ die oben angegebene Bedeutung hat und $R_0$ einen Rest der Formel IV bedeutet:

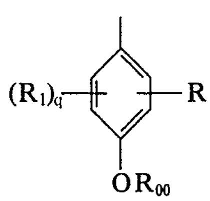

(IV)

in welcher

R, $R_{00}$, $R_1$ und q die oben angegebene Bedeutung haben, oder $R_0$ und $R_1$, wenn sie in ortho-Stellung zueinander stehen, zusammen mit den Kohlenstoffatomen, an die sie gebunden sind, einen gegebenenfalls substituierten Rest der Formel V

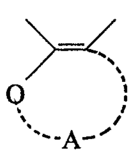

(V)

bilden können, wobei A ein Kohlenstoffrest enthaltend 4 bis 20 C-Atome ist, welcher ein substituiertes Chroman- oder Coumaransystem bildet, oder $R_0$ und $R_1$ zusammen einen Rest der Formel VI oder der Formel VIII bilden:

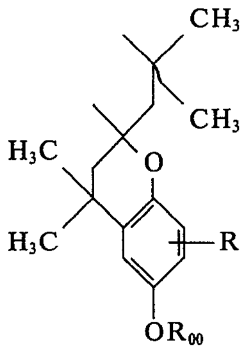

(VI)

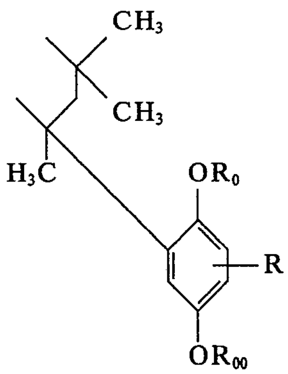

(VII)

wobei R, $R_0$ und $R_{00}$ die oben angegebene Bedeutung haben, mit der Maßgabe, daß die Verbindung der Formel I einen einzigen Rest der Formel III oder IV enthält, oder $R_{00}$ —H und $R_0$ ein Rest der Formel IVa oder IVb sind:

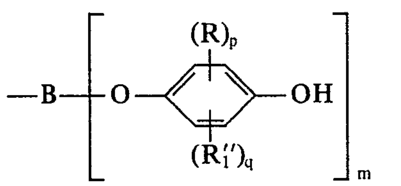

(IVa)

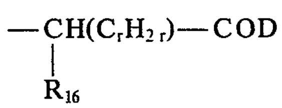

(IVb)

wobei p, q und R die oben angegebene Bedeutung haben, $R_1''$ $C_{1-8}$-Alkyl, $C_{7-9}$-Aralkyl oder ein Rest der Formel II, wie oben definiert, ist, m die Zahl 1 oder 2 ist, B bei m=1 $C_{2-12}$-Alkylen, das durch ein bis drei Sauerstoff- oder Schwefelatome unterbrochen sein kann, $C_{4-10}$-Alkenylen, $C_{5-12}$-Cycloalkylen, $C_{6-12}$-Arylen, $C_{8-12}$-Aralkylen, $C_{4-6}$-Alkynylen, Xylylen, $-CH_2CH(OH)CH_2-$, $-CH_2CH(OH)CH_2-O-Y-O-CH_2CH(OH)CH_2-$,

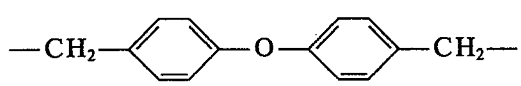

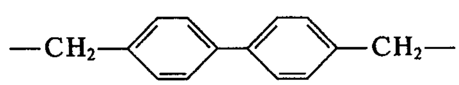

oder $-CH_2COO-B'-OCOCH_2-$, bedeutet, wobei Y $C_{2-10}$-Alkylen, $C_{6-12}$-Arylen,

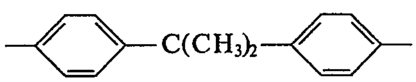

oder $C_{6-12}$-Cycloalkylen bedeutet,

B' $C_{2-8}$-Alkylen, $C_{4-8}$-Oxaalkylen oder Cyclohexylen und B bei m = 2 einen Rest der Formel

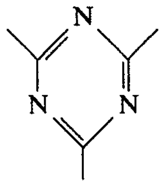 oder 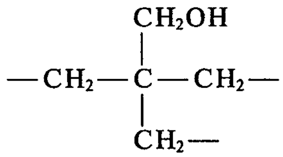 bedeuten,

r eine ganze Zahl von 0 bis 12 ist, und $R_{16}$ —H, $C_{1-12}$-Alkyl und D $-OR_4$ oder $-N(R_4)(R_5)$ bedeuten, wobei $R_4$ und $R_5$ die oben angegebene Bedeutung haben,

sowie Salze von Verbindungen der Formel I mit Basen oder organischen oder anorganischen Säuren, wenn Q eine saure oder basische Gruppe ist.

2. Verbindungen der Formel I nach Anspruch 1, worin die Gruppen R und $R_1$ in 2- bzw. 5-Stellung des Hydrochinonäthers der Formel I stehen, sowie Salze dieser Verbindungen.

3. Verbindungen gemäß Anspruch 2, dadurch gekennzeichnet, daß sie der Formel VIII entsprechen

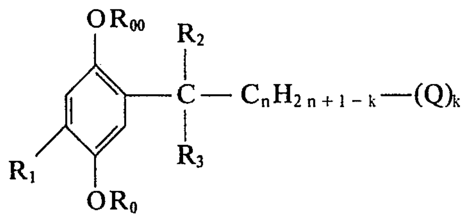 (VIII)

in welcher $R_2$, $R_3$, $R_0$, $R_{00}$, n, k und Q die im Anspruch 1 angegebene Bedeutung haben und $R_1$ eine Gruppe der Formel II, wie im Anspruch 1 definiert, ist, oder $R_1$ eine Gruppe der Formel IX

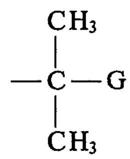 (IX)

bedeutet, worin G $C_{1-5}$-Alkyl oder Phenyl ist, sowie Salze von Verbindungen der Formel VIII.

4. Verbindungen der Formel VIII nach Anspruch 3, in welcher $R_1$, $R_0$, $R_{00}$, n und k die im Anspruch 3 angegebene Bedeutung haben, Q $-COOR_4$, $-CON(R_4)(R_5)$ oder $-OR_7$ bedeutet, wobei $R_4$, $R_5$ und $R_7$ die im Anspruch 1 angegebene Bedeutung haben, $R_2$ und $R_3$ unabhängig voneinander Methyl, Äthyl, n-Propyl, Isopropyl oder Neopentyl bedeuten, oder entweder $R_2$ oder $R_3$ durch eine Gruppe $-COOR_4$ substituiert sein können oder $R_2$ oder $R_3$ so an den Rest $-C_nH_{2n}-COOR_4$ gebunden sind, daß ein durch $-COOR_4$ substituierter $C_{5-8}$-Cycloalkylenrest gebildet wird, sowie Salze dieser Verbindungen.

5. Verbindungen der Formel VIII nach Anspruch 4, worin k die Zahl 1 ist, $R_1$ die im Anspruch 3 angegebene Bedeutung hat, Q $-COOR_4$, $-CON(R_4)(R_5)$ oder $-OR_7$ darstellt, n eine Zahl von 1 bis 10 ist, $R_2$ und $R_3$ unabhängig voneinander Methyl, Äthyl oder Neopentyl darstellen, oder entweder $R_2$ oder $R_3$ so an den Rest $-C_nH_{2n}-COOR_4$ gebunden sind, daß ein durch $-COOR_4$ substituierter Cyclohexylenrest gebildet wird, $R_4$ Wasserstoff oder $C_{1-20}$-Alkyl bedeutet, das durch 1 bis 3 Sauerstoffatome unterbrochen und/oder durch eine Gruppe $-OR_6$ substituiert sein kann, wobei $R_6$ Cyclopentyl, Cyclohexyl, Cyclooctyl, $C_{3-10}$-Alkenyl, Phenyl, Benzyl, Phenäthyl, Benzhydryl oder Naphthylmethyl bedeutet, ferner $R_4$ $C_{3-15}$-Alkenyl, Phenyl, das durch eine oder zwei $C_{1-4}$-Alkylgruppen substituiert sein kann, Benzyl, Phenäthyl, Cyclopentyl, Cyclohexyl, einen sauerstoffhaltigen 5- oder 6gliedrigen heterocyclischen Ring, der durch eine oder zwei $C_{1-4}$-Alkylgruppen substituiert sein kann, oder Methyl bedeutet, das durch einen sauerstoffhaltigen 5- oder 6gliedrigen heterocyclischen Ring substituiert ist, und $R_5$ die im Anspruch 1 angegebene Bedeutung hat, oder $R_4$ und $R_5$ zusammen mit dem Stickstoffatom, an das sie gebunden sind, einen 5- oder 6gliedrigen heterocyclischen Ring bilden können, der durch eine oder zwei $C_{1-4}$-Alkylgruppen substituiert sein kann, und $R_0$, $R_{00}$ und $R_7$ die im Anspruch 1 angegebene Bedeutung haben, sowie Salze dieser Verbindungen.

6. Verbindungen der Formel VIII nach Anspruch 5, worin n, k, Q und $R_1$ die im Anspruch 5 angegebene Bedeutung haben, $R_2$ und $R_3$ unabhängig voneinander Methyl, Äthyl oder Neopentyl bedeuten, oder eines von $R_2$ oder $R_3$ so an den Rest $-C_nH_{2n}-CR_4$ gebunden ist, daß ein durch $-COOR_4$ substituierter Cyclohexylenrest gebildet wird, $R_4$ $C_{1-20}$-Alkyl, das durch ein oder zwei Sauerstoffatome unterbrochen und/oder durch Cyclohexyloxy, $C_{3-10}$-Alkenyloxy, Phenoxy oder Benzyloxy substituiert sein kann, ferner $R_4$ $C_{3-15}$-Alkenyl, Phenyl, Benzyl, Phenäthyl, Cyclopentyl, Cyclohexyl, einen sauerstoffhaltigen 5- oder 6gliedrigen heterocyclischen Ring, oder Methyl bedeutet, das durch einen sauerstoffhaltigen 5-

oder 6gliedrigen heterocyclischen Ring substituiert ist, $R_5$ die im Anspruch 1 angegebene Bedeutung, oder $R_4$ und $R_5$ zusammen mit dem Stickstoffatom, an das sie gebunden sind, einen 5- oder 6gliedrigen heterocyclischen Ring bilden, der durch eine oder zwei $C_{1-4}$-Alkylgruppen substituiert sein kann, $R_7$ Wasserstoff oder $C_{1-15}$-Alkyl, $R_0$ und $R_{00}$ —H oder $C_{1-8}$-Alkyl, das durch ein oder zwei O-Atome unterbrochen sein kann, Cyclohexyl, Phenyl oder Benzyl bedeuten, oder $R_0$ und $R_1$ zusammen einen Rest der Formel V, wie im Anspruch 1 definiert, bilden, sowie Salze dieser Verbindungen.

7. Verbindungen der Formel VIII nach Anspruch 6, worin k, $R_1$, Q, n, $R_2$, $R_3$, $R_5$ und $R_7$ die im Anspruch 6 angegebene Bedeutung haben, und $R_4$ $C_{1-16}$-Alkyl, das durch ein Sauerstoffatom unterbrochen oder durch Phenoxy substituiert sein kann, ferner $R_4$ $C_{3-15}$-Alkenyl, Phenyl, Benzyl, Tetrahydrofuran-3-yl oder Tetrahydrofurfuryl bedeutet, oder $R_4$ und $R_5$ zusammen mit dem Stickstoffatom, an das sie gebunden sind, einen 5- oder 6gliedrigen heterocyclischen Ring bilden, der durch eine oder zwei $C_{1-4}$-Alkylgruppen substituiert sein kann, und $R_0$ und $R_{00}$ —H oder $C_{1-8}$-Alkyl sind, oder $R_0$ und $R_1$ zusammen einen Rest der Formel V, wie im Anspruch 1 definiert, bilden, sowie Salze dieser Verbindungen.

8. Verbindungen der Formel VIII nach Anspruch 7, worin k, $R_1$, Q und $R_5$, die im Anspruch 7 angegebene Bedeutung haben, $R_2$ und $R_3$ Methyl oder Neopentyl darstellen, $R_4$ $C_{1-16}$-Alkyl, das durch ein Sauerstoffatom unterbrochen oder durch Phenoxy substituiert sein kann, ferner $R_4$ $C_{3-15}$-Alkenyl, Phenyl, Benzyl, Tetrahydrofuran-3-yl oder Tetrahydrofurfuryl bedeutet, oder $R_4$ und $R_5$ zusammen mit dem Stickstoffatom, an das sie gebunden sind, einen 5- oder 6gliedrigen heterocyclischen Ring bilden, der durch eine oder zwei $C_{1-4}$-Alkylgruppen substituiert sein kann, $R_7$ —H oder $C_{1-15}$-Alkyl und $R_0$ und $R_{00}$ —H oder $C_{1-4}$-Alkyl sind, oder $R_0$ und $R_1$ zusammen einen Rest der Formel V, wie im Anspruch 1 definiert, bilden, und n die Zahl 1, 2 oder 3 ist, sowie Salze dieser Verbindungen.

9. Verbindungen der Formel VIII nach Anspruch 8, in welcher Q —$OR_7$ und $R_7$ $C_{1-8}$-Alkyl sind, sowie Salze dieser Verbindungen.

10. Verbindungen der Formel VIII nach Anspruch 3, in welcher eines von $R_0$ und $R_{00}$ —H und das andere $C_{1-4}$-Alkyl ist, oder $R_0$ und $R_1$ zusammen einen Rest der Formel V, wie im Anspruch 1 definiert, bilden.

11. Verbindungen der Formel XIV nach Anspruch 1,

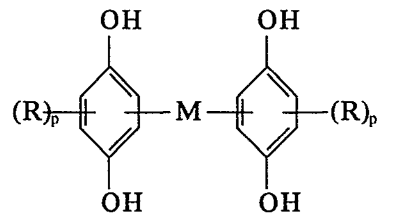

(XIV)

in welcher R, M und p die im Anspruch 1 angegebene Bedeutung haben.

12. Photographisches Material enthaltend als Stabilisator eine stabilisierende Menge einer oder mehrerer Verbindungen der Formel I gemäß Anspruch 1.

13. Photographisches Material nach Anspruch 12, dadurch gekennzeichnet, daß ein weiterer organischer Stabilisator, UV-Absorber, optischer Aufheller, Antischleiermittel und/oder Weichmacher mitverwendet werden.

14. Photographisches Material nach Anspruch 12, dadurch gekennzeichnet, daß ein Farbkuppler mitverwendet wird.

15. Photographisches Material nach Anspruch 13, dadurch gekennzeichnet, daß als weiterer organischer Stabilisator eine Hydrochinonverbindung der Formel XII

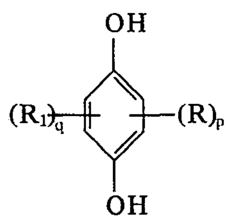

(XII)

eingesetzt wird, wobei $R_1$ $C_{1-8}$-Alkyl, $C_{7-9}$-Aralkyl oder einen Rest der Formel II oder III, wie im Anspruch 1 definiert, bedeutet, und R, p und q die im Anspruch 1 angegebene Bedeutung haben.

16. Verfahren zur Herstellung von Verbindungen der Formel I gemäß Anspruch 1, indem man in Gegenwart eines sauren Katalysators eine Verbindung der Formel X

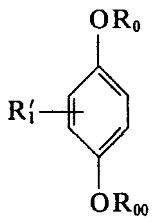

(X)

worin $R_1'$ Wasserstoff, $C_{1-8}$-Alkyl oder $C_{7-9}$-Aralkyl bedeutet, $R_0$ und $R_{00}$ die in Anspruch 1 genannte Bedeutung haben, oder $R_1'$ und $R_0$ zusammen einen Rest der Formel V, wie in Anspruch 1 definiert ist, bilden können, mit einem funktionellen Alkylierungsmittel, das zur Einführung einer oder zwei Gruppen der Formel II gemäß Anspruch 1 oder einer Gruppe der Formel IX gemäß Anspruch 3 in eine oder zwei aromatischen Ringe der Verbindung der Formel X befähigt ist, umsetzt.

## Claims

1. A compound of formula I

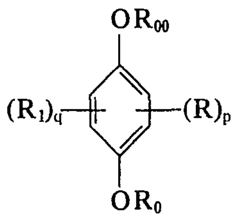

(I)

wherein p is 1 or 2 and q is 0 or 1, with the proviso that p + q is 1 or 2;

R is a residue of formula II

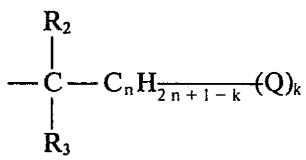

(II)

wherein

Q is selected from the residues consisting of:

i) $-COOR_4$ or $-CON(R_4)(R_5)$, wherein
$R_4$ independently is hydrogen; $C_1$—$C_{20}$alkyl which may be interrupted by one to five oxygen atoms and may be substituted by an $-OR_6$ group; a bifunctional $C_2$—$C_{20}$alkylene group; $C_3$—$C_{20}$alkenyl; $C_3$—$C_{12}$cycloalkyl; $C_6$—$C_{10}$aryl which may be substituted by one or two $C_1$—$C_8$alkyl groups; $C_7$—$C_{13}$aralkyl; a 5- or 6-membered oxygen-containing heterocycle which may be substituted by one or two $C_1$—$C_4$alkyl groups; or is methyl which is substituted by a 5- or 6-membered heterocycle which contains an oxygen atom and is unsubstituted or substituted by one or two $C_1$—$C_4$alkyl groups;
$R_5$ is hydrogen, $C_1$—$C_{20}$alkyl or $C_5$—$C_6$cycloalkyl, or $R_4$ and $R_5$, together with the nitrogen atom to which they are bonded, form a 5- or 6-membered heterocycle which may be substituted by one or two $C_1$—$C_4$alkyl groups;
$R_6$ is $C_3$—$C_{12}$cycloalkyl, $C_3$—$C_{20}$alkenyl, or is $C_6$—$C_{10}$aryl which may be substituted by one or two $C_1$—$C_4$alkyl groups, or is $C_7$—$C_{13}$aralkyl;

ii) $-OR_7$ or $-OCOR_7$, wherein
$R_7$ is hydrogen, $C_1$—$C_{20}$alkyl, $C_3$—$C_{20}$alkenyl, $C_3$—$C_{12}$cycloalkyl, $C_7$—$C_{13}$aralkyl, or is $C_6$—$C_{10}$aryl which may be substituted by one or two $C_1$—$C_4$alkyl groups;

iii) $-N(R_8)(R_9)$, wherein
$R_8$ and $R_9$ independently are hydrogen, $C_1$—$C_{20}$alkyl, $C_3$—$C_{20}$alkenyl, $C_3$—$C_{12}$cycloalkyl, $C_7$—$C_{13}$aralkyl, or are $C_6$—$C_{10}$aryl which may be substituted by one or two $C_1$—$C_4$alkyl groups, or are the group $-COR_7$, wherein $R_7$ is as defined above, or $R_8$ and $R_9$, together with the nitrogen atom to which they are bonded, form a 5- or 6-membered heterocyclic ring which is unsubstituted or substituted by one or two $C_1$—$C_4$alkyl groups;

iv) $-PO(OR_{10})([O]_xR_{11})$, wherein x is 0 or 1,
$R_{10}$ and $R_{11}$, when x is 1, are independently hydrogen, $C_1$—$C_{20}$alkyl, or are $C_6$—$C_{10}$aryl which may be substituted by one or two $C_1$—$C_4$alkyl groups, or $R_{10}$ and $R_{11}$ together are $C_2$—$C_3$alkylene which may be substituted by one to four $C_1$—$C_{20}$alkyl groups, and, when x is 0, $R_{10}$ is as defined above and $R_{11}$ is $C_1$—$C_5$alkyl;

v) $-SO_2R_{12}$, wherein $R_{12}$ is $-OH$, $-Cl$ or $N(R_5)(R_7)$, wherein $R_5$ and $R_7$ are as defined above, with the proviso that, when $R_{12}$ is $-OH$, $R_1$ is a residue of formula II;

vi) $-CN$, halogen or $-NO_2$;

vii) $-COR_{15}$, wherein $R_{15}$ is hydrogen, $C_1-C_{20}$alkyl or halogen;

n is an integer from 1 to 20;

k is 1 or 2;

$R_2$ and $R_3$ independently are $C_1-C_5$alkyl, with the proviso that, when Q is $-COOR_4$, one of $R_2$ or $R_3$ may be substituted by a $-COOR_4$ group in which the substituents $R_4$ may be identical or different, or $R_2$ or $R_3$ may be linked to the residue $-C_nH_{2n}-COOR_4$ such that there is formed a $C_5-C_{12}$cycloalkylene residue which is substituted by the group $-COOR_4$ in which the substituents $R_4$ may be identical or different, $R_4$ being as defined above, or, when Q is $-COR_{15}$, $R_2$ and $R_3$ may be linked to the residue $-C_nH_{2n}-COR_{15}$ such that there is formed a $C_5-C_{12}$cycloalkylene residue, $R_{15}$ being as defined above, with the proviso that, when the group Q is the residue $-COOR_4$, wherein $R_4$ is a bifunctional $C_2-C_{20}$alkylene residue, then p and k are 1 and the compound of formula I has the formula Ia:

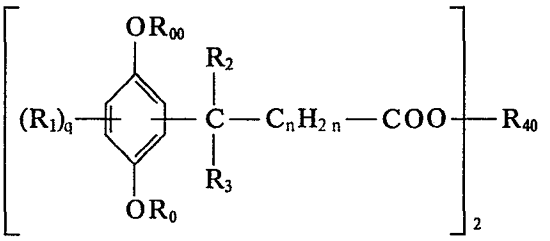

(Ia)

wherein $R_{40}$ is a bifunctional $C_2-C_{20}$alkylene residue, and, when Q is a $-CON(R_4)(R_5)$ residue, wherein $R_4$ is a bifunctional $C_2-C_{20}$alkylene residue, then p and k are 1 and the compound of formula I has the formula Ib:

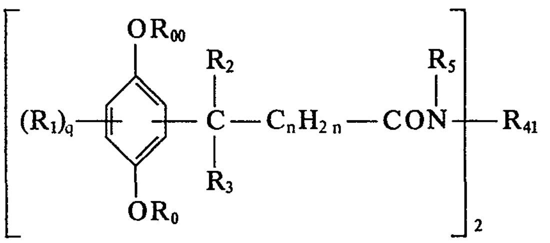

(Ib)

wherein $R_2$, $R_3$, q and n are as defined above and $R_{41}$ is $C_2-C_{20}$alkylene;

$R_1$ is $C_1-C_8$alkyl, or is $C_5-C_7$cycloalkyl which may be substituted by one or two methyl or ethyl groups, or is $C_7-C_9$aralkyl or a residue of formula II as defined above, and, when $R_1$ is a residue of formula II, $R_1$ and R may be identical or different; or

$R_1$ is a residue of formula III

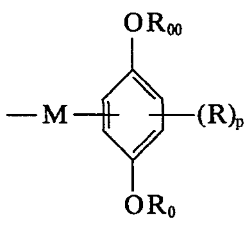

(III)

wherein

R and p are as defined above; M is a direct bond; $-C(R_{13})(R_{14})-$, wherein $R_{13}$ and $R_{14}$ independently are hydrogen, $C_1-C_{20}$alkyl which may be interrupted by one to three sulfur atoms, or are $C_6-C_{10}$aryl, or $R_{13}$ and $R_{14}$, together with the carbon atom to which they are bonded, may be 5- or 6-membered alkylene which may be substituted by one or two $C_1-C_8$alkyl groups; or M is $-S-$, $-S-S-$, $-SO_2-$, $-CH_2SCH_2-$, $-CH_2OCH_2$ or

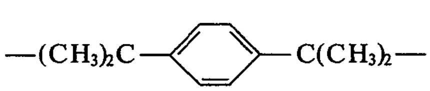

$R_0$ and $R_{00}$ independently are hydrogen, $C_1-C_{20}$alkyl which may be interrupted by one to five oxygen atoms; $C_3-C_{12}$cycloalkyl; $C_6-C_{10}$aryl which may be substituted by one or two

$C_1$—$C_4$alkyl groups; or are $C_7$—$C_{13}$aralkyl; with the proviso that $R_0$ and $R_{00}$ may not simultaneously be hydrogen, or $R_{00}$ is as defined above and $R_0$ is a residue of formula IV

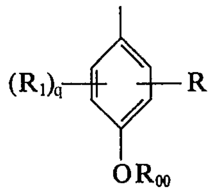

(IV)

wherein
R, $R_{00}$, $R_1$ and q are as defined above, or $R_0$ and $R_1$, when in ortho-position to one another, together with the carbon atom to which they are bonded, may form an unsubstituted or substituted residue of formula V

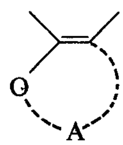

(V)

wherein A is a carbon residue containing 4 to 20 carbon atoms which forms a substituted chroman or coumaran system, or $R_0$ and $R_1$ together form a residue of formula VI or formula VII

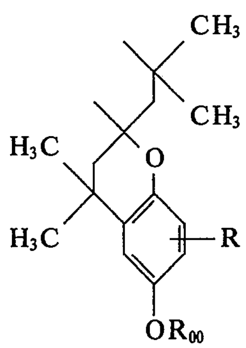

(VI)

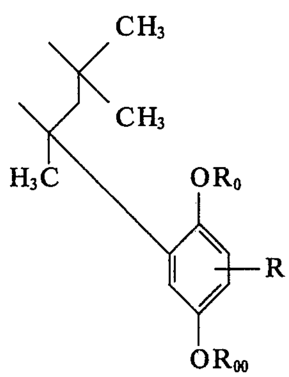

(VII)

wherein R, $R_0$ and $R_{00}$ are as defined above, with the proviso that the compound of formula I contains only one residue of formula III or IV, or $R_{00}$ is hydrogen and $R_0$ is a residue of formula IVa or IVb

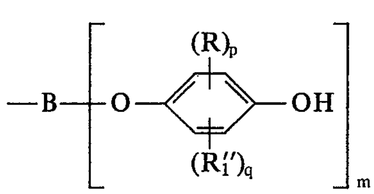

(IVa)

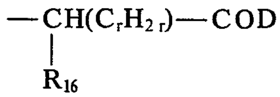

(IVb)

wherein p, q and R are as defined above, $R_1''$ is $C_1$—$C_8$alkyl, $C_7$—$C_9$aralkyl or a residue of formula II as defined above, m is 1 or 2, B, when m is 1, is $C_2$—$C_{12}$alkylene which may be interrupted by one to three oxygen or sulfur atoms, or is $C_4$—$C_{10}$alkenylene, $C_{5-12}$cycloalkylene, $C_{6-12}$arylene, $C_{8-12}$aralkylene, $C_{4-6}$alkynylene, xylylene, $-CH_2CH(OH)CH_2-$, $-CH_2CH(OH)CH_2-O-Y-O-CH_2CH(OH)CH_2-$,

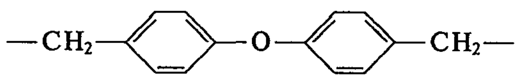

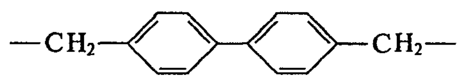

or $-CH_2COO-B'-OCOCH_2-$,
wherein Y is $C_2$—$C_{10}$alkylene, $C_6$—$C_{12}$arylene,

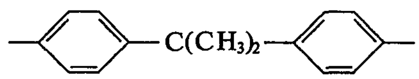

or $C_6$—$C_{12}$cycloalkylene,

B' is $C_2$—$C_8$alkylene, $C_4$—$C_8$oxaalkylene or cyclohexylene and B, when m is 2, is a residue of the the formulae

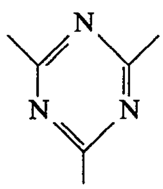

or

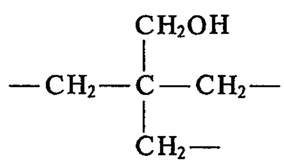

r is an integer from 0 to 12 and $R_{16}$ is hydrogen or $C_1$—$C_{12}$alkyl and D is $-OR_4$ or $-N(R_4)(R_5)$, wherein $R_4$ and $R_5$ are as defined above;

or a salt of a compound of formula I with a base or organic or inorganic acid, when Q is an acidic or basic group.

2. A compound of formula I according to claim 1, wherein the groups R and $R_1$ are in the 2- and 5-position respectively of the hydroquinone ether of formula I; or a salt thereof.

3. A compound according to claim 2 having the formula VIII:

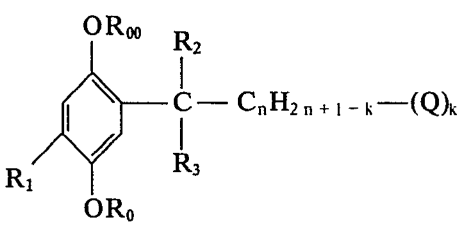

(VIII)

wherein $R_2$, $R_3$, $R_0$, $R_{00}$, n, k and Q are as defined in claim 1 and $R_1$ is a group of formula II, as defined in claim 1, or is a group of formula IX:

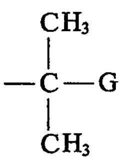

(IX)

wherein G is $C_1$—$C_5$alkyl or phenyl; or a salt thereof.

4. A compound of formula VIII according to claim 3, wherein $R_1$, $R_0$, $R_{00}$, n and k are as defined in claim 3, Q is $-COOR_4$, $-CON(R_4)(R_5)$ or $-OR_7$, wherein $R_4$, $R_5$ and $R_7$ are as defined in claim 1, $R_2$ and $R_3$ independently are methyl, ethyl, n-propyl, isopropyl or neopentyl, or either $R_2$ or $R_3$ may be substituted by a $-COOR_4$ group or $R_2$ or $R_3$ is linked to the residue $-C_nH_{2n}-COOR_4$ such that there is formed a $C_5$—$C_8$cycloalkylene residue which is substituted by $-COOR_4$; or a salt thereof.

5. A compound of formula VIII according to claim 4, wherein k is 1, $R_1$ is as defined in claim 3, Q is $-COOR_4$, $-CON(R_4)(R_5)$ or $-OR_7$, n is an integer from 1 to 10, $R_2$ and $R_3$ independently are methyl,

ethyl, or neopentyl, or either $R_2$ or $R_3$ is linked to the residue $-C_nH_{2n}-COOR_4$ such that there is formed a cyclohexylene residue which is substituted by $-COOR_4$, $R_4$ is hydrogen, $C_1-C_{20}$alkyl, which may be interrupted by one to three oxygen atoms and/or may be substituted by an $-OR_6$ group, wherein $R_6$ is cyclopentyl, cyclohexyl, cyclooctyl, $C_3-C_{10}$alkenyl, phenyl, benzyl, phenethyl, benzhydryl or naphthylmethyl, or $R_4$ is $C_3-C_{15}$alkenyl, or is phenyl which may be substituted by one or two $C_1-C_4$alkyl groups, or is benzyl, phenethyl, cyclopentyl or cyclohexyl or a 5- or 6-membered oxygen-containing heterocyclic ring which may be substituted by one or two $C_1-C_4$alkyl groups, or is methyl substituted by a 5- or 6-membered oxygen-containing heterocyclic ring, and $R_5$ is as defined in claim 1, or $R_4$ and $R_5$, together with the nitrogen atom to which they are bonded, form a 5- or 6-membered heterocyclic ring which may be substituted by one or two $C_1-C_4$alkyl groups; $R_0$, $R_{00}$and $R_7$ are as defined in claim 1, or a salt thereof.

6. A compound of formula VIII according to claim 5, wherein n, k, Q and $R_1$ are as defined in claim 5, $R_2$ and $R_3$ independently are methyl, ethyl or neopentyl, or one of $R_2$ or $R_3$ is linked to the residue $-C_nH_{2n}-COOR$ such that there is formed a cyclohexylene residue which is substituted by $-COOR_4$, $R_4$ is $C_1-C_{20}$-alkyl which may be interrupted by one or two oxygen atoms and/or may be substituted by cyclohexyloxy, $C_3-C_{10}$alkenyloxy, phenoxy or benzyloxy, or $R_4$ is $C_3-C_{15}$alkenyl, phenyl, benzyl, phenethyl, cyclopentyl, cyclohexyl, a 5- or 6-membered oxygen-containing heterocyclic ring or methyl which is substituted by a 5- or 6-membered oxygen-containing heterocyclic ring, $R_5$ is as defined in claim 1, or $R_4$ and $R_5$, together with the nitrogen atom to which they are bonded, form a 5- or 6-membered heterocyclic ring which may be substituted by one or two $C_1-C_4$alkyl groups, $R_7$ is hydrogen or $C_1-C_{15}$alkyl, and $R_0$ and $R_{00}$ are hydrogen or $C_1-C_8$alkyl which may be interrupted by one or two oxygen atoms, or are cyclohexyl, phenyl or benzyl, or $R_0$ and $R_1$ together form a residue of formula V as defined in claim 1; or a salt thereof.

7. A compound of formula VIII according to claim 6, wherein k, $R_1$, Q, n, $R_2$, $R_3$, $R_5$ and $R_7$ are as defined in claim 6 and $R_4$ is $C_1-C_{16}$alkyl which may be interrupted by an oxygen atom or may be substituted by phenoxy, or $R_4$ is $C_3-C_{15}$alkenyl, phenyl, benzyl, tetrahydrofuran-3-yl, or tetrahydrofurfuryl, or $R_4$ and $R_5$, together with the nitrogen atom to which they are bonded, form a 5- or 6-membered heterocyclic ring which may be substituted by one or two $C_1-C_4$alkyl groups, and $R_0$ and $R_{00}$ are hydrogen or $C_1-C_8$alkyl, or $R_0$ and $R_1$ together form a residue of formula V as defined in claim 1; or a salt thereof.

8. A compound of formula VIII according to claim 7, wherein k, $R_1$, Q and $R_5$ are as defined in claim 7, $R_2$ and $R_3$ are methyl or neopentyl, $R_4$ is $C_1-C_{16}$alkyl which may be interrupted by an oxygen atom or may be substituted by phenoxy, or $R_4$ is $C_3-C_{15}$alkenyl, phenyl, benzyl, tetrahydrofuran-3-yl or tetrahydrofurfuryl, or $R_4$ and $R_5$, together with the nitrogen atom to which they are bonded, form a 5- or 6-membered heterocyclic ring which may be substituted by one or two $C_1-C_4$alkyl groups, $R_7$ is hydrogen or $C_1-C_{15}$alkyl and $R_0$ and $R_{00}$ are hydrogen or $C_1-C_4$alkyl, or $R_0$ and $R_1$ together form a residue of formula V as defined in claim 1, and n is 1, 2 or 3; or a salt thereof.

9. A compound of formula VIII according to claim 8, wherein Q is $-OR_7$ and $R_7$ is $C_1-C_8$alkyl; or a salt thereof.

10. A compound of formula VIII according to claim 3, wherein one of $R_0$ and $R_{00}$ is hydrogen and the other is $C_1-C_4$alkyl, or $R_0$ and $R_1$ together form a residue of formula V as defined in claim 1.

11. A compound of formula XIV according to claim 1

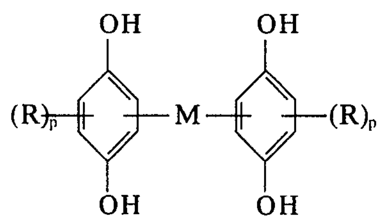

(XIV)

wherein R, M and p are as defined in claim 1.

12. Photographic material containing a stabilising amount of, as stabiliser, one or more compounds of formula I, as defined in claim 1.

13. Photographic material according to claim 12, wherein a further organic stabiliser, UV absorber, optical brightening agent, antifogging agent, and/or plasticizer are also present.

14. Photographic material according to claim 12, wherein a colour coupler is also present.

15. Photographic material according to claim 13, wherein the further organic stabiliser is a hydroquinone compound of formula XII

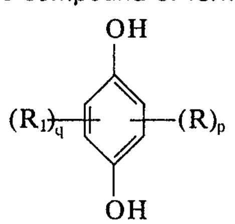

(XII)

wherein $R_1$ is $C_1$—$C_8$alkyl, $C_7$—$C_9$aralkyl or a residue of formula II or III as defined in claim 1 and R, p and q are as defined in claim 1.

16. A process for the preparation of a compound of formula I according to claim 1, which process comprises reacting, in the presence of an acid catalyst, a compound of formula X

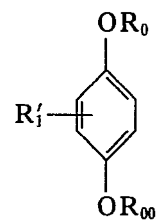

(X)

wherein $R_1'$ is hydrogen, $C_1$—$C_8$alkyl or $C_7$—$C_9$aralkyl, $R_0$ and $R_{00}$ are as defined in claim 1, or $R_1'$ and $R_0$ together may form a residue of formula V as claimed in claim 1, with a functional alkylating agent which is able to introduce one or two groups of formula II according to claim 1 or a group of formula IX according to claim 3 into one or two aromatic rings of the compound of formula X.

## Revendications

1. Composés de formule I

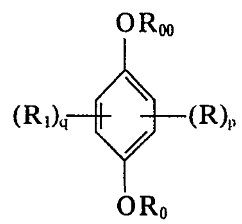

(I)

dans laquelle p vaut 1 ou 2 et q vaut 0 ou 1, à la condition que p + q vaille 1 ou 2,

R est un radical de formule II

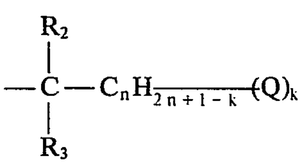

(II)

dans laquelle

Q correspond aux radicaux suivants:

i) $-COOR_4$ ou $-CON(R_4)(R_5)$, où

$R_4$ est, indépendamment, l'hydrogène, un radical alkyle en $C_{1-20}$ pouvant être interrompu par un à cinq atomes d'oxygène et substitué par un groupe $-OR_6$, un groupe alkylène en $C_{2-20}$ bifonctionnel, alcényle en $C_{3-20}$, cycloalkyle en $C_{3-12}$, aryle en $C_{6-10}$, qui peut être substitué par un ou deux groupes alkyle en $C_{1-8}$, aralkyle en $C_{7-13}$, un hétérocycle pentagonal ou hexagonal contenant de l'oxygène, pouvant être substitué par un ou deux groupes alkyle en $C_{1-4}$, ou le radical méthyle, lequel est substitué par un hétérocycle pentagonal ou hexagonal, contenant un atome d'oxygène, non-substitué ou substitué par un ou deux groupes alkyle en $C_{1-4}$,

$R_5$ est l'hydrogène, un radical alkyle en $C_{1-20}$ ou cycloalkyle en $C_{5-6}$, ou bien $R_4$ et $R_5$, avec l'atome d'azote auquel ils sont liés, forment un hétérocycle pentagonal ou hexagonal pouvant être substitué par un ou deux groupes alkyle en $C_{1-4}$, et

$R_6$ est un radical cycloalkyle en $C_3$—$_{12}$, alcényle en $C_{3-20}$, aryle en $C_{6-10}$, qui peut être substitué par un ou deux groupes alkyle en $C_{1-4}$, ou aralkyle en $C_{7-13}$,

ii) $-OR_7$ ou $-OCOR_7$, où

$R_7$ est l'hydrogène, un radical alkyle en $C_{1-20}$, alcényle en $C_{3-20}$, cycloalkyle en $C_{3-12}$, aralkyle en $C_{7-13}$ ou aryle en $C_{6-10}$, qui peut être substitué par un ou deux groupes alkyle en $C_{1-4}$,

iii) $-N(R_8)(R_9)$, où

$R_8$ et $R_9$, indépendamment l'un de l'autre, sont chacun $-H$, un radical alkyle en $C_{1-20}$, alcényle en $C_{3-20}$, cycloalkyle en $C_{3-12}$, aralkyle en $C_{7-13}$ ou aryle en $C_{6-10}$, qui peut être substitué par un ou deux groupes alkyle en $C_{1-4}$, ou encore le groupe $-COR_7$, dans lequel $R_7$ a la signification

donnée ci-dessus, ou bien $R_8$ et $R_9$, avec l'atome d'azote auquel ils sont liés, forme un noyau hétérocyclique pentagonal ou hexagonal, non-substitué ou substitué par un ou deux groupes alkyle en $C_{1-4}$,

iv) $-PO(OR_{10})([O]_xR_{11})$, où x est le nombre 0 ou 1, $R_{10}$ et $R_{11}$, pour x = 1, sont, indépendamment l'un de l'autre, chacun l'hydrogène, un radical alkyle en $C_{1-20}$, aryle en $C_{6-10}$, qui peut être substitué par un ou deux groupes alkyle en $C_{1-4}$, ou bien $R_{10}$ et $R_{11}$ représentent ensemble un radical alkylène en $C_{2-3}$ pouvant être substitué par un à quatre groupes alkyle en $C_{1-20}$, et, pour x = 0, $R_{10}$ a la signification donnée ci-dessus, et

$R_{11}$ est un radical alkyle en $C_{1-5}$,

v) $-SO_2R_{12}$, où $R_{12}$ est $-OH$, $-Cl$ ou $-N(R_5)(R_7)$ et $R_5$ et $R_7$ ont la signification donnée ci-dessus, à la condition que $R_1$ soit un radical de formule II quand $R_{12}$ est $-OH$,

vi) $-CN$, un halogène ou $-NO_2$, et

vii) $-COR_{15}$, où $R_{15}$ est $-H$ ou un radical alkyle en $C_{1-20}$, ou un halogène;

n est un nombre entier de 1 à 20, et

k est le nombre 1 ou 2,

$R_2$ et $R_3$, indépendamment l'un de l'autre, sont chacun un radical alkyle en $C_{1-5}$, où, quand Q est $-COOR_4$, l'un des radicaux $R_2$ ou $R_3$ peut être substitué par un groupe $-COOR_4$, les radicaux $R_4$ étant identiques ou différents, ou bien $R_2$ ou $R_3$ peuvent être liés au radical $-C_nH_{2n}-COOR_4$ de façon à former un radical cycloalkylène en $C_{5-12}$ substitué par le groupe $-COOR_4$, le radical $R_4$ étant identique ou différent, $R_4$ ayant la signification donnée ci-dessus, ou bien, quand Q est $-COR_{15}$, $R_2$ et $R_3$ sont liés au radical $C_nH_{2n}-COR_{15}$ de façon à former un radical cycloalkylène en $C_{5-12}$ où $R_{15}$ a la signification donnée ci-dessus, à la condition que p et k soient le nombre 1 et que le groupe de formule I ait la formule Ia quand le groupe Q est le radical $-COOR_4$, où $R_4$ est un radical alkylène en $C_{2-20}$ bifonctionnel,

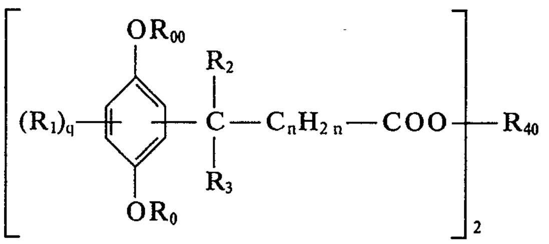

(Ia)

où $R_{40}$ est un radical alkylène en $C_{2-20}$ bifonctionnel, et, quand Q est un radical $-CON(R_4)(R_5)$ et $R_4$ est un radical alkylène en $C_{2-20}$ bifonctionnel, p et k soient le nombre 1 et le radical de formule I ait la formule Ib:

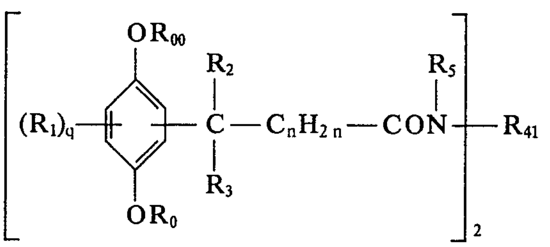

(Ib)

dans laquelle $R_2$, $R_3$, q et n ont la signification donnée ci-dessus $R_{41}$ est un radical alkylène en $C_{2-20}$,

$R_1$ est un radical alkyle en $C_{1-8}$, cycloalkyle en $C_{5-7}$ pouvant être substitué par un ou deux groupes méthyle ou éthyle, aralkyle en $C_{7-9}$, ou un radical de formule II, comme défini ci-dessus et, quand $R_1$ est un radical de formule II, $R_1$ et R peuvent être identiques ou différents, ou bien

$R_1$ est un radical de formule III

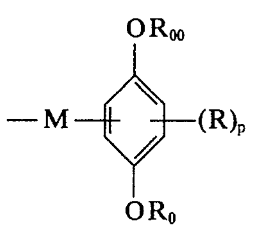

(III)

dans laquelle

R et p ont la signification donnée ci-dessus, M est une liaison directe, $-C(R_{13})(R_{14})-$, où $R_{13}$ et $R_{14}$ sont, indépendamment l'un de l'autre, chacun $-H$, un radical alkyle en $C_{1-20}$ pouvant être interrompu par un à trois atomes de soufre, ou aryle en $C_{6-10}$, ou bien $R_{13}$ et $R_{14}$ peuvent, avec l'atome d'azote auquel ils sont liés, représenter un alkylène à 5 ou 6 termes, lequel peut être substitué par un ou deux groupes alkyle en $C_{1-8}$, et de plus M est $-S-$, $-S-S-$, $-SO_2-$, $-CH_2SCH_2-$, $-CH_2OCH_2-$ ou

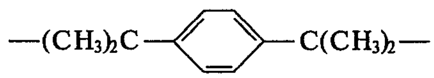

$R_0$ et $R_{00}$, indépendamment l'un de l'autre, sont chacun $-H$, un radical alkyle en $C_{1-20}$ pouvant être interrompu par 1 à 5 atomes d'oxygène, cycloalkyle en $C_{3-12}$, aryle en $C_{6-10}$ pouvant être substitué par un ou deux groupes alkyle en $C_{1-4}$, ou bien aralkyle en $C_{7-13}$, à la condition que $R_0$ et $R_{00}$ ne puissent simultanément être l'hydrogène, ou bien $R_{00}$ a la signification donnée ci-dessus et $R_0$ est un radical de formule IV

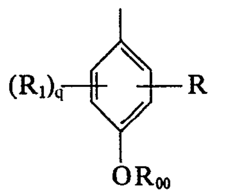

(IV)

dans laquelle R, $R_{00}$, $R_1$ et q ont la signification donnée ci-dessus, ou bien $R_0$ et $R_1$, quand ils sont en position ortho l'un par rapport à l'autre, peuvent, avec l'atome de carbone auquel ils sont liés, former un radical éventuellement substitué de formule V

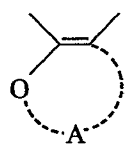

(V)

dans laquelle A est un radical hydrocarboné contenant 4 à 20 atomes de carbone, qui forme un système chromanne ou coumaranne substitué, ou bien $R_0$ et $R_1$ forment ensemble un radical de formule VI ou de formule VIII

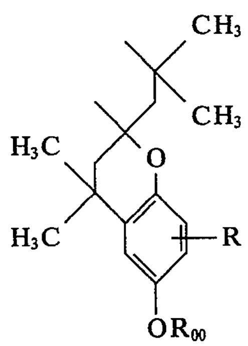

(VI)

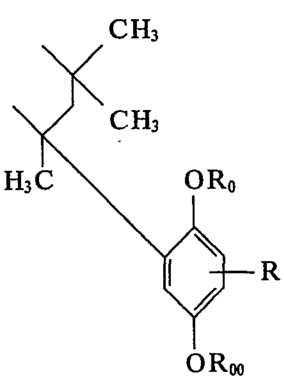

(VII)

où R, $R_0$ et $R_{00}$ ont la signification donnée ci-dessus, à la condition que le composé de formule I contienne un radical unique de formule III ou IV, ou bien $R_{00}$ soit –H et $R_0$ soit un radical de formule IVa ou IVb

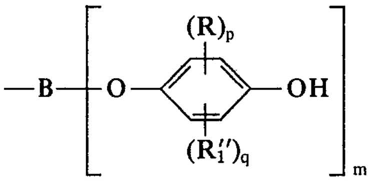

(IVa)

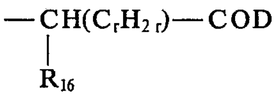

(IVb)

où p, q et r ont la signification donnée ci-dessus, $R_1''$ est un radical alkyle en $C_{1-8}$, aralkyle en $C_{7-9}$ ou un radical de formule II tel que défini ci-dessus, m est le nombre 1 ou 2, B, pour m = 1, est un radical alkylène en $C_{2-12}$ pouvant être interrompu par un à trois atomes d'oxygène ou de soufre, alcénylène en $C_{4-10}$, cycloalkylène en $C_{5-12}$, arylène en $C_{6-12}$, aralkylène en $C_{8-12}$, alcynylène en $C_{4-6}$, xylylène, $-CH_2CH(OH)CH_2-$, $-CH_2CH(OH)CH_2-O-Y-O-CH_2CH(OH)CH_2-$,

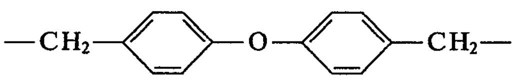

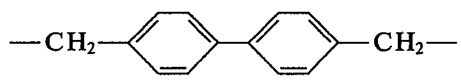

ou $-CH_2COO-B'-OCOCH_2-$, où Y est un radical alkylène en $C_{2-10}$, arylène en $C_{6-12}$,

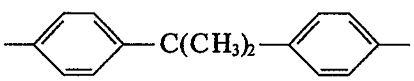

ou cycloalkylène en $C_{6-12}$,

B' est un radical alkylène en $C_{2-8}$, oxaalkylène en $C_{4-8}$ ou cyclohexylène, et B, pour m = 2, est un radical de formule

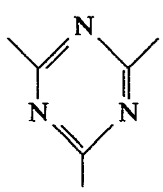

ou

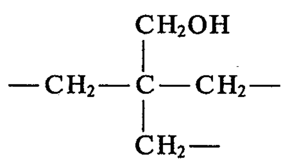

r est un nombre entier de 0 à 12, et $R_{16}$ est –H, un radical alkyle en $C_{1-12}$, et D est $-OR_4$ ou $-N(R_4)(R_5)$, où $R_4$ et $R_5$ ont la signification donnée ci-dessus,

ainsi que les sels de composés de formule I avec des bases ou des acides organiques ou inorganiques, quand Q est un groupe acide ou basique.

2. Composés de formule I selon la revendication 1, dans lesquels les groupes R et $R_1$ sont respectivement en position 2 et 5 de l'éther d'hydroquinone de formule I, ainsi que les sels de ces composés.

3. Composés selon la revendication 2, caractérisés en ce qu'ils ont la formule VIII

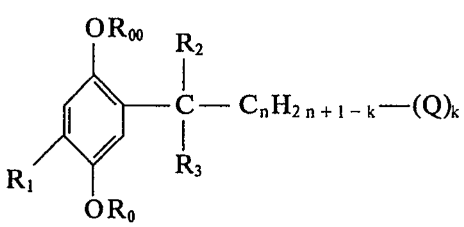

(VIII)

dans laquelle $R_2$, $R_3$, $R_0$, $R_{00}$, n, k et Q ont la signification donnée dans la revendication 1, et $R_1$ est un groupe de formule II, tel que défini dans la revendication 1, ou bien $R_1$ est un groupe de formule IX

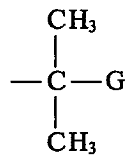

(IX)

dans laquelle G est un radical alkyle en $C_{1-5}$ ou phényle, ainsi que les sels de composés de formule VIII.

4. Composés de formule VIII selon la revendication 3, dans lesquels $R_1$, $R_0$, $R_{00}$, n et k ont la signification donnée dans la revendication 3, Q est $-COOR_4$, $-CON(R_4)(R_5)$ ou $-OR_7$, où $R_4$, $R_5$ et $R_7$ ont la signification donnée dans la revendication 1, $R_2$ et $R_3$, indépendamment l'un de l'autre, sont chacun le radical méthyle, éthyle, n-propyle, isopropyle ou néopentyle, ou bien soit $R_2$ ou $R_3$ peuvent être substitués par un groupe $-COOR_4$, soit $R_2$ ou $R_3$ sont liés au radical $-C_nH_{2n}-COOR_4$ de façon à former un radical cycloalkylène en $C_{5-8}$ substitué par un substituant $-COOR_4$, ainsi que les sels de ces composés.

5. Composés de formule VIII selon la revendication 4, dans lesquels k est le nombre 1, $R_1$ a la signification donnée dans la revendication 3, Q est $-COOR_4$, $-CON(R_4)(R_5)$ ou $-OR_7$, n est un nombre de 1 à 10, $R_2$ et $R_3$, indépendamment l'un de l'autre, sont chacun le radical méthyle, éthyle ou néopentyle, ou bien soit $R_2$, soit $R_3$, sont liés au radical $-C_nH_{2n}-COOR_4$ de façon à former un radical cyclohexylène substitué par un substituant $-COOR_4$, $R_4$ est l'hydrogène ou un radical alkyle en $C_{1-20}$ pouvant être interrompu par 1 à 3 atomes d'oxygène et/ou substitué par un groupe $-OR_6$, où $R_6$ est un radical cyclopentyle, cyclohexyle, cyclooctyle, alcényle en $C_{3-10}$, phényle, benzyle, phénéthyle, benzhydryle ou naphthylméthyle, de plus $R_4$ est un radical alcényle en $C_{3-15}$, phényle pouvant être substitué par un ou deux groupes alkyle en $C_{1-4}$, benzyle, phénéthyle, cyclopentyle, cyclohexyle, un noyau hétérocyclique pentagonal ou hexagonal contenant de l'oxygène et pouvant être substitué par un ou deux groupes alkyle en $C_{1-4}$, ou encore méthyle, qui est substitué par un noyau hétérocyclique pentagonal ou hexagonal contenant de l'oxygène, et $R_5$ a la signification donnée dans la revendication 1, ou bien $R_4$ et $R_5$ peuvent, avec l'atome d'azote auquel ils sont liés, former un noyau hétérocyclique pentagonal ou hexagonal, lequel peut être substitué par un ou deux groupes alkyle en $C_{1-4}$, et $R_0$, $R_{00}$ et $R_7$ ont la signification donnée dans la revendication 1, ainsi que les sels de ces composés.

6. Composés de formule VIII selon la revendication 5, dans lequels n, k, Q et $R_1$ ont la signification donnée dans la revendication 5, $R_2$ et $R_3$, indépendamment l'un de l'autre, sont chacun le radical méthyle, éthyle ou néopentyle, ou bien l'un des deux radicaux $R_2$ ou $R_3$ est lié au radical $-C_nH_{2n}-COOR_4$ de façon à former un radical cyclohexylène substitué par un substituant $-COOR_4$, $R_4$ est un radical en $C_{1-20}$ pouvant être interrompu par un ou deux atomes d'oxygène et/ou substitué par un radical cyclohexyloxy, alcényloxy en $C_{3-10}$, phénoxy ou benzyloxy, de plus $R_4$ est un radical alcényle en $C_{3-15}$, phényle, benzyle, phénéthyle, cyclopentyle, cyclohexyle, un noyau hétérocyclique pentagonal ou hexagonal contenant de l'oxygène, ou encore le radical méthyle, qui est substitué par un noyau hétérocyclique pentagonal ou hexagonal contenant de l'oxygène, $R_5$ a la signification donnée dans la revendication 1, ou bien $R_4$ et $R_5$ forment avec l'atome d'azote auquel ils sont liés un noyau hétérocyclique pentagonal ou hexagonal pouvant être substitué par un ou deux groupes alkyle en $C_{1-4}$, $R_7$ est l'hydrogène ou un radical alkyle en $C_{1-15}$, $R_0$ et $R_{00}$ sont $-H$ ou un radical alkyle en $C_{1-8}$ pouvant être interrompu par un ou deux atomes d'oxygène, cyclohexyle, phényle ou benzyle, ou bien $R_0$ et $R_1$ forment ensemble un radical de formule V tel que défini dans la revendication 1, ainsi que les sels de ces composés.

7. Composés de formule VIII selon la revendication 6, où k, $R_1$, Q, n, $R_2$, $R_3$, $R_5$ et $R_7$ ont la signification donnée dans la revendication 6, et $R_4$ est un radical alkyle en $C_{1-16}$ pouvant être interrompu par un atome d'oxygène ou substitué par un groupe phénoxy, de plus $R_4$ est un radical alcényle en $C_{3-15}$, phényle, benzyle, tétrahydrofurannyle-3 ou tetrahydrofurfuryle, ou bien $R_4$ et $R_5$, avec l'atome d'azote auquel ils sont liés, forment un noyau hétérocyclique pentagonal ou hexagonal pouvant être substitué par un ou deux groupes alkyle en $C_{1-4}$, et $R_0$ et $R_{00}$ sont $-H$ ou un radical alkyle en $C_{1-8}$, ou bien $R_0$ et $R_1$ forment ensemble un radical de formule V tel que défini dans la revendication 1, ainsi que les sels de ces composés.

8. Composés de formule VIII selon la revendication 7, dans lesquels k, $R_1$, Q et $R_5$ ont la signification donnée dans la revendication 7, $R_2$ et $R_3$ sont le radical méthyle ou néopentyle, $R_4$ est un radical alkyle en $C_{1-16}$ pouvant être interrompu par un atome d'oxygène ou substitué par un groupe phénoxy, de plus $R_4$ est un radical alcényle en $C_{3-15}$, phényle, benzyle, tetrahydrofurannyle-3 ou tétrahydrofurfuryle, ou bien $R_4$ et $R_5$ forment avec l'atome d'azote auquel ils sont liés un noyau hétérocyclique pentagonal ou hexagonal pouvant être substitué par un ou deux groupes alkyle en $C_{1-4}$, $R_7$ est $-H$ ou un radical alkyle en $C_{1-15}$, et $R_0$ et $R_{00}$ sont $-H$ ou un radical alkyle en $C_{1-4}$, ou bien $R_0$ et $R_1$ forment ensemble un radical de formule V tel que défini dans la revendication 1, et n est le nombre 1, 2 ou 3, ainsi que les sels de ces composés.

9. Composés de formule VIII selon la revendication 8, dans laquelle Q est $-OR_7$ et $R_7$ est un radical alkyle en $C_{1-8}$, ainsi que les sels de ces composés.

10. Composés de formule VIII selon la revendication 3, dans laquelle l'un des radicaux $R_0$ et $R_{00}$ est $-H$ et l'autre est un radical alkyle en $C_{1-4}$, ou bien $R_0$ et $R_1$ forment ensemble un radical de formule V tel que défini dans la revendication 1.

11. Composés de formule XIV selon la revendication 1

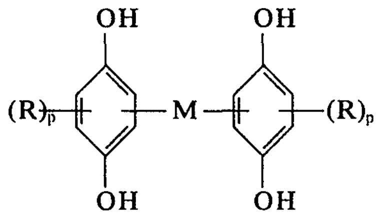

(XIV)

dans laquelle R, M et p ont la signification donnée dans la revendication 1.

12. Matériau photographique contenant en tant que stabilisant une quantité stabilisante d'un ou plusieurs composés de formule I selon la revendication 1.

13. Matériau photographique selon la revendication 12, caractérisé en ce qu'on utilise aussi un autre stabilisant organique, un absorbant UV, un azurant optique, un agent antivoile et/ou un plastifiant.

14. Matériau photographique selon la revendication 12, caractérisé en ce qu'il contient en outre un copulant chromogène.

15. Matériau photographique selon la revendication 13, caractérisé en ce qu'il contient en tant qu'autre stabilisant organique un composé de l'anthraquinone de formule XII

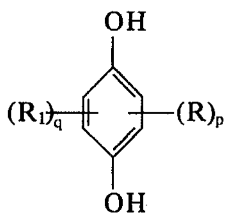

(XII)

dans laquelle $R_1$ est un radical alkyle en $C_{1-8}$, aralkyle en $C_{7-9}$ ou un radical de formule II ou III tel que défini dans la revendication 1, et R, p et q ont la signification donnée dans la revendication 1.

16. Procédé pour la préparation de composés de formule 1 selon la revendication 1, dans lequel on fait réagir, en présence d'un catalyseur acide, un composé de formule X

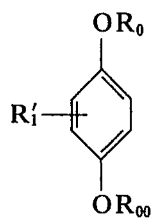

(X)

dans laquelle $R_1'$ est l'hydrogène, un radical alkyle en $C_{1-8}$ ou aralkyle en $C_{7-9}$, $R_0$ et $R_{00}$ ont la signification donnée dans la revendication 1, ou bien $R_1'$ et $R_0$ peuvent former ensemble un radical de formule V tel que défini dans la revendication 1, sur un agent d'alkylation fonctionnel, qui est à même d'introduire un ou deux groupes de formule II selon la revendication 1 ou un groupe de formule IX selon la revendication 3 dans un ou deux noyaux aromatiques de composés de formule X.